(19) Europäisches Patentamt
European Patent Office
Office européen des brevets

(11) EP 3 540 035 A1

(12) EUROPEAN PATENT APPLICATION

(43) Date of publication:
**18.09.2019 Bulletin 2019/38**

(21) Application number: **18161364.7**

(22) Date of filing: **13.03.2018**

(51) Int Cl.:
***C11D 1/94*** (2006.01) ***C07K 14/415*** (2006.01)
***C11D 11/00*** (2006.01) ***C11D 3/382*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **The Procter & Gamble Company**
**Cincinnati, OH 45202 (US)**

(72) Inventors:
- **GONZALES, Denis Alfred**
**1853 Strombeek-Bever (BE)**
- **BETTIOL, Jean-Luc Philippe**
**1853 Strombeek-Bever (BE)**

(74) Representative: **P&G Patent Belgium UK**
**N.V. Procter & Gamble Services Company S.A.**
**Temselaan 100**
**1853 Strombeek-Bever (BE)**

# (54) HAND DISHWASHING DETERGENT COMPOSITION

(57) The present invention is directed to a detergent composition having enhanced suds boosting and/or increased suds longevity, especially in the presence of greasy soils, wherein the detergent composition is a hand dishwashing detergent composition. The composition includes a specific surfactant system including an anionic surfactant and a primary co-surfactant, wherein the weight ratio of the anionic surfactant to the primary co-surfactant is less than 9:1, preferably from 5:1 to 1:1, more preferably from 4:1 to 2:1, and a plant derived protein or blend of plant derived proteins derived from a Leguminous plant family, preferably a Pulse protein selected from the group consisting of a Pea protein, a Chickpea protein, a Lentil protein, a Bean protein, and mixtures thereof, preferably a Pea protein.

EP 3 540 035 A1

SEQUENCE LISTING

<110> The Procter & Gamble Company

<120> HAND DISHWASHING DETERGENT COMPOSITION

<130> CM04958F

<140> 18161364.7
<141> 2018-03-13

<160> 79

<170> PatentIn version 3.5

<210> 1
<211> 517
<212> PRT
<213> NONE

<400> 1

```
Met Ala Lys Leu Leu Ala Leu Ser Leu Ser Phe Cys Phe Leu Leu Leu
1               5                   10                  15

Gly Gly Cys Phe Ala Leu Arg Glu Gln Pro Gln Gln Asn Glu Cys Gln
            20                  25                  30

Leu Glu Arg Leu Asp Ala Leu Glu Pro Asp Asn Arg Ile Glu Ser Glu
        35                  40                  45

Gly Gly Leu Ile Glu Thr Trp Asn Pro Asn Asn Lys Gln Phe Arg Cys
    50                  55                  60

Ala Gly Val Ala Leu Ser Arg Ala Thr Leu Gln Arg Asn Ala Leu Arg
65                  70                  75                  80

Arg Pro Tyr Tyr Ser Asn Ala Pro Gln Glu Ile Phe Ile Gln Gln Gly
                85                  90                  95

Asn Gly Tyr Phe Gly Met Val Phe Pro Gly Cys Pro Glu Thr Phe Glu
            100                 105                 110

Glu Pro Gln Glu Ser Glu Gln Gly Glu Gly Arg Arg Tyr Arg Asp Arg
        115                 120                 125

His Gln Lys Val Asn Arg Phe Arg Glu Gly Asp Ile Ile Ala Val Pro
    130                 135                 140

Thr Gly Ile Val Phe Trp Met Tyr Asn Asp Gln Asp Thr Pro Val Ile
145                 150                 155                 160

Ala Val Ser Leu Thr Asp Ile Arg Ser Ser Asn Asn Gln Leu Asp Gln
```

165 170 175

Met Pro Arg Arg Phe Tyr Leu Ala Gly Asn His Glu Gln Glu Phe Leu
180 185 190

Gln Tyr Gln His Gln Gln Gly Gly Lys Gln Glu Gln Glu Asn Glu Gly
195 200 205

Asn Asn Ile Phe Ser Gly Phe Lys Arg Asp Tyr Leu Glu Asp Ala Phe
210 215 220

Asn Val Asn Arg His Ile Val Asp Arg Leu Gln Gly Arg Asn Glu Asp
225 230 235 240

Glu Glu Lys Gly Ala Ile Val Lys Val Lys Gly Gly Leu Ser Ile Ile
245 250 255

Ser Pro Pro Glu Lys Gln Ala Arg His Gln Arg Gly Ser Arg Gln Glu
260 265 270

Glu Asp Glu Asp Glu Glu Lys Gln Pro Arg His Gln Arg Gly Ser Arg
275 280 285

Gln Glu Glu Glu Glu Asp Glu Asp Glu Glu Arg Gln Pro Arg His Gln
290 295 300

Arg Arg Arg Gly Glu Glu Glu Glu Glu Asp Lys Lys Glu Arg Gly Gly
305 310 315 320

Ser Gln Lys Gly Lys Ser Arg Arg Gln Gly Asp Asn Gly Leu Glu Glu
325 330 335

Thr Val Cys Thr Ala Lys Leu Arg Leu Asn Ile Gly Pro Ser Ser Ser
340 345 350

Pro Asp Ile Tyr Asn Pro Glu Ala Gly Arg Ile Lys Thr Val Thr Ser
355 360 365

Leu Asp Leu Pro Val Leu Arg Trp Leu Lys Leu Ser Ala Glu His Gly
370 375 380

Ser Leu His Lys Asn Ala Met Phe Val Pro His Tyr Asn Leu Asn Ala
385 390 395 400

Asn Ser Ile Ile Tyr Ala Leu Lys Gly Arg Ala Arg Leu Gln Val Val
405 410 415

Asn Cys Asn Gly Asn Thr Val Phe Asp Gly Glu Leu Glu Ala Gly Arg
420 425 430

Ala Leu Thr Val Pro Gln Asn Tyr Ala Val Ala Ala Lys Ser Leu Ser
435 440 445

Asp Arg Phe Ser Tyr Val Ala Phe Lys Thr Asn Asp Arg Ala Gly Ile
450 455 460

Ala Arg Leu Ala Gly Thr Ser Ser Val Ile Asn Asn Leu Pro Leu Asp
465 470 475 480

Val Val Ala Ala Thr Phe Asn Leu Gln Arg Asn Glu Ala Arg Gln Leu
485 490 495

Lys Ser Asn Asn Pro Phe Lys Phe Leu Val Pro Ala Arg Glu Ser Glu
500 505 510

Asn Arg Ala Ser Ala
515

<210> 2
<211> 503
<212> PRT
<213> NONE

<400> 2

Met Ser Lys Pro Phe Leu Ser Leu Leu Ser Leu Ser Leu Leu Leu Phe
1 5 10 15

Ala Ser Ala Cys Leu Ala Thr Ser Ser Glu Phe Asp Arg Leu Asn Gln
20 25 30

Cys Gln Leu Asp Ser Ile Asn Ala Leu Glu Pro Asp His Arg Val Glu
35 40 45

Ser Glu Ala Gly Leu Thr Glu Thr Trp Asn Pro Asn His Pro Glu Leu
50 55 60

Lys Cys Ala Gly Val Ser Leu Ile Arg Arg Thr Ile Asp Pro Asn Gly
65 70 75 80

Leu His Leu Pro Ser Phe Ser Pro Ser Pro Gln Leu Ile Phe Ile Ile
85 90 95

Gln Gly Lys Gly Val Leu Gly Leu Ser Phe Pro Gly Cys Pro Glu Thr
100 105 110

```
Tyr Glu Glu Pro Arg Ser Ser Gln Ser Arg Gln Glu Ser Arg Gln Gln
        115                 120                 125

Gln Gly Asp Ser His Gln Lys Val Arg Arg Phe Arg Lys Gly Asp Ile
    130                 135                 140

Ile Ala Ile Pro Ser Gly Ile Pro Tyr Trp Thr Tyr Asn His Gly Asp
145                 150                 155                 160

Glu Pro Leu Val Ala Ile Ser Leu Leu Asp Thr Ser Asn Ile Ala Asn
                165                 170                 175

Gln Leu Asp Ser Thr Pro Arg Val Phe Tyr Leu Gly Gly Asn Pro Glu
            180                 185                 190

Thr Glu Phe Pro Glu Thr Gln Glu Glu Gln Gln Gly Arg His Arg Gln
        195                 200                 205

Lys His Ser Tyr Pro Val Gly Arg Arg Ser Gly His His Gln Gln Glu
    210                 215                 220

Glu Glu Ser Glu Glu Gln Asn Glu Gly Asn Ser Val Leu Ser Gly Phe
225                 230                 235                 240

Ser Ser Glu Phe Leu Ala Gln Thr Phe Asn Thr Glu Glu Asp Thr Ala
                245                 250                 255

Lys Arg Leu Arg Ser Pro Arg Asp Glu Arg Ser Gln Ile Val Arg Val
            260                 265                 270

Glu Gly Gly Leu Arg Ile Ile Lys Pro Lys Gly Lys Glu Glu Glu Glu
        275                 280                 285

Lys Glu Gln Ser His Ser His Ser His Arg Glu Glu Lys Glu Glu Glu
    290                 295                 300

Glu Glu Glu Glu Glu Asp Glu Glu Glu Lys Gln Arg Ser Glu Glu Arg
305                 310                 315                 320

Lys Asn Gly Leu Glu Glu Thr Ile Cys Ser Ala Lys Ile Arg Glu Asn
                325                 330                 335

Ile Ala Asp Ala Ala Arg Ala Asp Leu Tyr Asn Pro Arg Ala Gly Arg
            340                 345                 350

Ile Ser Thr Ala Asn Ser Leu Thr Leu Pro Val Leu Arg Tyr Leu Arg
        355                 360                 365
```

Leu Ser Ala Glu Tyr Val Arg Leu Tyr Arg Asn Gly Ile Tyr Ala Pro
370 375 380

His Trp Asn Ile Asn Ala Asn Ser Leu Leu Tyr Val Ile Arg Gly Glu
385 390 395 400

Gly Arg Val Arg Ile Val Asn Cys Gln Gly Asn Thr Val Phe Asp Asn
405 410 415

Lys Val Arg Lys Gly Gln Leu Val Val Val Pro Gln Asn Phe Val Val
420 425 430

Ala Glu Gln Ala Gly Glu Glu Glu Gly Leu Glu Tyr Val Val Phe Lys
435 440 445

Thr Asn Asp Arg Ala Ala Val Ser His Val Gln Gln Val Phe Arg Ala
450 455 460

Thr Pro Ser Glu Val Leu Ala Asn Ala Phe Gly Leu Arg Gln Arg Gln
465 470 475 480

Val Thr Glu Leu Lys Leu Ser Gly Asn Arg Gly Pro Leu Val His Pro
485 490 495

Arg Ser Gln Ser Gln Ser His
500

<210> 3
<211> 520
<212> PRT
<213> NONE

<400> 3

Met Ala Thr Lys Leu Leu Ala Leu Ser Leu Ser Phe Cys Phe Leu Leu
1 5 10 15

Leu Gly Gly Cys Phe Ala Leu Arg Glu Gln Pro Glu Gln Asn Glu Cys
20 25 30

Gln Leu Glu Arg Leu Asn Ala Leu Glu Pro Asp Asn Arg Ile Glu Ser
35 40 45

Glu Gly Gly Leu Ile Glu Thr Trp Asn Pro Asn Asn Lys Gln Phe Arg
50 55 60

Cys Ala Gly Val Ala Leu Ser Arg Ala Thr Leu Gln His Asn Ala Leu
65 70 75 80

```
Arg Arg Pro Tyr Tyr Ser Asn Ala Pro Gln Glu Ile Phe Ile Gln Gln
                85                  90                  95

Gly Asn Gly Tyr Phe Gly Met Val Phe Pro Gly Cys Pro Glu Thr Phe
            100                 105                 110

Glu Glu Pro Gln Glu Ser Glu Gln Gly Glu Gly Arg Arg Tyr Arg Asp
        115                 120                 125

Arg His Gln Lys Val Asn Arg Phe Arg Glu Gly Asp Ile Ile Ala Val
    130                 135                 140

Pro Thr Gly Ile Val Phe Trp Met Tyr Asn Asp Gln Asp Thr Pro Val
145                 150                 155                 160

Ile Ala Val Ser Leu Thr Asp Ile Arg Ser Ser Asn Asn Gln Leu Asp
                165                 170                 175

Gln Met Pro Arg Arg Phe Tyr Leu Ala Gly Asn His Glu Gln Glu Phe
            180                 185                 190

Leu Arg Tyr Gln His Gln Gln Gly Gly Lys Gln Glu Gln Glu Asn Glu
        195                 200                 205

Gly Asn Asn Ile Phe Ser Gly Phe Lys Arg Asp Phe Leu Glu Asp Ala
    210                 215                 220

Phe Asn Val Asn Arg His Ile Val Asp Arg Leu Gln Gly Arg Asn Glu
225                 230                 235                 240

Asp Glu Glu Lys Gly Ala Ile Val Lys Val Lys Gly Gly Leu Ser Ile
                245                 250                 255

Ile Ser Pro Pro Glu Lys Gln Ala Arg His Gln Arg Gly Ser Arg Gln
            260                 265                 270

Glu Glu Asp Glu Asp Glu Asp Glu Glu Arg Gln Pro Arg His Gln Arg
        275                 280                 285

Gly Ser Arg Gln Glu Glu Glu Glu Asp Glu Asp Glu Glu Arg Gln Pro
    290                 295                 300

Arg His Gln Arg Arg Arg Gly Glu Glu Glu Glu Glu Asp Lys Lys Glu
305                 310                 315                 320

Arg Arg Gly Ser Gln Lys Gly Lys Ser Arg Arg Gln Gly Asp Asn Gly
                325                 330                 335
```

Leu Glu Glu Thr Val Cys Thr Ala Lys Leu Arg Leu Asn Ile Gly Pro
            340                 345                 350

Ser Ser Ser Pro Asp Ile Tyr Asn Pro Glu Ala Gly Arg Ile Lys Thr
        355                 360                 365

Val Thr Ser Leu Asp Leu Pro Val Leu Arg Trp Leu Lys Leu Ser Ala
    370                 375                 380

Glu His Gly Ser Leu His Lys Asn Ala Met Phe Val Pro His Tyr Asn
385                 390                 395                 400

Leu Asn Ala Asn Ser Ile Ile Tyr Ala Leu Lys Gly Arg Ala Arg Leu
                405                 410                 415

Gln Val Val Asn Cys Asn Gly Asn Thr Val Phe Asp Gly Glu Leu Glu
            420                 425                 430

Ala Gly Arg Ala Leu Thr Val Pro Gln Asn Tyr Ala Val Ala Ala Lys
        435                 440                 445

Ser Leu Ser Asp Arg Phe Ser Tyr Val Ala Phe Lys Thr Asn Asp Arg
    450                 455                 460

Ala Gly Ile Ala Arg Leu Ala Gly Thr Ser Ser Val Ile Asn Asn Leu
465                 470                 475                 480

Pro Leu Asp Val Val Ala Ala Thr Phe Asn Leu Gln Arg Asn Glu Ala
                485                 490                 495

Arg Gln Leu Lys Ser Asn Asn Pro Phe Lys Phe Leu Val Pro Ala Arg
            500                 505                 510

Gln Ser Glu Asn Arg Ala Ser Ala
        515                 520

<210> 4
<211> 566
<212> PRT
<213> NONE

<400> 4

Met Ala Arg His Phe Leu Ser Ser Phe Ser Leu Cys Phe Leu Leu Phe
1               5                   10                  15

Thr Thr Ala Cys Leu Ala His His Ser Glu Ser Asp Arg Phe Asn Gln
            20                  25                  30

Cys Gln Leu Asp Thr Ile Asn Ala Leu Glu Pro Asp His Arg Val Glu
35 40 45

Ser Glu Ala Gly Leu Thr Glu Thr Trp Asn Pro Asn His Pro Glu Leu
50 55 60

Lys Cys Ala Gly Val Ser Leu Ile Arg Arg Thr Ile Asp Pro Asn Gly
65 70 75 80

Leu His Leu Pro Ser Tyr Ser Pro Ser Pro Gln Leu Ile Phe Ile Ile
85 90 95

Gln Gly Lys Gly Val Leu Gly Leu Ala Val Pro Gly Cys Pro Glu Thr
100 105 110

Tyr Glu Glu Pro Arg Ser Gln Ser Arg Arg Gln Gln Gln Gln Arg Asp
115 120 125

Ser His Gln Lys Ile Arg Arg Phe Ser Lys Gly Asp Val Ile Ala Ile
130 135 140

Pro Pro Gly Ile Pro Tyr Trp Thr Tyr Asn His Gly His Glu Pro Leu
145 150 155 160

Val Ala Ile Thr Leu Leu Asp Thr Ser Asn Thr Leu Asn Gln Leu Asp
165 170 175

Ser Thr Pro Arg Val Phe Tyr Leu Gly Gly Asn Pro Glu Ile Glu Phe
180 185 190

Pro Glu Thr Gln Gln Lys Gln His Glu Pro Arg Gln Gln Arg Tyr Ser
195 200 205

Phe Leu Val Gly Arg Arg Gly Gly Gln Gln Gln Glu Glu Glu Ser Glu
210 215 220

Glu Gln Asn Glu Gly Asn Ser Val Leu Ser Gly Phe Asn Val Glu Phe
225 230 235 240

Leu Ala His Ser Leu Asn Thr Lys Glu Asp Thr Ala Lys Arg Leu Arg
245 250 255

Ser Pro Gln Asp Glu Arg Gly Gln Ile Val Lys Val Glu Asp Gly Leu
260 265 270

His Ile Ile Ser Pro Glu Leu Gln Glu Glu Glu Glu Gln Ser His Ser

275 280 285

Gln Arg Lys Glu Glu Glu Glu Glu Glu Gln Glu Gln Arg His Arg Lys
290 295 300

His Ser Lys Lys Glu Asp Glu Asp Glu Asp Glu Glu Glu Glu Glu Glu
305 310 315 320

Arg Glu Gln Arg His Arg Lys His Ser Glu Lys Glu Glu Glu Asp Glu
325 330 335

Asp Glu Pro Arg Ser Tyr Glu Thr Arg Arg Lys Trp Lys Lys His Thr
340 345 350

Ala Glu Lys Glu Arg Glu Ser His Gly Gln Gly Glu Glu Glu Glu Glu
355 360 365

Leu Glu Lys Glu Glu Glu Glu Glu Glu Gly Ile Gln Arg Gln His Ser
370 375 380

Lys Gly Arg Lys Asn Gly Leu Glu Glu Thr Ile Cys Ser Ala Lys Ile
385 390 395 400

Arg Glu Asn Ile Ala Arg Pro Ser Arg Gly Asp Leu Tyr Asn Ser Gly
405 410 415

Ala Gly Arg Ile Ser Thr Val Asn Ser Leu Thr Leu Pro Ile Leu Arg
420 425 430

Asn Leu Arg Leu Ser Ala Glu Tyr Val Leu Leu Tyr Arg Asn Gly Ile
435 440 445

Tyr Ala Pro His Trp Asn Ile Asn Ala Asn Ser Leu Leu Tyr Val Ile
450 455 460

Arg Gly Glu Gly Arg Val Arg Ile Val Asn Ser Glu Gly Asn Lys Val
465 470 475 480

Phe Asp Asp Lys Val Ser Leu Gly Gln Leu Val Val Val Pro Gln Asn
485 490 495

Phe Val Val Ala Gln Gln Ala Gly Asn Glu Glu Gly Phe Glu Tyr Val
500 505 510

Val Phe Lys Thr Asn Asp Arg Ala Ala Val Ser His Val Asn Gln Val
515 520 525

Phe Arg Ala Thr Pro Gly Glu Val Leu Ala Asn Ala Phe Gly Leu Arg
    530                 535                 540

His Ser Gln Val Ala Gln Ile Lys Ser Asn Gly Asn Arg Gly Pro Leu
545                 550                 555                 560

Val Gln Pro Gln Ser Gln
                565

<210> 5
<211> 517
<212> PRT
<213> NONE

<400> 5

Met Ala Lys Leu Leu Ala Leu Ser Leu Ser Phe Cys Phe Leu Leu Leu
1               5                   10                  15

Gly Gly Cys Phe Ala Leu Arg Glu Gln Pro Gln Gln Asn Glu Cys Gln
            20                  25                  30

Leu Glu Arg Leu Asp Ala Leu Glu Pro Asp Asn Arg Ile Glu Ser Glu
        35                  40                  45

Gly Gly Leu Ile Glu Thr Trp Asn Pro Asn Asn Lys Gln Phe Arg Cys
    50                  55                  60

Ala Gly Val Ala Leu Ser Arg Ala Thr Leu Gln Arg Asn Ala Leu Arg
65                  70                  75                  80

Arg Pro Tyr Tyr Ser Asn Ala Pro Gln Glu Ile Phe Ile Gln Gln Gly
                85                  90                  95

Asn Gly Tyr Phe Gly Met Val Phe Pro Gly Cys Pro Glu Thr Phe Glu
            100                 105                 110

Glu Pro Gln Glu Ser Glu Gln Gly Glu Gly Arg Arg Tyr Arg Asp Arg
        115                 120                 125

His Gln Lys Val Asn Arg Phe Arg Glu Gly Asp Ile Ile Ala Val Pro
    130                 135                 140

Thr Gly Ile Val Phe Trp Met Tyr Asn Asp Gln Asp Thr Pro Val Ile
145                 150                 155                 160

Ala Val Ser Leu Thr Asp Ile Arg Ser Ser Asn Asn Gln Leu Asp Gln
                165                 170                 175

```
Met Pro Arg Arg Phe Tyr Leu Ala Gly Asn His Glu Gln Glu Phe Leu
            180                 185                 190

Gln Tyr Gln His Gln Gln Gly Gly Lys Gln Glu Gln Glu Asn Glu Gly
        195                 200                 205

Asn Asn Ile Phe Ser Gly Phe Lys Arg Asp Phe Leu Glu Asp Ala Phe
    210                 215                 220

Asn Val Asn Arg His Ile Val Asp Arg Leu Gln Gly Arg Asn Glu Asp
225                 230                 235                 240

Glu Glu Lys Gly Ala Ile Val Lys Val Lys Gly Gly Leu Ser Ile Ile
                245                 250                 255

Ser Pro Pro Glu Lys Gln Ala Arg His Gln Arg Gly Ser Arg Gln Glu
            260                 265                 270

Glu Asp Glu Asp Glu Glu Lys Gln Pro Arg His Gln Arg Gly Ser Arg
        275                 280                 285

Gln Glu Glu Glu Glu Asp Glu Asp Glu Glu Arg Gln Pro Arg His Gln
    290                 295                 300

Arg Arg Arg Gly Glu Glu Glu Glu Glu Asp Lys Lys Glu Arg Gly Gly
305                 310                 315                 320

Ser Gln Lys Gly Lys Ser Arg Arg Gln Gly Asp Asn Gly Leu Glu Glu
                325                 330                 335

Thr Val Cys Thr Ala Lys Leu Arg Leu Asn Ile Gly Pro Ser Ser Ser
            340                 345                 350

Pro Asp Ile Tyr Asn Pro Glu Ala Gly Arg Ile Lys Thr Val Thr Ser
        355                 360                 365

Leu Asp Leu Pro Val Leu Arg Trp Leu Lys Leu Ser Ala Glu His Gly
    370                 375                 380

Ser Leu His Lys Asn Ala Met Phe Val Pro His Tyr Asn Leu Asn Ala
385                 390                 395                 400

Asn Ser Ile Ile Tyr Ala Leu Lys Gly Arg Ala Arg Leu Gln Val Val
                405                 410                 415

Asn Cys Asn Gly Asn Thr Val Phe Asp Gly Glu Leu Glu Ala Gly Arg
            420                 425                 430
```

Ala Leu Thr Val Pro Gln Asn Tyr Ala Val Ala Ala Lys Ser Leu Ser
435 440 445

Asp Arg Phe Ser Tyr Val Ala Phe Lys Thr Asn Asp Arg Ala Gly Ile
450 455 460

Ala Arg Leu Ala Gly Thr Ser Ser Val Ile Asn Asn Leu Pro Leu Asp
465 470 475 480

Val Val Ala Ala Thr Phe Asn Leu Gln Arg Asn Glu Ala Arg Gln Leu
485 490 495

Lys Ser Asn Asn Pro Phe Lys Phe Leu Val Pro Ala Arg Glu Ser Glu
500 505 510

Asn Arg Ala Ser Ala
515

<210> 6
<211> 216
<212> PRT
<213> NONE

<400> 6

Lys Gly Gly Leu Arg Ile Ile Ser Pro Pro Glu Lys Gln Ala Arg His
1 5 10 15

Gln Arg Gly Ser Gln Lys Gly Lys Ser Arg Arg Gln Gly Asp Asn Gly
20 25 30

Leu Glu Glu Thr Val Cys Thr Ala Lys Leu Arg Leu Asn Ile Gly Pro
35 40 45

Ser Ser Ser Pro Asp Ile Tyr Asn Pro Glu Ala Gly Arg Ile Lys Thr
50 55 60

Val Thr Ser Leu Asp Leu Pro Val Leu Arg Trp Leu Lys Leu Ser Ala
65 70 75 80

Glu His Gly Ser Leu His Lys Asn Thr Met Phe Val Pro His Tyr Asn
85 90 95

Leu Asn Ala Asn Ser Ile Ile Tyr Ala Leu Lys Gly Arg Ala Arg Leu
100 105 110

Gln Val Val Asn Cys Asn Gly Asn Thr Val Phe Asp Gly Lys Leu Glu
115 120 125

Ala Gly Arg Ala Leu Thr Val Pro Gln Asn Tyr Ala Val Ala Ala Lys
130 135 140

Ser Leu Asn Asp Arg Phe Ser Tyr Val Ala Phe Lys Thr Asn Asp Arg
145 150 155 160

Ala Gly Ile Ala Arg Leu Ala Gly Thr Ser Ser Val Ile Asn Asp Leu
165 170 175

Pro Leu Asp Val Val Ala Ala Thr Phe Lys Leu Gln Arg Asp Glu Ala
180 185 190

Arg Gln Leu Lys Ser Asn Asn Pro Phe Lys Phe Leu Val Pro Ala Arg
195 200 205

Gln Ser Glu Asn Arg Ala Ser Ala
210 215

<210> 7
<211> 338
<212> PRT
<213> NONE

<400> 7

Gly Asn Ser Val Leu Ser Gly Phe Asn Val Glu Phe Leu Ala His Ser
1 5 10 15

Leu Asn Thr Lys Glu Asp Thr Ala Lys Arg Leu Arg Ser Pro Gln Asp
20 25 30

Glu Arg Gly Gln Ile Val Lys Val Glu Asp Gly Leu His Ile Ile Ser
35 40 45

Pro Glu Leu Gln Glu Glu Glu Glu Gln Ser His Ser Gln Arg Lys Glu
50 55 60

Glu Glu Glu Glu Glu Gln Glu Gln Arg His Arg Lys His Ser Lys Lys
65 70 75 80

Glu Asp Glu Asp Glu Asp Glu Glu Glu Glu Glu Glu Arg Glu Gln Arg
85 90 95

His Arg Lys His Ser Glu Lys Glu Glu Glu Asp Glu Asp Glu Pro Arg
100 105 110

Ser Tyr Glu Thr Arg Arg Lys Trp Lys Lys His Thr Ala Glu Lys Lys
115 120 125

Arg Glu Ser His Gly Gln Gly Glu Glu Glu Glu Glu Leu Glu Lys Glu
    130                 135                 140

Glu Glu Glu Glu Glu Glu Ile Gln Arg Gln His Ser Lys Gly Arg Lys
145                 150                 155                 160

Asn Gly Leu Glu Glu Thr Ile Cys Ser Ala Lys Ile Arg Glu Asn Ile
                165                 170                 175

Ala Arg Pro Ser Arg Gly Asp Leu Tyr Asn Ser Gly Ala Gly Arg Ile
            180                 185                 190

Ser Thr Val Asn Ser Leu Thr Leu Pro Ile Leu Arg Asn Leu Arg Leu
        195                 200                 205

Ser Ala Glu Tyr Val Leu Leu Tyr Arg Asn Gly Ile Tyr Ala Pro His
    210                 215                 220

Trp Asn Ile Asn Ala Asn Ser Leu Leu Tyr Val Ile Arg Gly Glu Gly
225                 230                 235                 240

Arg Val Arg Ile Val Asn Ser Glu Gly Asn Lys Val Phe Asp Asp Lys
                245                 250                 255

Val Ser Leu Gly Gln Leu Val Val Val Pro Gln Asn Phe Val Val Ala
            260                 265                 270

Gln Gln Ala Gly Asn Glu Glu Gly Phe Glu Tyr Val Val Phe Lys Thr
        275                 280                 285

Asn Asp Arg Ala Ala Val Ser His Val Asn Gln Val Phe Arg Ala Thr
    290                 295                 300

Pro Gly Glu Val Leu Ala Asn Ala Phe Gly Leu Arg His Ser Gln Val
305                 310                 315                 320

Ala Gln Ile Lys Ser Asn Gly Asn Arg Gly Pro Leu Val Gln Pro Gln
                325                 330                 335

Ser Gln

<210> 8
<211> 350
<212> PRT
<213> NONE

<400> 8

```
Ile Pro Tyr Trp Thr Tyr Asn His Gly Asp Glu Pro Leu Val Ala Ile
1               5                   10                  15

Ser Leu Leu Asp Thr Ser Asn Ile Ala Asn Gln Leu Asp Ser Thr Pro
            20                  25                  30

Arg Val Phe Tyr Leu Gly Gly Asn Pro Glu Thr Glu Phe Pro Glu Thr
        35                  40                  45

Gln Glu Glu Gln Gln Gly Arg His Arg Gln Lys His Ser Tyr Pro Val
    50                  55                  60

Gly Arg Arg Ser Gly His His Gln Gln Glu Glu Glu Ser Glu Glu Gln
65                  70                  75                  80

Asn Glu Gly Asn Ser Val Leu Ser Gly Val Ser Ser Glu Phe Leu Ala
                85                  90                  95

Gln Thr Phe Asn Thr Glu Glu Asp Thr Ala Lys Arg Leu Arg Ser Pro
            100                 105                 110

Arg Asp Glu Arg Ser Gln Ile Val Arg Val Glu Gly Gly Leu Arg Ile
        115                 120                 125

Ile Asn Pro Lys Gly Lys Glu Glu Glu Glu Glu Lys Glu Gln Ser His
    130                 135                 140

Ser His Ser His Arg Glu Glu Glu Glu Glu Glu Glu Glu Asp Glu Glu
145                 150                 155                 160

Lys Gln Arg Ser Glu Glu Arg Lys Asn Gly Leu Glu Glu Thr Ile Cys
                165                 170                 175

Ser Ala Lys Ile Arg Glu Asn Ile Ala Asp Ala Ala Gly Ala Asp Leu
            180                 185                 190

Tyr Asn Pro Arg Ala Gly Arg Ile Arg Thr Ala Asn Ser Leu Thr Leu
        195                 200                 205

Pro Val Leu Arg Tyr Leu Arg Leu Ser Ala Glu Tyr Val Arg Leu Tyr
    210                 215                 220

Arg Asn Gly Ile Tyr Ala Pro His Trp Asn Ile Asn Ala Asn Ser Leu
225                 230                 235                 240

Leu Tyr Val Ile Arg Gly Glu Gly Arg Val Arg Ile Val Asn Phe Gln
                245                 250                 255
```

Gly Asp Ala Val Phe Asp Asn Lys Val Arg Lys Gly Gln Leu Val Val
260 265 270

Val Pro Gln Asn Phe Val Val Ala Glu Gln Ala Gly Glu Glu Glu Gly
275 280 285

Leu Glu Tyr Val Val Phe Lys Thr Asn Asp Arg Ala Ala Val Ser His
290 295 300

Val Gln Gln Val Leu Arg Ala Thr Pro Ala Glu Val Leu Ala Asn Ala
305 310 315 320

Phe Gly Leu Arg Gln Arg Gln Val Thr Glu Leu Lys Leu Ser Gly Asn
325 330 335

Arg Gly Pro Leu Val His Pro Gln Ser Gln Ser Gln Ser His
340 345 350

<210> 9
<211> 459
<212> PRT
<213> NONE

<400> 9

Met Ala Ala Thr Thr Met Lys Ala Ser Phe Pro Leu Leu Met Leu Met
1 5 10 15

Gly Ile Ser Phe Leu Ala Ser Val Cys Val Ser Ser Arg Ser Asp Pro
20 25 30

Gln Asn Pro Phe Ile Phe Lys Ser Asn Lys Phe Gln Thr Leu Phe Glu
35 40 45

Asn Glu Asn Gly His Ile Arg Leu Leu Gln Lys Phe Asp Gln Arg Ser
50 55 60

Lys Ile Phe Glu Asn Leu Gln Asn Tyr Arg Leu Leu Glu Tyr Lys Ser
65 70 75 80

Lys Pro His Thr Ile Phe Leu Pro Gln His Thr Asp Ala Asp Tyr Ile
85 90 95

Leu Val Val Leu Ser Gly Lys Ala Ile Leu Thr Val Leu Lys Pro Asp
100 105 110

Asp Arg Asn Ser Phe Asn Leu Glu Arg Gly Asp Thr Ile Lys Leu Pro
115 120 125

```
Ala Gly Thr Ile Ala Tyr Leu Val Asn Arg Asp Asp Asn Glu Glu Leu
    130                 135                 140

Arg Val Leu Asp Leu Ala Ile Pro Val Asn Arg Pro Gly Gln Leu Gln
145                 150                 155                 160

Ser Phe Leu Leu Ser Gly Asn Gln Asn Gln Gln Asn Tyr Leu Ser Gly
                165                 170                 175

Phe Ser Lys Asn Ile Leu Glu Ala Ser Phe Asn Thr Asp Tyr Glu Glu
            180                 185                 190

Ile Glu Lys Val Leu Leu Glu Glu His Glu Lys Glu Thr Gln His Arg
        195                 200                 205

Arg Ser Leu Lys Asp Lys Arg Gln Gln Ser Gln Glu Glu Asn Val Ile
    210                 215                 220

Val Lys Leu Ser Arg Gly Gln Ile Glu Glu Leu Ser Lys Asn Ala Lys
225                 230                 235                 240

Ser Thr Ser Lys Lys Ser Val Ser Ser Glu Ser Glu Pro Phe Asn Leu
                245                 250                 255

Arg Ser Arg Gly Pro Ile Tyr Ser Asn Glu Phe Gly Lys Phe Phe Glu
            260                 265                 270

Ile Thr Pro Glu Lys Asn Pro Gln Leu Gln Asp Leu Asp Ile Phe Val
        275                 280                 285

Asn Ser Val Glu Ile Lys Glu Gly Ser Leu Leu Leu Pro His Tyr Asn
    290                 295                 300

Ser Arg Ala Ile Val Ile Val Thr Val Asn Glu Gly Lys Gly Asp Phe
305                 310                 315                 320

Glu Leu Val Gly Gln Arg Asn Glu Asn Gln Gln Glu Gln Arg Lys Glu
                325                 330                 335

Asp Asp Glu Glu Glu Glu Gln Gly Glu Glu Glu Ile Asn Lys Gln Val
            340                 345                 350

Gln Asn Tyr Lys Ala Lys Leu Ser Ser Gly Asp Val Phe Val Ile Pro
        355                 360                 365

Ala Gly His Pro Val Ala Val Lys Ala Ser Ser Asn Leu Asp Leu Leu
```

```
    370                 375                 380

Gly Phe Gly Ile Asn Ala Glu Asn Asn Gln Arg Asn Phe Leu Ala Gly
385                 390                 395                 400

Asp Glu Asp Asn Val Ile Ser Gln Ile Gln Arg Pro Val Lys Glu Leu
                405                 410                 415

Ala Phe Pro Gly Ser Ala Gln Glu Val Asp Arg Ile Leu Glu Asn Gln
            420                 425                 430

Lys Gln Ser His Phe Ala Asp Ala Gln Pro Gln Gln Arg Glu Arg Gly
        435                 440                 445

Ser Arg Glu Thr Arg Asp Arg Leu Ser Ser Val
    450                 455


<210>  10
<211>  438
<212>  PRT
<213>  NONE

<400>  10

Met Ala Ala Thr Pro Ile Lys Pro Leu Met Leu Leu Ala Ile Ala Phe
1               5                   10                  15

Leu Ala Ser Val Cys Val Ser Ser Arg Ser Asp Gln Glu Asn Pro Phe
            20                  25                  30

Ile Phe Lys Ser Asn Arg Phe Gln Thr Leu Tyr Glu Asn Glu Asn Gly
        35                  40                  45

His Ile Arg Leu Leu Gln Lys Phe Asp Lys Arg Ser Lys Ile Phe Glu
    50                  55                  60

Asn Leu Gln Asn Tyr Arg Leu Leu Glu Tyr Lys Ser Lys Pro His Thr
65                  70                  75                  80

Leu Phe Leu Pro Gln Tyr Thr Asp Ala Asp Phe Ile Leu Val Val Leu
                85                  90                  95

Ser Gly Lys Ala Thr Leu Thr Val Leu Lys Ser Asn Asp Arg Asn Ser
            100                 105                 110

Phe Asn Leu Glu Arg Gly Asp Ala Ile Lys Leu Pro Ala Gly Thr Ile
        115                 120                 125

Ala Tyr Leu Ala Asn Arg Asp Asp Asn Glu Asp Leu Arg Val Leu Asp
```

130 135 140

Leu Ala Ile Pro Val Asn Lys Pro Gly Gln Leu Gln Ser Phe Leu Leu
145 150 155 160

Ser Gly Thr Gln Asn Gln Pro Ser Leu Leu Ser Gly Phe Ser Lys Asn
165 170 175

Ile Leu Glu Ala Ala Phe Asn Thr Asn Tyr Glu Glu Ile Glu Lys Val
180 185 190

Leu Leu Glu Gln Gln Glu Gln Glu Pro Gln His Arg Arg Ser Leu Lys
195 200 205

Asp Arg Arg Gln Glu Ile Asn Glu Glu Asn Val Ile Val Lys Val Ser
210 215 220

Arg Glu Gln Ile Glu Glu Leu Ser Lys Asn Ala Lys Ser Ser Ser Lys
225 230 235 240

Lys Ser Val Ser Ser Glu Ser Gly Pro Phe Asn Leu Arg Ser Arg Asn
245 250 255

Pro Ile Tyr Ser Asn Lys Phe Gly Lys Phe Phe Glu Ile Thr Pro Glu
260 265 270

Lys Asn Gln Gln Leu Gln Asp Leu Asp Ile Phe Val Asn Ser Val Asp
275 280 285

Ile Lys Glu Gly Ser Leu Leu Leu Pro Asn Tyr Asn Ser Arg Ala Ile
290 295 300

Val Ile Val Thr Val Thr Glu Gly Lys Gly Asp Phe Glu Leu Val Gly
305 310 315 320

Gln Arg Asn Glu Asn Gln Gly Lys Glu Asn Asp Lys Glu Glu Glu Gln
325 330 335

Glu Glu Glu Thr Ser Lys Gln Val Gln Leu Tyr Arg Ala Lys Leu Ser
340 345 350

Pro Gly Asp Val Phe Val Ile Pro Ala Gly His Pro Val Ala Ile Asn
355 360 365

Ala Ser Ser Asp Leu Asn Leu Ile Gly Phe Gly Ile Asn Ala Glu Asn
370 375 380

Asn Glu Arg Asn Phe Leu Ala Gly Glu Glu Asp Asn Val Ile Ser Gln
385 390 395 400

Val Glu Arg Pro Val Lys Glu Leu Ala Phe Pro Gly Ser Ser His Glu
405 410 415

Val Asp Arg Leu Leu Lys Asn Gln Lys Gln Ser Tyr Phe Ala Asn Ala
420 425 430

Gln Pro Leu Gln Arg Glu
435

<210> 11
<211> 437
<212> PRT
<213> NONE

<400> 11

Met Ala Ala Thr Pro Ile Lys Pro Leu Met Leu Leu Ala Ile Ala Phe
1 5 10 15

Leu Ala Ser Val Cys Val Ser Ser Arg Ser Asp Gln Glu Asn Pro Phe
20 25 30

Ile Phe Lys Ser Asn Arg Phe Gln Thr Leu Tyr Glu Asn Glu Asn Gly
35 40 45

His Ile Arg Leu Leu Gln Lys Phe Asp Lys Arg Ser Lys Ile Phe Glu
50 55 60

Asn Leu Gln Asn Tyr Arg Leu Leu Glu Tyr Lys Ser Lys Pro Arg Thr
65 70 75 80

Leu Phe Leu Pro Gln Tyr Thr Asp Ala Asp Phe Ile Leu Val Val Leu
85 90 95

Ser Gly Lys Ala Thr Leu Thr Val Leu Lys Ser Asn Asp Arg Asn Ser
100 105 110

Phe Asn Leu Glu Arg Gly Asp Thr Ile Lys Leu Pro Ala Gly Thr Ile
115 120 125

Ala Tyr Leu Ala Asn Arg Asp Asp Asn Glu Asp Leu Arg Val Leu Asp
130 135 140

Leu Ala Ile Pro Val Asn Lys Pro Gly Gln Leu Gln Ser Phe Leu Leu
145 150 155 160

```
Ser Gly Thr Gln Asn Gln Pro Ser Leu Leu Ser Gly Phe Ser Lys Asn
                165                 170                 175

Ile Leu Glu Ala Ala Phe Asn Thr Asn Tyr Glu Glu Ile Glu Lys Val
            180                 185                 190

Leu Leu Glu Gln Gln Glu Gln Glu Pro Gln His Arg Ser Leu Lys Asp
        195                 200                 205

Arg Arg Gln Glu Ile Asn Glu Glu Asn Val Ile Val Lys Val Ser Arg
    210                 215                 220

Glu Gln Ile Glu Glu Leu Ser Lys Asn Ala Lys Ser Ser Ser Lys Lys
225                 230                 235                 240

Ser Val Ser Ser Glu Ser Gly Pro Phe Asn Leu Arg Ser Arg Asn Pro
                245                 250                 255

Ile Tyr Ser Asn Lys Phe Gly Lys Phe Phe Glu Ile Thr Pro Glu Lys
            260                 265                 270

Asn Gln Gln Leu Gln Asp Leu Asp Ile Phe Val Asn Ser Val Asp Ile
        275                 280                 285

Lys Glu Gly Ser Leu Leu Leu Pro Asn Tyr Asn Ser Arg Ala Ile Val
    290                 295                 300

Ile Val Thr Val Thr Glu Gly Lys Gly Asp Phe Glu Leu Val Gly Gln
305                 310                 315                 320

Arg Asn Glu Asn Gln Gly Lys Glu Asn Asp Lys Glu Glu Glu Gln Glu
                325                 330                 335

Glu Glu Thr Ser Lys Gln Val Gln Leu Tyr Arg Ala Lys Leu Ser Pro
            340                 345                 350

Gly Asp Val Phe Val Ile Pro Ala Gly His Pro Val Ala Ile Asn Ala
        355                 360                 365

Ser Ser Asp Leu Asn Leu Ile Gly Phe Gly Ile Asn Ala Glu Asn Asn
    370                 375                 380

Glu Arg Asn Phe Leu Ala Gly Glu Glu Asp Asn Val Ile Ser Gln Val
385                 390                 395                 400

Glu Arg Pro Val Lys Glu Leu Ala Phe Pro Gly Ser Ser His Glu Val
                405                 410                 415
```

Asp Arg Leu Leu Lys Asn Gln Lys Gln Ser Tyr Phe Ala Asn Ala Gln
420 425 430

Pro Leu Gln Arg Glu
435

<210> 12
<211> 441
<212> PRT
<213> NONE

<400> 12

Met Ala Ala Thr Pro Met Lys Ala Ser Phe Pro Leu Leu Met Leu Met
1 5 10 15

Gly Ile Ser Phe Leu Ala Ser Val Cys Val Ser Ser Arg Ser Asp Pro
20 25 30

Gln Asn Pro Phe Ile Phe Lys Ser Asn Lys Phe Gln Thr Leu Phe Glu
35 40 45

Asn Glu Asn Gly His Ile Arg Leu Leu Gln Lys Phe Asp Gln Arg Ser
50 55 60

Lys Ile Phe Glu Asn Leu Gln Asn Tyr Arg Leu Leu Glu Tyr Lys Ser
65 70 75 80

Lys Pro Arg Thr Leu Phe Leu Pro Gln Tyr Thr Asp Ala Asp Phe Ile
85 90 95

Leu Val Val Leu Ser Gly Lys Ala Thr Leu Thr Val Leu Lys Ser Asn
100 105 110

Asp Arg Asn Ser Phe Asn Leu Glu Arg Gly Asp Thr Ile Lys Leu Pro
115 120 125

Ala Gly Thr Ile Ala Tyr Leu Ala Asn Arg Asp Asp Asn Glu Asp Leu
130 135 140

Arg Val Leu Asp Leu Ala Ile Pro Val Asn Lys Pro Gly Gln Leu Gln
145 150 155 160

Ser Phe Leu Leu Ser Gly Thr Gln Asn Gln Pro Ser Leu Leu Ser Gly
165 170 175

Phe Ser Lys Asn Ile Leu Glu Ala Ala Phe Asn Thr Asn Tyr Glu Glu
180 185 190

```
Ile Glu Lys Val Leu Leu Glu Gln Gln Glu Gln Glu Pro Gln His Arg
        195                 200                 205

Ser Leu Lys Asp Arg Arg Gln Glu Ile Asn Glu Glu Asn Val Ile Val
    210                 215                 220

Lys Val Ser Arg Glu Gln Ile Glu Glu Leu Ser Lys Asn Ala Lys Ser
225                 230                 235                 240

Ser Ser Lys Lys Ser Val Ser Ser Glu Ser Gly Pro Phe Asn Leu Arg
                245                 250                 255

Ser Arg Asn Pro Ile Tyr Ser Asn Lys Phe Gly Lys Phe Phe Glu Ile
            260                 265                 270

Thr Pro Glu Lys Asn Gln Gln Leu Gln Asp Leu Asp Ile Phe Val Asn
        275                 280                 285

Ser Val Asp Ile Lys Glu Gly Ser Leu Leu Leu Pro Asn Tyr Asn Ser
    290                 295                 300

Arg Ala Ile Val Ile Val Thr Val Thr Glu Gly Lys Gly Asp Phe Glu
305                 310                 315                 320

Leu Val Gly Gln Arg Asn Glu Asn Gln Gly Lys Glu Asn Asp Lys Glu
                325                 330                 335

Glu Glu Gln Glu Glu Glu Thr Ser Lys Gln Val Gln Leu Tyr Arg Ala
            340                 345                 350

Lys Leu Ser Pro Gly Asp Val Phe Val Ile Pro Ala Gly His Pro Val
        355                 360                 365

Ala Ile Asn Ala Ser Ser Asp Leu Asn Leu Ile Gly Phe Gly Ile Asn
    370                 375                 380

Ala Glu Asn Asn Glu Arg Asn Phe Leu Ala Gly Glu Glu Asp Asn Val
385                 390                 395                 400

Ile Ser Gln Val Glu Arg Pro Val Lys Glu Leu Ala Phe Pro Gly Ser
                405                 410                 415

Ser His Glu Val Asp Arg Leu Leu Lys Asn Gln Lys Gln Ser Tyr Phe
            420                 425                 430

Ala Asn Ala Gln Pro Leu Gln Arg Glu
        435                 440
```

<210> 13
<211> 438
<212> PRT
<213> NONE

<400> 13

Met Ala Ala Thr Pro Ile Lys Pro Leu Met Leu Leu Ala Ile Ala Phe
1               5                   10                  15

Leu Ala Ser Val Cys Val Ser Ser Arg Ser Asp Gln Glu Asn Pro Phe
            20                  25                  30

Ile Phe Lys Ser Asn Arg Phe Gln Thr Leu Tyr Glu Asn Glu Asn Gly
        35                  40                  45

His Ile Arg Leu Leu Gln Lys Phe Asp Lys Arg Ser Lys Ile Phe Glu
    50                  55                  60

Asn Leu Gln Asn Tyr Arg Leu Leu Glu Tyr Lys Ser Lys Pro His Thr
65                  70                  75                  80

Leu Phe Leu Pro Gln Tyr Thr Asp Ala Asp Phe Ile Leu Val Val Leu
                85                  90                  95

Ser Gly Lys Ala Thr Leu Thr Val Leu Lys Ser Asn Asp Arg Asn Ser
            100                 105                 110

Phe Asn Leu Glu Arg Gly Asp Ala Ile Lys Leu Pro Ala Gly Thr Ile
        115                 120                 125

Ala Tyr Leu Ala Asn Arg Asp Asp Asn Glu Asp Leu Arg Val Leu Asp
    130                 135                 140

Leu Ala Ile Pro Val Asn Lys Pro Gly Gln Leu Gln Ser Phe Leu Leu
145                 150                 155                 160

Ser Gly Thr Gln Asn Gln Pro Ser Leu Leu Ser Gly Phe Ser Lys Asn
                165                 170                 175

Ile Leu Glu Ala Ala Phe Asn Thr Asp Tyr Glu Glu Ile Glu Lys Val
            180                 185                 190

Leu Leu Glu Glu His Glu Lys Glu Thr Gln His Arg Arg Ser Leu Lys
        195                 200                 205

Asp Lys Arg Gln Gln Ser Gln Glu Glu Asn Val Ile Val Lys Leu Ser
    210                 215                 220

Arg Gly Gln Ile Glu Glu Leu Ser Lys Asn Ala Lys Ser Thr Ser Lys
225 230 235 240

Lys Gly Val Ser Ser Glu Ser Glu Pro Phe Asn Leu Arg Ser Arg Gly
245 250 255

Pro Ile Tyr Ser Asn Glu Phe Gly Lys Phe Phe Glu Ile Thr Pro Gly
260 265 270

Lys Asn Gln Gln Leu Gln Asp Leu Asp Ile Phe Val Asn Ser Val Asp
275 280 285

Ile Lys Glu Gly Ser Leu Leu Leu Pro Asn Tyr Asn Ser Arg Ala Ile
290 295 300

Val Ile Val Thr Val Thr Glu Gly Lys Gly Asp Phe Glu Leu Val Gly
305 310 315 320

Gln Arg Asn Glu Asn Gln Gly Lys Glu Asn Asp Lys Glu Glu Glu Gln
325 330 335

Glu Glu Glu Thr Ser Lys Gln Val Gln Leu Tyr Arg Ala Lys Leu Ser
340 345 350

Pro Gly Asp Val Phe Val Ile Pro Ala Gly His Pro Val Ala Ile Asn
355 360 365

Ala Ser Ser Asp Leu Asn Leu Ile Gly Phe Gly Ile Asn Ala Glu Asn
370 375 380

Asn Glu Arg Asn Phe Leu Ala Gly Glu Glu Asp Asn Val Ile Ser Gln
385 390 395 400

Val Glu Arg Pro Val Lys Glu Leu Ala Phe Pro Gly Ser Ser His Glu
405 410 415

Val Asp Arg Leu Leu Lys Asn Gln Lys Gln Ser Tyr Phe Ala Asn Ala
420 425 430

Gln Pro Leu Gln Arg Glu
435

<210> 14
<211> 438
<212> PRT
<213> NONE

<400> 14

Met Ala Ala Thr Pro Ile Lys Pro Leu Met Leu Leu Ala Ile Ala Phe
1 5 10 15

Leu Ala Ser Val Cys Val Ser Ser Arg Ser Asp Gln Glu Asn Pro Phe
20 25 30

Ile Phe Lys Ser Asn Arg Phe Gln Thr Leu Tyr Glu Asn Glu Asn Gly
35 40 45

His Ile Arg Leu Leu Gln Lys Phe Asp Lys Arg Ser Lys Ile Phe Glu
50 55 60

Asn Leu Gln Asn Tyr Arg Leu Leu Glu Tyr Lys Ser Lys Pro Arg Thr
65 70 75 80

Leu Phe Leu Pro Gln Tyr Thr Asp Ala Asp Phe Ile Leu Val Val Leu
85 90 95

Ser Gly Lys Ala Thr Leu Thr Val Leu Lys Ser Asn Asp Arg Asn Ser
100 105 110

Phe Asn Leu Glu Arg Gly Asp Thr Ile Lys Leu Pro Ala Gly Thr Ile
115 120 125

Ala Tyr Leu Ala Asn Arg Asp Asp Asn Glu Asp Leu Arg Val Leu Asp
130 135 140

Leu Thr Ile Pro Val Asn Lys Pro Gly Gln Leu Gln Ser Phe Leu Leu
145 150 155 160

Ser Gly Thr Gln Asn Gln Pro Ser Leu Leu Ser Gly Phe Ser Lys Asn
165 170 175

Ile Leu Glu Ala Ala Phe Asn Thr Asn Tyr Glu Glu Ile Glu Lys Val
180 185 190

Leu Leu Glu Gln Gln Glu Gln Glu Pro Gln His Arg Arg Ser Leu Lys
195 200 205

Asp Arg Arg Gln Glu Ile Asn Glu Glu Asn Val Ile Val Lys Val Ser
210 215 220

Arg Glu Gln Ile Glu Glu Leu Ser Lys Asn Ala Lys Ser Ser Ser Lys
225 230 235 240

Lys Ser Val Ser Ser Glu Ser Gly Pro Phe Asn Leu Arg Ser Arg Asn

245 250 255

Pro Ile Tyr Ser Asn Lys Phe Gly Lys Phe Phe Glu Ile Thr Pro Glu
260 265 270

Lys Asn Gln Gln Leu Gln Asp Leu Asp Ile Phe Val Asn Ser Val Asp
275 280 285

Ile Lys Glu Gly Ser Leu Leu Leu Pro Asn Tyr Asn Ser Arg Ala Ile
290 295 300

Val Ile Val Thr Val Thr Glu Gly Lys Gly Asp Phe Glu Leu Val Gly
305 310 315 320

Gln Arg Asn Glu Asn Gln Gly Lys Glu Asn Asp Lys Glu Glu Glu Gln
325 330 335

Glu Glu Glu Thr Ser Lys Gln Val Gln Leu Tyr Arg Ala Lys Leu Ser
340 345 350

Pro Gly Asp Val Phe Val Ile Pro Ala Gly His Pro Val Ala Ile Asn
355 360 365

Ala Ser Ser Asp Leu Asn Leu Ile Gly Phe Gly Ile Asn Ala Glu Asn
370 375 380

Asn Glu Arg Asn Phe Leu Ala Gly Glu Glu Asp Asn Val Ile Ser Gln
385 390 395 400

Val Glu Arg Pro Val Lys Glu Leu Ala Phe Pro Gly Ser Ser His Glu
405 410 415

Val Asp Arg Leu Leu Lys Asn Gln Lys Gln Ser Tyr Phe Ala Asn Ala
420 425 430

Gln Pro Leu Gln Arg Glu
435

<210> 15
<211> 438
<212> PRT
<213> NONE

<400> 15

Met Ala Ala Thr Pro Ile Lys Pro Leu Met Leu Leu Ala Ile Ala Phe
1 5 10 15

Leu Ala Ser Val Cys Val Ser Ser Arg Ser Asp Gln Glu Asn Pro Phe

```
            20                  25                  30

Ile Phe Lys Ser Asn Arg Phe Gln Thr Leu Tyr Glu Asn Glu Asn Gly
        35                  40                  45

His Ile Arg Leu Leu Gln Lys Phe Asp Lys Arg Ser Lys Ile Phe Glu
    50                  55                  60

Asn Leu Gln Asn Tyr Arg Leu Leu Glu Tyr Lys Ser Lys Pro His Thr
65                  70                  75                  80

Leu Phe Leu Pro Gln Tyr Thr Asp Ala Asp Phe Ile Leu Val Val Leu
                85                  90                  95

Asn Gly Lys Ala Thr Leu Thr Val Leu Lys Ser Asn Asp Arg Asn Ser
            100                 105                 110

Phe Asn Leu Glu Arg Gly Asp Thr Ile Lys Leu Pro Ala Gly Thr Ile
        115                 120                 125

Ala Tyr Leu Ala Asn Arg Asp Asp Asn Glu Asp Leu Arg Val Leu Asp
    130                 135                 140

Leu Ala Ile Pro Val Asn Lys Pro Gly Gln Leu Gln Ser Phe Leu Leu
145                 150                 155                 160

Ser Gly Thr Gln Asn Gln Pro Ser Leu Leu Ser Gly Phe Ser Lys Asn
                165                 170                 175

Ile Leu Glu Ala Ala Phe Asn Thr Asn Tyr Glu Glu Ile Glu Lys Val
            180                 185                 190

Leu Leu Glu Gln Gln Glu Gln Glu Pro Gln His Arg Arg Ser Leu Lys
        195                 200                 205

Asp Arg Arg Gln Glu Ile Asn Glu Glu Asn Val Ile Val Lys Val Ser
    210                 215                 220

Arg Glu Gln Ile Glu Glu Leu Ser Lys Asn Ala Lys Ser Ser Ser Lys
225                 230                 235                 240

Lys Ser Val Ser Ser Glu Ser Gly Pro Phe Asn Leu Arg Ser Arg Asn
                245                 250                 255

Pro Ile Tyr Ser Asn Lys Phe Gly Lys Phe Phe Glu Ile Thr Pro Glu
            260                 265                 270
```

Lys Asn Gln Gln Leu Gln Asp Leu Asp Ile Phe Val Asn Ser Val Asp
275 280 285

Ile Lys Glu Gly Ser Leu Leu Leu Pro Asn Tyr Asn Ser Arg Ala Ile
290 295 300

Val Ile Val Thr Val Thr Glu Gly Lys Gly Asp Phe Glu Leu Val Gly
305 310 315 320

Gln Arg Asn Glu Asn Gln Gly Lys Glu Asn Asp Lys Glu Glu Glu Gln
325 330 335

Glu Glu Glu Thr Ser Lys Gln Val Gln Leu Tyr Lys Ala Lys Leu Ser
340 345 350

Pro Gly Asp Val Phe Val Ile Pro Ala Gly His Pro Val Ala Ile Asn
355 360 365

Ala Ser Ser Asp Leu Asn Leu Ile Gly Phe Gly Ile Asn Ala Glu Asn
370 375 380

Asn Glu Arg Asn Phe Leu Ala Gly Glu Glu Asp Asn Val Ile Ser Gln
385 390 395 400

Val Gln Arg Pro Val Lys Glu Leu Ala Phe Pro Gly Ser Ser His Glu
405 410 415

Ile Asp Arg Leu Leu Lys Asn Gln Lys Gln Ser Tyr Phe Ala Asn Ala
420 425 430

Gln Pro Leu Gln Arg Glu
435

<210> 16
<211> 415
<212> PRT
<213> NONE

<400> 16

Ser Arg Ser Asp Gln Glu Asn Pro Phe Ile Phe Lys Ser Asn Arg Phe
1 5 10 15

Gln Thr Leu Tyr Glu Asn Glu Asn Gly His Ile Arg Leu Leu Gln Lys
20 25 30

Phe Asp Lys Arg Ser Lys Ile Phe Glu Asn Leu Gln Asn Tyr Arg Leu
35 40 45

```
Leu Glu Tyr Lys Ser Lys Pro His Thr Leu Phe Leu Pro Gln Tyr Thr
    50                  55                  60

Asp Ala Asp Phe Ile Leu Val Val Leu Ser Gly Lys Ala Thr Leu Thr
65                  70                  75                  80

Val Leu Lys Ser Asn Asp Arg Asn Ser Phe Asn Leu Glu Arg Gly Asp
                85                  90                  95

Ala Ile Lys Leu Pro Ala Gly Thr Ile Ala Tyr Leu Ala Asn Arg Asp
            100                 105                 110

Asp Asn Glu Asp Leu Arg Val Leu Asp Leu Ala Ile Pro Val Asn Lys
        115                 120                 125

Pro Gly Gln Leu Gln Ser Phe Leu Leu Ser Gly Thr Gln Asn Gln Pro
    130                 135                 140

Ser Leu Leu Ser Gly Phe Ser Lys Asn Ile Leu Glu Ala Ala Phe Asn
145                 150                 155                 160

Thr Asn Tyr Glu Glu Ile Glu Lys Val Leu Leu Glu Gln Gln Glu Gln
                165                 170                 175

Glu Pro Gln His Arg Arg Ser Leu Lys Asp Arg Arg Gln Glu Ile Asn
            180                 185                 190

Glu Glu Asn Val Ile Val Lys Val Ser Arg Glu Gln Ile Glu Glu Leu
        195                 200                 205

Ser Lys Asn Ala Lys Ser Ser Ser Lys Lys Ser Val Ser Ser Glu Ser
    210                 215                 220

Gly Pro Phe Asn Leu Arg Ser Arg Asn Pro Ile Tyr Ser Asn Lys Phe
225                 230                 235                 240

Gly Lys Phe Phe Glu Ile Thr Pro Glu Lys Asn Gln Gln Leu Gln Asp
                245                 250                 255

Leu Asp Ile Phe Val Asn Ser Val Asp Ile Lys Glu Gly Ser Leu Leu
            260                 265                 270

Leu Pro Asn Tyr Asn Ser Arg Ala Ile Val Ile Val Thr Val Thr Glu
        275                 280                 285

Gly Lys Gly Asp Phe Glu Leu Val Gly Gln Arg Asn Glu Asn Gln Gly
    290                 295                 300
```

Lys Glu Asn Asp Lys Glu Glu Glu Gln Glu Glu Glu Thr Ser Lys Gln
305 310 315 320

Val Gln Leu Tyr Arg Ala Lys Leu Ser Pro Gly Asp Val Phe Val Ile
325 330 335

Pro Ala Gly His Pro Val Ala Ile Asn Ala Ser Ser Asp Leu Asn Leu
340 345 350

Ile Gly Phe Gly Ile Asn Ala Glu Asn Asn Glu Arg Asn Phe Leu Ala
355 360 365

Gly Glu Glu Asp Asn Val Ile Ser Gln Val Glu Arg Pro Val Lys Glu
370 375 380

Leu Ala Phe Pro Gly Ser Ser His Glu Val Asp Arg Leu Leu Lys Asn
385 390 395 400

Gln Lys Gln Ser Tyr Phe Ala Asn Ala Gln Pro Leu Gln Arg Glu
405 410 415

<210> 17
<211> 415
<212> PRT
<213> NONE

<400> 17

Ser Arg Ser Asp Gln Glu Asn Pro Phe Ile Phe Lys Ser Asn Arg Phe
1 5 10 15

Gln Thr Leu Tyr Glu Asn Glu Asn Gly His Ile Arg Leu Leu Gln Lys
20 25 30

Phe Asp Lys Arg Ser Lys Ile Phe Glu Asn Leu Gln Asn Tyr Arg Leu
35 40 45

Leu Glu Tyr Lys Ser Lys Pro Arg Thr Leu Phe Leu Pro Gln Cys Thr
50 55 60

Asp Ala Asp Phe Ile Leu Val Val Leu Ser Gly Lys Ala Thr Leu Thr
65 70 75 80

Val Leu Lys Ser Asn Asp Arg Asn Ser Phe Asn Leu Glu Arg Gly Asp
85 90 95

Thr Ile Lys Leu Pro Ala Gly Thr Ile Ala Tyr Leu Ala Asn Arg Asp
100 105 110

```
Asp Asn Glu Asp Leu Arg Val Leu Asp Leu Thr Ile Pro Val Asn Lys
        115                 120                 125

Pro Gly Gln Leu Gln Ser Phe Leu Leu Ser Gly Thr Gln Asn Gln Pro
    130                 135                 140

Ser Leu Leu Ser Gly Phe Ser Lys Asn Ile Leu Glu Ala Ala Phe Asn
145                 150                 155                 160

Thr Asn Tyr Glu Glu Ile Glu Lys Val Leu Leu Glu Gln Gln Glu Gln
                165                 170                 175

Glu Pro Gln His Arg Arg Ser Leu Lys Asp Arg Arg Gln Glu Ile Asn
            180                 185                 190

Glu Glu Asn Val Ile Val Lys Val Ser Arg Glu Gln Ile Glu Glu Leu
        195                 200                 205

Ser Lys Asn Ala Lys Ser Ser Ser Lys Lys Ser Val Ser Ser Glu Ser
    210                 215                 220

Gly Pro Phe Asn Leu Arg Ser Arg Asn Pro Ile Tyr Ser Asn Lys Phe
225                 230                 235                 240

Gly Lys Phe Phe Glu Ile Thr Pro Glu Lys Asn Gln Gln Leu Gln Asp
                245                 250                 255

Leu Asp Ile Phe Val Asn Ser Val Asp Ile Lys Glu Gly Ser Leu Leu
            260                 265                 270

Leu Pro Asn Tyr Asn Ser Arg Ala Ile Val Ile Val Thr Val Thr Glu
        275                 280                 285

Gly Lys Gly Asp Phe Glu Leu Val Gly Gln Arg Asn Glu Asn Gln Gly
    290                 295                 300

Lys Glu Asn Asp Lys Glu Glu Glu Gln Glu Glu Glu Thr Ser Lys Gln
305                 310                 315                 320

Val Gln Leu Tyr Arg Ala Lys Leu Ser Pro Gly Asp Val Phe Val Ile
                325                 330                 335

Pro Ala Gly His Pro Val Ala Ile Asn Ala Ser Ser Asp Leu Asn Leu
            340                 345                 350

Ile Gly Phe Gly Ile Asn Ala Glu Asn Asn Glu Arg Asn Phe Leu Ala
        355                 360                 365
```

Gly Glu Glu Asp Asn Val Ile Ser Gln Val Glu Arg Pro Val Lys Glu
370 375 380

Leu Ala Phe Pro Gly Ser Ser His Glu Val Asp Arg Leu Leu Lys Asn
385 390 395 400

Gln Lys Gln Ser Tyr Phe Ala Asn Ala Gln Pro Leu Gln Arg Glu
405 410 415

<210> 18
<211> 451
<212> PRT
<213> NONE

<400> 18

Met Ala Thr Thr Ile Lys Ser Arg Phe Pro Leu Leu Leu Leu Leu Gly
1 5 10 15

Ile Met Phe Leu Ala Ser Val Val Cys Val Thr Tyr Ala Asn Tyr Asp
20 25 30

Glu Gly Ser Glu Pro Arg Val Pro Ala Gln Arg Glu Arg Gly Arg Gln
35 40 45

Glu Gly Glu Lys Glu Glu Lys Arg His Gly Glu Trp Arg Pro Ser Tyr
50 55 60

Glu Lys Glu Glu Asp Glu Glu Glu Gly Gln Arg Glu Arg Gly Arg Gln
65 70 75 80

Glu Gly Glu Lys Glu Glu Lys Arg His Gly Glu Trp Arg Pro Ser Tyr
85 90 95

Glu Lys Gln Glu Asp Glu Glu Glu Lys Gln Lys Tyr Arg Tyr Gln Arg
100 105 110

Glu Lys Glu Asp Gly Glu Glu Lys Gln Lys Tyr Gln Tyr Gln Arg Glu
115 120 125

Lys Lys Glu Glu Lys Glu Val Gln Pro Gly Arg Glu Arg Trp Glu Arg
130 135 140

Glu Glu Asp Glu Glu Gln Val Glu Glu Glu Trp Arg Gly Ser Gln Arg
145 150 155 160

Arg Glu Asp Pro Glu Glu Arg Ala Arg Leu Arg His Arg Glu Glu Arg
165 170 175

Thr Lys Arg Asp Arg Arg His Gln Ser Glu Gly Glu Glu Glu Glu Arg
180 185 190

Ser Ser Glu Ser Leu Glu Arg Arg Asn Pro Phe Leu Phe Lys Ser Asn
195 200 205

Lys Phe Leu Thr Leu Phe Glu Asn Glu Asn Gly His Ile Arg Leu Leu
210 215 220

Gln Arg Phe Asp Lys Arg Ser Asp Leu Phe Glu Asn Leu Gln Asn Tyr
225 230 235 240

Arg Leu Val Glu Tyr Arg Ala Lys Pro His Thr Ile Phe Leu Pro Gln
245 250 255

His Ile Asp Ala Asp Leu Ile Leu Val Val Leu Asn Gly Lys Ala Ile
260 265 270

Leu Thr Val Leu Ser Pro Asn Asp Arg Asn Ser Tyr Asn Leu Glu Arg
275 280 285

Gly Asp Thr Ile Lys Leu Pro Ala Gly Thr Thr Ser Tyr Leu Val Asn
290 295 300

Gln Asp Asp Glu Glu Asp Leu Arg Leu Val Asp Leu Val Ile Pro Val
305 310 315 320

Asn Gly Pro Gly Lys Phe Glu Ala Phe Asp Leu Ala Lys Asn Lys Asn
325 330 335

Gln Tyr Leu Arg Gly Phe Ser Lys Asn Ile Leu Glu Ala Ser Tyr Asn
340 345 350

Gly Ala Leu Met Leu Pro His Tyr Asn Ser Arg Ala Ile Val Val Leu
355 360 365

Leu Val Asn Glu Gly Lys Gly Asn Leu Glu Leu Leu Gly Leu Lys Asn
370 375 380

Glu Gln Gln Glu Arg Glu Asp Arg Lys Glu Arg Asn Asn Glu Val Gln
385 390 395 400

Arg Tyr Glu Ala Arg Leu Ser Pro Gly Asp Val Val Ile Ile Pro Ala
405 410 415

Gly His Pro Val Ala Ile Thr Ala Ser Ser Asn Leu Asn Leu Leu Gly

420 425 430

Phe Gly Ile Asn Ala Glu Asn Asn Glu Arg Asn Phe Leu Ser Gly Ser
435 440 445

Asp Asp Asn
450

<210> 19
<211> 571
<212> PRT
<213> NONE

<400> 19

Met Ala Thr Thr Val Lys Ser Arg Phe Pro Leu Leu Leu Phe Leu Gly
1 5 10 15

Ile Ile Phe Leu Ala Ser Val Cys Val Thr Tyr Ala Asn Tyr Asp Glu
20 25 30

Gly Ser Glu Thr Arg Val Pro Gly Gln Arg Glu Arg Gly Arg Gln Glu
35 40 45

Gly Glu Lys Glu Glu Lys Arg His Gly Glu Trp Arg Pro Ser Tyr Glu
50 55 60

Lys Glu Glu His Glu Glu Glu Lys Gln Lys Tyr Arg Tyr Gln Arg Glu
65 70 75 80

Lys Lys Glu Gln Lys Glu Val Gln Pro Gly Arg Glu Arg Trp Glu Arg
85 90 95

Glu Glu Asp Glu Glu Gln Val Glu Glu Glu Trp Arg Gly Ser Gln Arg
100 105 110

Arg Glu Asp Pro Glu Glu Arg Ala Arg Leu Arg His Arg Glu Glu Arg
115 120 125

Thr Lys Arg Asp Arg Arg His Gln Arg Glu Gly Glu Glu Glu Glu Arg
130 135 140

Ser Ser Glu Ser Gln Glu His Arg Asn Pro Phe Leu Phe Lys Ser Asn
145 150 155 160

Lys Phe Leu Thr Leu Phe Glu Asn Glu Asn Gly His Ile Arg Arg Leu
165 170 175

Gln Arg Phe Asp Lys Arg Ser Asp Leu Phe Glu Asn Leu Gln Asn Tyr

```
                180                 185                 190

Arg Leu Val Glu Tyr Arg Ala Lys Pro His Thr Ile Phe Leu Pro Gln
        195                 200                 205

His Ile Asp Ala Asp Leu Ile Leu Val Val Leu Asn Gly Lys Ala Ile
    210                 215                 220

Leu Thr Val Leu Ser Pro Asn Asp Arg Asn Ser Tyr Asn Leu Glu Arg
225                 230                 235                 240

Gly Asp Thr Ile Lys Ile Pro Ala Gly Thr Thr Ser Tyr Leu Val Asn
                245                 250                 255

Gln Asp Asp Glu Glu Asp Leu Arg Val Val Asp Phe Val Ile Pro Val
            260                 265                 270

Asn Arg Pro Gly Lys Phe Glu Ala Phe Gly Leu Ser Glu Asn Lys Asn
        275                 280                 285

Gln Tyr Leu Arg Gly Phe Ser Lys Asn Ile Leu Glu Ala Ser Leu Asn
    290                 295                 300

Thr Lys Tyr Glu Thr Ile Glu Lys Val Leu Leu Glu Glu Gln Glu Lys
305                 310                 315                 320

Lys Pro Gln Gln Leu Arg Asp Arg Lys Arg Thr Gln Gln Gly Glu Glu
                325                 330                 335

Arg Asp Ala Ile Ile Lys Val Ser Arg Glu Gln Ile Glu Glu Leu Arg
            340                 345                 350

Lys Leu Ala Lys Ser Ser Ser Lys Lys Ser Leu Pro Ser Glu Phe Glu
        355                 360                 365

Pro Phe Asn Leu Arg Ser His Lys Pro Glu Tyr Ser Asn Lys Phe Gly
    370                 375                 380

Lys Leu Phe Glu Ile Thr Pro Glu Lys Lys Tyr Pro Gln Leu Gln Asp
385                 390                 395                 400

Leu Asp Ile Leu Val Ser Cys Val Glu Ile Asn Lys Gly Ala Leu Met
                405                 410                 415

Leu Pro His Tyr Asn Ser Arg Ala Ile Val Val Leu Leu Val Asn Glu
            420                 425                 430
```

Gly Lys Gly Asn Leu Glu Leu Leu Gly Leu Lys Asn Glu Gln Gln Glu
435 440 445

Arg Glu Asp Arg Lys Glu Arg Asn Asn Glu Val Gln Arg Tyr Glu Ala
450 455 460

Arg Leu Ser Pro Gly Asp Val Val Ile Ile Pro Ala Gly His Pro Val
465 470 475 480

Ala Ile Ser Ala Ser Ser Asn Leu Asn Leu Leu Gly Phe Gly Ile Asn
485 490 495

Ala Lys Asn Asn Gln Arg Asn Phe Leu Ser Gly Ser Asp Asp Asn Val
500 505 510

Ile Ser Gln Ile Glu Asn Pro Val Lys Glu Leu Thr Phe Pro Gly Ser
515 520 525

Ser Gln Glu Val Asn Arg Leu Ile Lys Asn Gln Lys Gln Ser His Phe
530 535 540

Ala Ser Ala Glu Pro Glu Gln Lys Glu Glu Glu Ser Gln Arg Lys Arg
545 550 555 560

Ser Pro Leu Ser Ser Val Leu Asp Ser Phe Tyr
565 570

<210> 20
<211> 511
<212> PRT
<213> NONE

<400> 20

Met Ala Thr Thr Val Lys Ser Arg Phe Pro Leu Leu Leu Phe Leu Gly
1 5 10 15

Ile Ile Phe Leu Ala Ser Val Cys Val Thr Tyr Ala Asn Tyr Asp Glu
20 25 30

Gly Ser Glu Thr Arg Val Pro Gly Gln Arg Glu Arg Gly Arg Gln Glu
35 40 45

Gly Glu Lys Glu Glu Lys Arg His Gly Glu Trp Arg Pro Ser Tyr Glu
50 55 60

Lys Glu Glu His Glu Glu Glu Lys Gln Lys Tyr Arg Tyr Gln Arg Glu
65 70 75 80

```
Lys Lys Glu Gln Lys Glu Val Gln Pro Gly Arg Glu Arg Trp Glu Arg
                85                  90                  95

Glu Glu Asp Glu Glu Gln Val Glu Glu Glu Trp Arg Gly Ser Gln Arg
            100                 105                 110

Arg Glu Asp Pro Glu Glu Arg Ala Arg Leu Arg His Arg Glu Glu Arg
        115                 120                 125

Thr Lys Arg Asp Arg Arg His Gln Arg Glu Gly Glu Glu Glu Glu Arg
    130                 135                 140

Ser Ser Glu Ser Gln Glu His Arg Asn Pro Phe Leu Phe Lys Ser Asn
145                 150                 155                 160

Lys Phe Leu Thr Leu Phe Glu Asn Glu Asn Gly His Thr Arg Arg Leu
                165                 170                 175

Gln Arg Phe Asp Lys Arg Ser Asp Leu Phe Glu Asn Leu Gln Asn Tyr
            180                 185                 190

Arg Leu Val Glu Tyr Arg Ala Lys Pro His Thr Ile Phe Leu Pro Gln
        195                 200                 205

His Ile Asp Ala Asp Leu Ile Leu Val Val Leu Asn Gly Lys Ala Ile
    210                 215                 220

Leu Thr Val Leu Ser Pro Asn Asp Arg Asn Ser Tyr Asn Leu Glu Arg
225                 230                 235                 240

Gly Asp Thr Ile Lys Val Pro Ala Gly Thr Thr Ser Tyr Leu Val Asn
                245                 250                 255

Gln Asp Asp Glu Glu Ala Leu Arg Val Val Asp Phe Val Ile Pro Val
            260                 265                 270

Asn Arg Pro Gly Lys Phe Glu Ala Phe Gly Leu Ser Glu Asn Lys Asn
        275                 280                 285

Gln Tyr Leu Arg Gly Phe Ser Lys Asn Ile Leu Glu Ala Ser Leu Asn
    290                 295                 300

Thr Lys Tyr Glu Thr Ile Glu Lys Val Leu Leu Glu Glu Gln Glu Lys
305                 310                 315                 320

Lys Pro Gln Gln Leu Arg Asp Arg Lys Arg Thr Gln Gln Gly Glu Glu
                325                 330                 335
```

Arg Asp Ala Ile Ile Lys Val Ser Arg Glu Gln Ile Glu Glu Leu Arg
340 345 350

Lys Leu Ala Lys Ser Ser Ser Lys Lys Ser Leu Pro Ser Glu Phe Glu
355 360 365

Pro Phe Asn Leu Arg Ser His Lys Pro Glu Tyr Ser Asn Lys Phe Gly
370 375 380

Lys Leu Phe Glu Ile Thr Pro Glu Lys Lys Tyr Pro Gln Leu Gln Asp
385 390 395 400

Leu Asp Ile Leu Val Ser Cys Val Glu Ile Asn Lys Gly Ala Leu Met
405 410 415

Leu Pro His Tyr Asn Ser Arg Ala Ile Val Val Leu Leu Val Asn Glu
420 425 430

Gly Lys Gly Asn Leu Glu Leu Leu Gly Leu Lys Asn Glu Gln Gln Glu
435 440 445

Arg Glu Asp Arg Lys Glu Arg Asn Asn Glu Val Gln Arg Tyr Glu Ala
450 455 460

Arg Leu Ser Pro Gly Asp Val Val Ile Ile Pro Ala Gly His Pro Val
465 470 475 480

Ala Ile Ser Ala Ser Ser Asn Leu Asn Leu Leu Gly Phe Gly Ile Asn
485 490 495

Ala Lys Asn Asn Gln Arg Asn Phe Leu Ser Gly Ser Asp Asp Asn
500 505 510

<210> 21
<211> 511
<212> PRT
<213> NONE

<400> 21

Met Ala Thr Thr Val Lys Ser Arg Phe Pro Leu Leu Leu Phe Leu Gly
1 5 10 15

Ile Ile Phe Leu Ala Ser Val Cys Val Thr Tyr Ala Asn Tyr Asp Glu
20 25 30

Gly Ser Glu Thr Arg Val Pro Gly Gln Arg Glu Arg Gly Arg Gln Glu
35 40 45

Gly Glu Lys Glu Glu Lys Arg His Gly Glu Trp Arg Pro Ser Tyr Glu
50 55 60

Lys Glu Glu Asp Glu Glu Glu Lys Gln Lys Tyr Arg Tyr Gln Arg Glu
65 70 75 80

Lys Lys Glu Gln Lys Glu Val Gln Pro Gly Cys Glu Arg Trp Glu Arg
85 90 95

Glu Glu Asp Glu Glu Gln Val Asp Glu Glu Trp Arg Gly Ser Gln Arg
100 105 110

Arg Glu Asp Pro Glu Glu Arg Ala Arg Leu Arg His Arg Glu Glu Arg
115 120 125

Thr Lys Arg Asp Arg Arg His Gln Arg Glu Gly Glu Glu Glu Glu Arg
130 135 140

Ser Ser Gln Ser Gln Glu His Arg Asn Pro Phe Leu Phe Lys Ser Asn
145 150 155 160

Lys Phe Leu Thr Leu Phe Glu Asn Glu Asn Gly His Ile Arg Arg Leu
165 170 175

Gln Arg Phe Asp Lys Arg Ser Asp Leu Phe Glu Asn Leu Gln Asn Tyr
180 185 190

Arg Leu Val Glu Tyr Arg Ala Lys Pro His Thr Ile Phe Leu Pro Gln
195 200 205

His Ile Asp Ala Asp Leu Ile Leu Val Val Leu Asn Gly Lys Ala Ile
210 215 220

Leu Thr Val Leu Ser Pro Asn Asp Arg Asn Ser Tyr Asn Leu Glu Arg
225 230 235 240

Gly Asp Thr Ile Lys Ile Pro Ala Gly Thr Thr Ser Tyr Leu Val Asn
245 250 255

Gln Asp Asp Glu Glu Asp Leu Arg Val Val Asp Phe Val Ile Pro Val
260 265 270

Asn Arg Pro Gly Lys Phe Glu Ala Phe Gly Leu Ser Glu Asn Lys Asn
275 280 285

Gln Tyr Leu Arg Gly Phe Ser Lys Asn Ile Leu Glu Ala Ser Leu Asn
290 295 300

Thr Lys Tyr Glu Thr Ile Glu Lys Val Leu Leu Glu Glu Gln Glu Lys
305 310 315 320

Lys Pro Gln Gln Leu Arg Asp Arg Lys Arg Arg Gln Gln Gly Glu Glu
325 330 335

Arg Asp Ala Ile Ile Lys Val Ser Arg Glu Gln Ile Glu Glu Leu Arg
340 345 350

Lys Leu Ala Lys Ser Ser Ser Lys Lys Ser Leu Pro Ser Glu Phe Glu
355 360 365

Pro Phe Asn Leu Arg Ser His Lys Pro Glu Tyr Ser Asn Lys Phe Gly
370 375 380

Lys Leu Phe Glu Ile Thr Pro Glu Lys Lys Tyr Pro Gln Leu Gln Asp
385 390 395 400

Leu Asp Ile Leu Val Ser Cys Val Glu Ile Asn Lys Gly Ala Leu Met
405 410 415

Leu Pro His Tyr Asn Ser Arg Ala Ile Val Val Leu Leu Val Asn Glu
420 425 430

Gly Lys Gly Asn Leu Glu Leu Leu Gly Leu Lys Asn Glu Gln Gln Glu
435 440 445

Arg Glu Asp Arg Lys Glu Arg Asn Asn Glu Val Gln Arg Tyr Glu Ala
450 455 460

Arg Leu Ser Pro Gly Asp Val Val Ile Ile Pro Ala Gly His Pro Val
465 470 475 480

Ala Ile Thr Ala Ser Ser Asn Leu Asn Leu Leu Ala Phe Gly Ile Asn
485 490 495

Ala Glu Asn Asn Gln Arg Asn Phe Leu Ser Gly Ser Asp Asp Asn
500 505 510

<210> 22
<211> 613
<212> PRT
<213> NONE

<400> 22

Met Ala Thr Thr Ile Lys Ser Arg Phe Pro Leu Leu Leu Leu Leu Gly
1 5 10 15

```
Ile Ile Phe Leu Ala Ser Val Val Cys Val Thr Tyr Ala Asn Tyr Asp
            20                  25                  30

Glu Gly Ser Glu Pro Arg Val Pro Ala Gln Arg Glu Arg Gly Arg Gln
        35                  40                  45

Glu Gly Glu Lys Glu Glu Lys Arg His Gly Glu Trp Arg Pro Ser Tyr
    50                  55                  60

Glu Lys Glu Glu Asp Glu Glu Glu Gly Gln Arg Glu Arg Gly Arg Gln
65                  70                  75                  80

Glu Gly Glu Lys Glu Glu Lys Arg His Gly Glu Trp Arg Pro Ser Tyr
                85                  90                  95

Glu Lys Gln Glu Asp Glu Glu Glu Lys Gln Lys Tyr Arg Tyr Gln Arg
            100                 105                 110

Glu Lys Glu Asp Glu Glu Glu Lys Gln Lys Tyr Gln Tyr Gln Arg Glu
        115                 120                 125

Lys Lys Glu Gln Lys Glu Val Gln Pro Gly Arg Glu Arg Trp Glu Arg
    130                 135                 140

Glu Glu Asp Glu Glu Gln Val Asp Glu Glu Trp Arg Gly Ser Gln Arg
145                 150                 155                 160

Arg Glu Asp Pro Glu Glu Arg Ala Arg Leu Arg His Arg Glu Glu Arg
                165                 170                 175

Thr Lys Arg Asp Arg Arg His Gln Arg Glu Gly Glu Glu Glu Glu Arg
            180                 185                 190

Ser Ser Glu Ser Gln Glu Arg Arg Asn Pro Phe Leu Phe Lys Ser Asn
        195                 200                 205

Lys Phe Leu Thr Leu Phe Glu Asn Glu Asn Gly His Ile Arg Leu Leu
    210                 215                 220

Gln Arg Phe Asp Lys Arg Ser Asp Leu Phe Glu Asn Leu Gln Asn Tyr
225                 230                 235                 240

Arg Leu Val Glu Tyr Arg Ala Lys Pro His Thr Ile Phe Leu Pro Gln
                245                 250                 255

His Ile Asp Ala Asp Leu Ile Leu Val Val Leu Ser Gly Lys Ala Ile
```

260 265 270

Leu Thr Val Leu Ser Pro Asn Asp Arg Asn Ser Tyr Asn Leu Glu Arg
275 280 285

Gly Asp Thr Ile Lys Leu Pro Ala Gly Thr Thr Ser Tyr Leu Val Asn
290 295 300

Gln Asp Asp Glu Glu Asp Leu Arg Leu Val Asp Leu Val Ile Pro Val
305 310 315 320

Asn Gly Pro Gly Lys Phe Glu Ala Phe Asp Leu Ala Lys Asn Lys Asn
325 330 335

Gln Tyr Leu Arg Gly Phe Ser Lys Asn Ile Leu Glu Ala Ser Tyr Asn
340 345 350

Thr Arg Tyr Glu Thr Ile Glu Lys Val Leu Leu Glu Glu Gln Glu Lys
355 360 365

Asp Arg Lys Arg Arg Gln Gln Gly Glu Glu Thr Asp Ala Ile Val Lys
370 375 380

Val Ser Arg Glu Gln Ile Glu Glu Leu Lys Lys Leu Ala Lys Ser Ser
385 390 395 400

Ser Lys Lys Ser Leu Pro Ser Glu Phe Glu Pro Ile Asn Leu Arg Ser
405 410 415

His Lys Pro Glu Tyr Ser Asn Lys Phe Gly Lys Leu Phe Glu Ile Thr
420 425 430

Pro Glu Lys Lys Tyr Pro Gln Leu Gln Asp Leu Asp Leu Phe Val Ser
435 440 445

Cys Val Glu Ile Asn Glu Gly Ala Leu Met Leu Pro His Tyr Asn Ser
450 455 460

Arg Ala Ile Val Val Leu Leu Val Asn Glu Gly Lys Gly Asn Leu Glu
465 470 475 480

Leu Leu Gly Leu Lys Asn Glu Gln Gln Glu Arg Glu Asp Arg Lys Glu
485 490 495

Arg Asn Asn Glu Val Gln Arg Tyr Glu Ala Arg Leu Ser Pro Gly Asp
500 505 510

Val Val Ile Ile Pro Ala Gly His Pro Val Ala Ile Thr Ala Ser Ser
515 520 525

Asn Leu Asn Leu Leu Gly Phe Gly Ile Asn Ala Glu Asn Asn Glu Arg
530 535 540

Asn Phe Leu Ser Gly Ser Asp Asp Asn Val Ile Ser Gln Ile Glu Asn
545 550 555 560

Pro Val Lys Glu Leu Thr Phe Pro Gly Ser Val Gln Glu Ile Asn Arg
565 570 575

Leu Ile Lys Asn Gln Lys Gln Ser His Phe Ala Asn Ala Glu Pro Glu
580 585 590

Gln Lys Glu Gln Gly Ser Gln Gly Lys Arg Ser Pro Leu Ser Ser Ile
595 600 605

Leu Gly Thr Phe Tyr
610

<210> 23
<211> 572
<212> PRT
<213> NONE

<400> 23

Met Ala Thr Thr Ile Lys Ser Arg Phe Pro Leu Leu Leu Leu Leu Gly
1 5 10 15

Ile Ile Phe Leu Ala Ser Val Val Ser Val Thr Tyr Ala Asn Tyr Asp
20 25 30

Glu Gly Ser Glu Pro Arg Val Pro Ala Gln Arg Glu Arg Gly Arg Gln
35 40 45

Glu Gly Glu Lys Glu Glu Lys Arg His Gly Glu Trp Arg Pro Ser Tyr
50 55 60

Glu Lys Glu Glu Asp Glu Glu Glu Gly Gln Arg Glu Arg Gly Arg Gln
65 70 75 80

Glu Gly Glu Lys Glu Glu Lys Arg His Gly Glu Trp Gly Pro Ser Tyr
85 90 95

Glu Lys Gln Glu Asp Glu Glu Glu Lys Gln Lys Tyr Arg Tyr Gln Arg
100 105 110

```
Glu Lys Glu Asp Glu Glu Glu Lys Gln Lys Tyr Gln Tyr Gln Arg Glu
        115                 120                 125

Lys Lys Glu Gln Lys Glu Val Gln Pro Gly Arg Glu Arg Trp Glu Arg
    130                 135                 140

Glu Glu Asp Glu Glu Gln Val Asp Glu Glu Trp Arg Gly Ser Gln Arg
145                 150                 155                 160

Arg Glu Asp Pro Glu Glu Arg Ala Arg Leu Arg His Arg Glu Glu Arg
                165                 170                 175

Thr Lys Arg Asp Arg Arg His Gln Arg Glu Gly Glu Glu Glu Glu Arg
            180                 185                 190

Ser Ser Glu Ser Gln Glu Arg Arg Asn Pro Phe Leu Phe Lys Ser Asn
        195                 200                 205

Lys Phe Leu Thr Leu Phe Glu Asn Glu Asn Gly His Ile Arg Leu Leu
    210                 215                 220

Gln Arg Phe Asp Lys Arg Ser Asp Leu Phe Glu Asn Leu Gln Asn Tyr
225                 230                 235                 240

Arg Leu Val Glu Tyr Arg Ala Lys Pro His Thr Ile Phe Leu Pro Gln
                245                 250                 255

His Ile Asp Ala Asp Leu Ile Leu Val Val Leu Asn Gly Lys Ala Ile
            260                 265                 270

Leu Thr Val Leu Ser Pro Asn Asp Arg Asn Ser Tyr Asn Leu Glu Arg
        275                 280                 285

Gly Asp Thr Ile Lys Leu Pro Ala Gly Thr Thr Ser Tyr Leu Val Asn
    290                 295                 300

Gln Asp Asp Glu Glu Asp Leu Arg Leu Val Asp Leu Val Ile Pro Val
305                 310                 315                 320

Asn Gly Pro Gly Lys Phe Glu Ala Phe Asp Leu Ala Lys Asn Lys Asn
                325                 330                 335

Gln Tyr Leu Arg Gly Phe Ser Lys Asn Ile Leu Glu Ala Ser Tyr Asn
            340                 345                 350

Ala Phe Asp Leu Ala Lys Asn Lys Asn Gln Tyr Leu Arg Gly Phe Ser
        355                 360                 365
```

Lys Asn Ile Leu Glu Ala Ser Tyr Asn Thr Lys Tyr Glu Thr Ile Glu
    370                 375                 380

Lys Val Leu Leu Glu Glu Gln Glu Lys Glu Pro Gln Gln Arg Arg Ala
385                 390                 395                 400

Ile Val Lys Val Ser Arg Gly Gln Ile Glu Glu Leu Arg Lys Leu Ala
                405                 410                 415

Lys Ser Ser Ser Lys Lys Ser Leu Pro Ser Glu Phe Glu Pro Ile Asn
            420                 425                 430

Leu Arg Ser His Lys Pro Glu Tyr Ser Asn Lys Phe Gly Lys Leu Phe
        435                 440                 445

Glu Ile Thr Pro Glu Lys Lys Tyr Pro Gln Leu Gln Asp Leu Asp Leu
    450                 455                 460

Phe Val Ser Cys Val Glu Ile Asn Glu Gly Ala Leu Met Leu Pro His
465                 470                 475                 480

Tyr Asn Ser Arg Ala Ile Val Val Leu Leu Val Asn Glu Gly Lys Gly
                485                 490                 495

Asn Leu Glu Leu Leu Gly Leu Lys Asn Glu Gln Gln Glu Arg Glu Asp
            500                 505                 510

Arg Lys Glu Arg Asn Asn Glu Val Gln Arg Tyr Glu Ala Arg Leu Ser
        515                 520                 525

Pro Gly Asp Val Val Ile Ile Pro Ala Gly His Pro Val Ala Ile Thr
    530                 535                 540

Ala Ser Ser Asn Leu Asn Leu Leu Ala Phe Gly Ile Asn Ala Glu Asn
545                 550                 555                 560

Asn Glu Arg Asn Phe Leu Ser Gly Ser Asp Asp Asn
                565                 570

<210> 24
<211> 527
<212> PRT
<213> NONE

<400> 24

Met Ala Thr Thr Ile Lys Ser Arg Phe Pro Leu Leu Leu Leu Leu Gly
1               5                   10                  15

```
Ile Ile Phe Leu Ala Ser Val Val Cys Val Thr Tyr Ala Asn Tyr Asp
            20                  25                  30

Glu Gly Ser Glu Pro Arg Val Pro Ala Gln Arg Glu Arg Gly Arg Gln
        35                  40                  45

Glu Gly Glu Lys Glu Glu Lys Arg His Gly Glu Trp Arg Pro Ser Tyr
    50                  55                  60

Glu Lys Gln Glu Asp Glu Glu Glu Lys Gln Lys Tyr Arg Tyr Gln Arg
65                  70                  75                  80

Glu Lys Glu Asp Glu Glu Glu Lys Gln Lys Tyr Arg Tyr Gln Arg Glu
                85                  90                  95

Lys Lys Glu Glu Lys Glu Val Arg Pro Gly Arg Glu Arg Trp Glu Arg
            100                 105                 110

Glu Glu Asp Glu Glu Gln Val Glu Glu Glu Trp Arg Gly Ser Gln Arg
        115                 120                 125

Arg Glu Asp Pro Glu Glu Arg Ala Arg Leu Arg His Arg Glu Glu Arg
    130                 135                 140

Thr Lys Arg Asp Arg Arg His Gln Arg Glu Gly Glu Glu Glu Glu Arg
145                 150                 155                 160

Ser Ser Glu Ser Gln Glu Arg Gly Asn Pro Phe Leu Leu Lys Ser Asn
                165                 170                 175

Lys Phe Leu Thr Leu Phe Glu Asn Glu Asn Gly His Ile Arg Leu Leu
            180                 185                 190

Gln Arg Phe Asp Lys Arg Ser Asp Leu Phe Glu Asn Leu Gln Asn Tyr
        195                 200                 205

Arg Leu Val Glu Tyr Arg Ala Lys Pro His Thr Ile Phe Leu Pro Gln
    210                 215                 220

His Ile Asp Ala Asp Leu Ile Leu Val Val Leu Ser Gly Lys Ala Ile
225                 230                 235                 240

Leu Thr Val Leu Ser Pro Asn Asp Arg Asn Ser Tyr Asn Leu Glu Arg
                245                 250                 255

Gly Asp Thr Ile Lys Leu Pro Ala Gly Thr Thr Ser Tyr Leu Val Asn
            260                 265                 270
```

```
Gln Asp Asp Glu Glu Asp Leu Arg Leu Val Asp Leu Val Ile Pro Val
        275                 280                 285

Asn Gly Pro Gly Lys Phe Glu Ala Phe Asp Leu Ala Lys Asn Lys Asn
    290                 295                 300

Gln Tyr Leu Arg Gly Phe Ser Lys Asn Ile Leu Glu Ala Ser Tyr Asn
305                 310                 315                 320

Thr Lys Tyr Glu Thr Ile Glu Lys Val Leu Leu Glu Glu Gln Glu Lys
                325                 330                 335

Glu Pro Gln Gln Arg Arg Asp Arg Lys Arg Arg Gln Gln Gly Gln Glu
            340                 345                 350

Thr Asp Ala Ile Val Lys Val Ser Arg Glu Gln Ile Glu Glu Leu Arg
        355                 360                 365

Lys Leu Ala Lys Ser Ser Ser Lys Lys Ser Leu Pro Ser Glu Phe Glu
    370                 375                 380

Pro Ile Asn Leu Arg Ser His Lys Pro Glu Tyr Ser Asn Lys Phe Gly
385                 390                 395                 400

Lys Leu Phe Glu Ile Thr Pro Glu Lys Lys Tyr Pro Gln Leu Gln Asp
                405                 410                 415

Leu Asp Leu Phe Val Ser Cys Val Glu Ile Asn Glu Gly Ala Leu Met
            420                 425                 430

Leu Pro His Tyr Asn Ser Arg Ala Ile Val Val Leu Leu Val Asn Glu
        435                 440                 445

Gly Lys Gly Asn Val Glu Leu Leu Gly Leu Lys Asn Glu Gln Gln Glu
    450                 455                 460

Arg Glu Asp Arg Lys Glu Arg Asn Asn Glu Val Gln Arg Tyr Glu Ala
465                 470                 475                 480

Arg Leu Ser Pro Gly Asp Val Val Ile Ile Pro Ala Gly His Pro Val
                485                 490                 495

Ala Ile Thr Ala Ser Ser Asn Leu Asn Leu Leu Gly Phe Gly Ile Asn
            500                 505                 510

Ala Glu Asn Asn Glu Arg Asn Phe Leu Ser Gly Ser Asp Asp Asn
```

515 520 525

<210> 25
<211> 130
<212> PRT
<213> NONE

<400> 25

Met Ala Ser Val Lys Leu Ala Ser Leu Ile Val Leu Phe Ala Thr Leu
1 5 10 15

Gly Met Phe Leu Thr Lys Asn Val Gly Ala Ala Ser Cys Asn Gly Val
20 25 30

Cys Ser Pro Phe Glu Met Pro Pro Cys Gly Thr Ser Ala Cys Arg Cys
35 40 45

Ile Pro Val Gly Leu Val Ile Gly Tyr Cys Arg Asn Pro Ser Gly Val
50 55 60

Phe Leu Arg Thr Asn Asp Glu His Pro Asn Leu Cys Glu Ser Asp Ala
65 70 75 80

Asp Cys Arg Lys Lys Gly Ser Gly Lys Phe Cys Gly His Tyr Pro Asn
85 90 95

Pro Gly Ile Glu Tyr Gly Trp Cys Phe Ala Ser Lys Ser Glu Ala Glu
100 105 110

Asp Phe Phe Ser Lys Ile Thr Gln Lys Asp Leu Leu Lys Ser Val Ser
115 120 125

Thr Ala
130

<210> 26
<211> 130
<212> PRT
<213> NONE

<400> 26

Met Ala Ser Val Lys Leu Ala Ser Leu Ile Val Leu Phe Ala Thr Leu
1 5 10 15

Gly Met Phe Leu Thr Lys Asn Val Gly Ala Ile Ser Cys Asn Gly Val
20 25 30

Cys Ser Pro Phe Asp Ile Pro Pro Cys Gly Ser Pro Leu Cys Arg Cys
35 40 45

Ile Pro Ala Gly Leu Val Ile Gly Asn Cys Arg Asn Pro Tyr Gly Val
50 55 60

Phe Leu Arg Thr Asn Asp Glu His Pro Asn Leu Cys Glu Ser Asp Ala
65 70 75 80

Asp Cys Arg Lys Lys Gly Ser Gly Thr Phe Cys Gly His Tyr Pro Asn
85 90 95

Pro Asp Ile Glu Tyr Gly Trp Cys Phe Ala Ser Lys Ser Glu Ala Glu
100 105 110

Asp Val Phe Ser Lys Ile Thr Pro Lys Asp Leu Leu Lys Ser Val Ser
115 120 125

Thr Ala
130

<210> 27
<211> 130
<212> PRT
<213> NONE

<400> 27

Met Ala Ser Val Lys Leu Ala Ser Leu Ile Val Leu Phe Ala Thr Leu
1 5 10 15

Gly Met Phe Leu Thr Lys Asn Val Gly Ala Ala Ser Cys Asn Gly Val
20 25 30

Cys Ser Pro Phe Glu Met Pro Pro Cys Gly Thr Ser Ala Cys Arg Cys
35 40 45

Ile Pro Val Gly Leu Phe Ile Gly Tyr Cys Arg Asn Pro Ser Gly Val
50 55 60

Phe Leu Lys Ala Asn Asp Glu His Pro Asn Leu Cys Glu Ser Asp Ala
65 70 75 80

Asp Cys Arg Lys Lys Gly Ser Gly Asn Phe Cys Gly His Tyr Pro Asn
85 90 95

Pro Asp Ile Glu Tyr Gly Trp Cys Phe Ala Ser Lys Ser Glu Ala Glu
100 105 110

Asp Phe Phe Ser Lys Ile Thr Pro Lys Asp Leu Leu Lys Ser Val Ser
115 120 125

```
Thr Ala
    130


<210>  28
<211>  130
<212>  PRT
<213>  NONE

<400>  28

Met Ala Ser Val Lys Leu Ala Ser Leu Met Val Leu Phe Ala Thr Leu
1               5                   10                  15


Gly Met Phe Leu Thr Lys Asn Val Gly Ala Ala Ser Cys Asn Gly Val
            20                  25                  30


Cys Ser Pro Phe Glu Met Pro Pro Cys Gly Ser Ser Ala Cys Arg Cys
        35                  40                  45


Ile Pro Val Gly Leu Val Val Gly Tyr Cys Arg His Pro Ser Gly Val
    50                  55                  60


Phe Leu Arg Thr Asn Asp Glu His Pro Asn Leu Cys Glu Ser Asp Ala
65                  70                  75                  80


Asp Cys Arg Lys Lys Gly Ser Gly Asn Phe Cys Gly His Tyr Pro Asn
                85                  90                  95


Pro Asp Ile Glu Tyr Gly Trp Cys Phe Ala Ser Lys Ser Glu Ala Glu
            100                 105                 110


Asp Phe Phe Ser Lys Ile Thr Gln Lys Asp Leu Leu Lys Ser Val Ser
        115                 120                 125


Thr Ala
    130


<210>  29
<211>  130
<212>  PRT
<213>  NONE

<400>  29

Met Ala Ser Val Lys Leu Ala Ser Leu Ile Val Leu Phe Ala Thr Leu
1               5                   10                  15


Gly Met Phe Leu Thr Lys Asn Val Gly Ala Ala Ser Cys Asn Gly Val
            20                  25                  30
```

Cys Ser Pro Phe Glu Met Pro Pro Cys Gly Thr Ser Ala Cys Arg Cys
35 40 45

Ile Pro Val Gly Leu Val Val Gly Tyr Cys Arg Asn Pro Ser Gly Val
50 55 60

Phe Leu Arg Thr Asn Asp Glu His Pro Asn Leu Cys Glu Ser Asp Ala
65 70 75 80

Asp Cys Arg Lys Lys Gly Ser Gly Asn Phe Cys Gly His Tyr Pro Asn
85 90 95

Pro Asp Ile Glu Tyr Gly Trp Cys Phe Ala Ser Lys Ser Glu Ala Glu
100 105 110

Asp Phe Phe Ser Lys Ile Thr Pro Lys Asp Leu Leu Lys Ser Val Ser
115 120 125

Thr Ala
130

<210> 30
<211> 130
<212> PRT
<213> NONE

<400> 30

Met Ala Ser Val Lys Leu Ala Ser Leu Ile Val Leu Phe Ala Thr Leu
1 5 10 15

Gly Met Phe Leu Thr Lys Asn Val Gly Ala Ala Ser Cys Asn Gly Val
20 25 30

Cys Ser Pro Phe Glu Met Pro Pro Cys Gly Ser Ser Ala Cys Arg Cys
35 40 45

Ile Pro Val Gly Leu Leu Ile Gly Tyr Cys Arg Asn Pro Ser Gly Val
50 55 60

Phe Leu Lys Gly Asn Asp Glu His Pro Asn Leu Cys Glu Ser Asp Ala
65 70 75 80

Asp Cys Lys Lys Lys Gly Ser Gly Asn Phe Cys Gly His Tyr Pro Asn
85 90 95

Pro Asp Ile Glu Tyr Gly Trp Cys Phe Ala Ser Lys Ser Glu Ala Glu
100 105 110

```
Asp Val Phe Ser Lys Ile Thr Pro Lys Asp Leu Leu Lys Ser Val Ser
        115                 120                 125

Thr Ala
    130

<210>  31
<211>  231
<212>  PRT
<213>  NONE

<400>  31

Met Thr Lys Thr Gly Tyr Ile Asn Ala Ala Phe Arg Ser Ser Gln Asn
1               5                   10                  15

Asn Glu Ala Tyr Leu Phe Ile Asn Asp Lys Tyr Val Leu Leu Asp Tyr
            20                  25                  30

Ala Pro Gly Thr Ser Asn Asp Lys Val Leu Tyr Gly Pro Thr Pro Val
        35                  40                  45

Arg Asp Gly Phe Lys Ser Leu Asn Gln Thr Val Phe Gly Ser Tyr Gly
    50                  55                  60

Val Asp Cys Ser Phe Asp Thr Asp Asn Asp Glu Ala Phe Ile Phe Tyr
65                  70                  75                  80

Glu Lys Phe Cys Ala Leu Ile Asp Tyr Ala Pro His Ser Asn Lys Asp
                85                  90                  95

Lys Ile Ile Leu Gly Pro Lys Lys Ile Ala Asp Met Phe Pro Phe Phe
            100                 105                 110

Glu Gly Thr Val Phe Glu Asn Gly Ile Asp Ala Ala Tyr Arg Ser Thr
        115                 120                 125

Arg Gly Lys Glu Val Tyr Leu Phe Lys Gly Asp Gln Tyr Ala Arg Ile
    130                 135                 140

Asp Tyr Glu Thr Asn Ser Met Val Asn Lys Glu Ile Lys Ser Ile Arg
145                 150                 155                 160

Asn Gly Phe Pro Cys Phe Arg Asn Thr Ile Phe Glu Ser Gly Thr Asp
                165                 170                 175

Ala Ala Phe Ala Ser His Lys Thr Asn Glu Val Tyr Phe Phe Lys Gly
            180                 185                 190
```

Asp Tyr Tyr Ala Arg Val Thr Val Thr Pro Gly Ala Thr Asp Asp Gln
        195                 200                 205

Ile Met Asp Gly Val Arg Lys Thr Leu Asp Tyr Trp Pro Ser Leu Arg
    210                 215                 220

Gly Ile Ile Pro Leu Glu Asn
225                 230

<210> 32
<211> 484
<212> PRT
<213> NONE

<400> 32

Met Ser Lys Pro Phe Leu Ser Leu Leu Ser Leu Ser Leu Leu Leu Phe
1               5                   10                  15

Thr Ser Thr Cys Leu Ala Thr Ser Ser Glu Phe Asp Arg Leu Asn Gln
            20                  25                  30

Cys Arg Leu Asp Asn Ile Asn Ala Leu Glu Pro Asp His Arg Val Glu
        35                  40                  45

Ser Glu Ala Gly Leu Thr Glu Thr Trp Asn Pro Asn His Pro Glu Leu
    50                  55                  60

Arg Cys Ala Gly Val Ser Leu Ile Arg Arg Thr Ile Asp Pro Asn Gly
65                  70                  75                  80

Leu His Leu Pro Ser Tyr Ser Pro Ser Pro Gln Leu Ile Tyr Ile Ile
                85                  90                  95

Gln Gly Lys Gly Val Ile Gly Leu Thr Leu Pro Gly Cys Pro Gln Thr
            100                 105                 110

Tyr Gln Glu Pro Arg Ser Ser Gln Ser Arg Gln Gly Ser Arg Gln Gln
        115                 120                 125

Gln Pro Asp Ser His Gln Lys Ile Arg Arg Phe Arg Lys Gly Asp Ile
    130                 135                 140

Ile Ala Ile Pro Ser Gly Ile Pro Tyr Trp Thr Tyr Asn Asn Gly Asp
145                 150                 155                 160

Glu Pro Leu Val Ala Ile Ser Leu Leu Asp Thr Ser Asn Ile Ala Asn
                165                 170                 175

```
Gln Leu Asp Ser Thr Pro Arg Val Phe Tyr Leu Val Gly Asn Pro Glu
            180                 185                 190

Val Glu Phe Pro Glu Thr Gln Glu Glu Gln Gln Glu Arg His Gln Gln
        195                 200                 205

Lys His Ser Leu Pro Val Gly Arg Arg Gly Gly Gln His Gln Gln Glu
    210                 215                 220

Glu Glu Ser Glu Glu Gln Lys Asp Gly Asn Ser Val Leu Ser Gly Phe
225                 230                 235                 240

Ser Ser Glu Phe Leu Ala His Thr Phe Asn Thr Glu Glu Asp Thr Ala
                245                 250                 255

Lys Arg Leu Arg Ser Pro Arg Asp Lys Arg Asn Gln Ile Val Arg Val
            260                 265                 270

Glu Gly Gly Leu Arg Ile Ile Asn Pro Glu Gly Gln Gln Glu Glu Glu
        275                 280                 285

Glu Glu Glu Glu Glu Glu Lys Gln Arg Ser Glu Gln Gly Arg Asn Gly
    290                 295                 300

Leu Glu Glu Thr Ile Cys Ser Leu Lys Ile Arg Glu Asn Ile Ala Gln
305                 310                 315                 320

Pro Ala Arg Ala Asp Leu Tyr Asn Pro Arg Ala Gly Ser Ile Ser Thr
                325                 330                 335

Ala Asn Ser Leu Thr Leu Pro Ile Leu Arg Tyr Leu Arg Leu Ser Ala
            340                 345                 350

Glu Tyr Val Arg Leu Tyr Arg Asn Gly Ile Tyr Ala Pro His Trp Asn
        355                 360                 365

Ile Asn Ala Asn Ser Leu Leu Tyr Val Ile Arg Gly Glu Gly Arg Val
    370                 375                 380

Arg Ile Val Asn Ser Gln Gly Asn Ala Val Phe Asp Asn Lys Val Thr
385                 390                 395                 400

Lys Gly Gln Leu Val Val Val Pro Gln Asn Phe Val Val Ala Glu Gln
                405                 410                 415

Ala Gly Glu Glu Glu Gly Leu Glu Tyr Leu Val Phe Lys Thr Asn Asp
            420                 425                 430
```

Arg Ala Ala Val Ser His Val Gln Gln Val Phe Arg Ala Thr Pro Ala
435 440 445

Asp Val Leu Ala Asn Ala Phe Gly Leu Arg Gln Arg Gln Val Thr Glu
450 455 460

Leu Lys Leu Ser Gly Asn Arg Gly Pro Leu Val His Pro Gln Ser Gln
465 470 475 480

Ser Gln Ser Asn

<210> 33
<211> 335
<212> PRT
<213> NONE

<400> 33

Gly Ile Pro Tyr Trp Thr Tyr Asn Asn Gly Asp Glu Pro Leu Val Ala
1 5 10 15

Ile Ser Leu Leu Asp Thr Ser Asn Ile Ala Asn Gln Leu Asp Ser Thr
20 25 30

Pro Arg Val Phe Tyr Leu Gly Gly Asn Pro Glu Val Glu Phe Pro Glu
35 40 45

Thr Gln Glu Glu Gln Gln Glu Arg His Gln Gln Lys His Ser Leu Pro
50 55 60

Val Gly Arg Arg Gly Gly Gln His Gln Gln Glu Glu Glu Ser Glu Glu
65 70 75 80

Gln Lys Asp Gly Asn Ser Val Leu Ser Gly Phe Ser Ser Glu Phe Leu
85 90 95

Ala Gln Thr Phe Asn Thr Glu Glu Asp Thr Ala Lys Arg Leu Arg Ser
100 105 110

Pro Arg Asp Lys Arg Asn Gln Ile Val Arg Val Glu Gly Gly Leu Arg
115 120 125

Ile Ile Asn Pro Glu Gly Gln Gln Glu Glu Glu Glu Glu Glu Glu Glu
130 135 140

Glu Lys Gln Arg Ser Glu Gln Gly Arg Asn Gly Leu Glu Glu Thr Ile
145 150 155 160

```
Cys Ser Leu Lys Ile Arg Glu Asn Ile Ala Gln Pro Ala Arg Ala Asp
                165                 170                 175

Leu Tyr Asn Pro Arg Ala Gly Ser Ile Ser Thr Ala Asn Ser Leu Thr
            180                 185                 190

Leu Pro Ile Leu Arg Tyr Leu Arg Leu Ser Ala Glu Tyr Val Arg Leu
        195                 200                 205

Tyr Arg Asn Gly Ile Tyr Ala Pro His Trp Asn Ile Asn Ala Asn Ser
    210                 215                 220

Leu Leu Tyr Val Ile Arg Gly Glu Gly Arg Val Arg Ile Val Asn Ser
225                 230                 235                 240

Gln Gly Asn Ala Val Phe Asp Asn Lys Val Arg Lys Gly Gln Leu Val
                245                 250                 255

Val Val Pro Gln Asn Phe Val Val Ala Glu Gln Ala Gly Glu Glu Glu
            260                 265                 270

Gly Leu Glu Tyr Leu Val Phe Lys Thr Asn Asp Arg Ala Ala Val Ser
        275                 280                 285

His Val Gln Gln Val Phe Arg Ala Thr Pro Ala Asp Val Leu Ala Asn
    290                 295                 300

Ala Phe Gly Leu Arg Gln Arg Gln Val Thr Glu Leu Lys Leu Ser Gly
305                 310                 315                 320

Asn Arg Gly Pro Leu Val His Pro Gln Ser Gln Ser Gln Ser Asn
                325                 330                 335


<210>  34
<211>  329
<212>  PRT
<213>  NONE

<400>  34

Gly Ile Pro Tyr Trp Thr Tyr Asn Asn Gly Asp Glu Pro Leu Val Ala
1               5                   10                  15

Ile Ser Leu Leu Asp Thr Ser Asn Ile Ala Asn Gln Leu Asp Ser Thr
            20                  25                  30

Pro Arg Val Phe Tyr Leu Gly Gly Asn Pro Glu Val Glu Phe Pro Glu
        35                  40                  45
```

```
Thr Gln Glu Glu Gln Gln Glu Arg His Gln Gln Lys His Ser Leu Pro
    50                  55                  60

Val Gly Arg Arg Gly Gly Gln His Gln Gln Glu Glu Asp Gly Asn Ser
65                  70                  75                  80

Val Leu Ser Gly Phe Ser Ser Glu Phe Leu Ala Gln Thr Phe Asn Thr
                85                  90                  95

Glu Glu Asp Thr Ala Lys Arg Leu Arg Ser Pro Arg Asp Lys Arg Asn
            100                 105                 110

Gln Ile Val Arg Val Glu Gly Gly Leu Arg Ile Ile Asn Pro Glu Gly
        115                 120                 125

Gln Gln Glu Glu Glu Glu Glu Glu Glu Glu Glu Lys Gln Arg Ser Glu
    130                 135                 140

Gln Gly Arg Asn Gly Leu Glu Glu Thr Ile Cys Ser Leu Lys Ile Arg
145                 150                 155                 160

Glu Asn Ile Ala Gln Pro Ala Arg Ala Asp Leu Tyr Asn Pro Arg Ala
                165                 170                 175

Gly Ser Ile Ser Thr Ala Asn Ser Leu Thr Leu Pro Ile Leu Arg Tyr
            180                 185                 190

Leu Arg Leu Ser Ala Glu Tyr Val Arg Leu Tyr Arg Asn Gly Ile Tyr
        195                 200                 205

Ala Pro His Trp Asn Ile Asn Ala Asn Ser Leu Leu Tyr Val Ile Arg
    210                 215                 220

Gly Glu Gly Arg Val Arg Ile Val Asn Ser Gln Gly Asn Ala Val Phe
225                 230                 235                 240

Asp Asn Lys Val Arg Lys Gly Gln Leu Val Val Val Pro Gln Asn Phe
                245                 250                 255

Val Val Ala Glu Gln Ala Gly Glu Glu Glu Gly Leu Glu Tyr Leu Val
            260                 265                 270

Phe Lys Thr Asn Asp Arg Ala Ala Val Ser His Val Gln Gln Val Phe
        275                 280                 285

Arg Ala Thr Pro Ala Asp Val Leu Ala Asn Ala Phe Gly Leu Arg Gln
```

290 295 300

Arg Gln Val Thr Glu Leu Lys Leu Ser Gly Asn Arg Gly Pro Leu Val
305 310 315 320

His Pro Gln Ser Gln Ser Gln Ser Asn
325

<210> 35
<211> 335
<212> PRT
<213> NONE

<400> 35

Gly Ile Pro Tyr Trp Thr Tyr Asn Asn Gly Asp Glu Pro Leu Val Ala
1 5 10 15

Ile Ser Leu Leu Asp Thr Ser Asn Ile Ala Asn Gln Leu Asp Ser Thr
20 25 30

Pro Arg Val Phe Tyr Leu Gly Gly Asn Pro Glu Val Glu Phe Pro Glu
35 40 45

Thr Gln Glu Glu Gln Gln Glu Arg His Gln Gln Lys His Ser Leu Pro
50 55 60

Val Gly Arg Arg Gly Gly Gln His Gln Gln Glu Glu Glu Ser Glu Glu
65 70 75 80

Gln Lys Asp Gly Asn Ser Val Leu Ser Gly Phe Ser Ser Glu Phe Leu
85 90 95

Ala Gln Thr Phe Asn Thr Glu Glu Asp Thr Ala Lys Arg Leu Arg Ser
100 105 110

Pro Arg Asp Lys Arg Asn Gln Ile Val Arg Val Glu Gly Gly Leu Arg
115 120 125

Ile Ile Asn Pro Glu Gly Gln Gln Glu Glu Glu Glu Gln Glu Glu Glu
130 135 140

Glu Lys Gln Arg Ser Glu Gln Gly Arg Asn Gly Leu Glu Glu Thr Ile
145 150 155 160

Cys Ser Leu Lys Ile Arg Glu Asn Ile Ala Gln Pro Ala Arg Ala Asp
165 170 175

Leu Tyr Asn Pro Arg Ala Gly Ser Ile Ser Thr Ala Asn Ser Leu Thr

180 185 190

Leu Pro Ile Leu Arg Tyr Leu Arg Leu Ser Ala Glu Tyr Val Arg Leu
195 200 205

Tyr Arg Asn Gly Ile Tyr Ala Pro His Trp Asn Ile Asn Ala Asn Ser
210 215 220

Leu Leu Tyr Val Ile Arg Gly Glu Gly Arg Val Arg Ile Val Asn Ser
225 230 235 240

Gln Gly Asn Ala Val Phe Asp Asn Lys Val Arg Lys Gly Gln Leu Val
245 250 255

Val Val Pro Gln Asn Phe Val Val Ala Glu Gln Ala Gly Glu Glu Glu
260 265 270

Gly Leu Glu Tyr Leu Val Phe Lys Thr Asn Asp Arg Ala Ala Val Ser
275 280 285

His Val Gln Gln Val Phe Arg Ala Thr Pro Ala Asp Val Leu Ala Asn
290 295 300

Ala Phe Gly Leu Arg Gln Arg Gln Val Thr Glu Leu Lys Leu Ser Gly
305 310 315 320

Asn Arg Gly Pro Leu Val His Pro His Ser Gln Ser Gln Ser Asn
325 330 335

<210> 36
<211> 606
<212> PRT
<213> NONE

<400> 36

Met Ser Arg Pro Phe Thr Leu Ser Leu Leu Ser Leu Cys Leu Leu Leu
1 5 10 15

Phe Ala Pro Ala Cys Leu Gly Thr Thr Asn Leu Phe Asn Arg Cys Arg
20 25 30

Ile Asn Ser Leu Asn Ala Leu Lys Pro Asp His Arg Val Glu Ser Asn
35 40 45

Gly Gly Leu Ile Glu Thr Trp Ser Ser Thr His Arg Glu Leu Glu Cys
50 55 60

Ala Gly Val Thr Phe Ser Arg Arg Thr Ile Tyr Arg Asn Gly Leu His

```
65                  70                  75                  80

Met Pro Ser Tyr Ser Pro Tyr Pro Gln Met Ile Ile Ala Val Gln Gly
                85                  90                  95

Lys Gly Ala Leu Gly Leu Ala Ile Pro Gly Cys Pro Gln Thr Tyr Glu
            100                 105                 110

Glu Ala Val Asp Glu Ser Thr Ser Ser Gln Lys Pro Ser Asp Cys His
        115                 120                 125

Gln Lys Ile Leu Gln Phe Ser Glu Gly His Val Leu Leu Ile Pro Pro
    130                 135                 140

Gly Val Pro Tyr Trp Thr Tyr Asn Thr Gly His Glu Ser Leu Ile Ile
145                 150                 155                 160

Val Ser Leu Leu Tyr Thr Ser Asn Asp Tyr Asn Gln Leu Asp Gln Ser
                165                 170                 175

Pro Arg Glu Phe Tyr Leu Ala Gly Asn Pro Asp Ile Glu His Pro Glu
            180                 185                 190

Ala Ile Lys Glu Gln Lys Gln Val Glu Glu Glu Gly Ser Asn Val Leu
        195                 200                 205

Gly Gly Phe Gly Lys Arg Phe Leu Ala Arg Ser Leu Asp Ile Asp Glu
    210                 215                 220

Asp Ile Ala Lys Lys Leu Val Ser Pro Glu Asp Glu Met Lys Gln Ile
225                 230                 235                 240

Val Lys Leu Lys Asp Gly Leu Ser Val Ile Ser Pro Lys Trp Gln Gly
                245                 250                 255

Leu Gln Asp Glu Asp Glu Asp Asp Lys Glu Glu Asp Glu Asp Glu Ser
            260                 265                 270

Gln Gly Lys Arg Val His Lys Lys Glu Glu Lys Glu Val Glu Pro Leu
        275                 280                 285

Pro His Gly Lys Arg Val His Lys Lys Glu Glu Lys Glu Val Glu Pro
    290                 295                 300

Leu Pro Pro His Gly Lys Arg Val His Lys Glu Glu Glu Lys Glu Val
305                 310                 315                 320
```

```
Glu Pro Leu Pro Pro Arg Lys His Val His Lys Glu Glu Glu Lys Glu
                325                 330                 335

Val Glu Pro Leu Pro His Arg Lys His Leu Arg Lys Glu Glu Glu Glu
            340                 345                 350

Glu Lys Pro Arg Ala Arg Arg Thr Arg Gly Pro Thr Pro Ser Pro Gly
        355                 360                 365

Gly Glu Gly His Arg Val Val Glu Glu Glu Glu Glu Tyr Asp Glu Pro
    370                 375                 380

Val Arg His Lys Thr Arg His Glu Lys Ser Trp Lys Glu Asp Gln Pro
385                 390                 395                 400

Ala Glu Glu Val Val Glu Lys Gly Glu Ala His Glu Glu Trp Glu Thr
                405                 410                 415

Lys Gln Ser Lys Asp Lys Asn Arg Gly Ser Asn Gly Ile Glu Glu Thr
            420                 425                 430

Leu Cys Thr Leu Lys Leu Gln His Asn Ile Ala Arg Ala Ser Ser Ala
        435                 440                 445

Asp Phe Phe Asn Pro Lys Ala Gly Arg Ile Ser Asn Leu Asn Ser Leu
    450                 455                 460

Thr Leu Pro Val Leu Gln Gln Leu Gly Leu Ser Ala Gln Tyr Val Val
465                 470                 475                 480

Leu Tyr Lys Asn Gly Ile Tyr Ser Pro His Trp Asn Leu Asn Ala Asn
                485                 490                 495

Ser Val Ile Tyr Val Ile Arg Gly Gln Gly Gln Val Arg Val Val Asn
            500                 505                 510

Ser Glu Gly Ile Ala Val Phe Asp Asp Glu Leu Lys Lys Gly Gln Leu
        515                 520                 525

Leu Val Val Pro Gln Asn Phe Met Val Ala Glu Glu Ala Gly Glu Gln
    530                 535                 540

Gly Phe Glu Tyr Val Val Phe Lys Thr Asn Pro Asn Ala Val Thr Ser
545                 550                 555                 560

Tyr Leu Lys Asp Thr Phe Arg Ser Phe Pro Ala Glu Val Ile Ala Lys
                565                 570                 575
```

Ile Tyr Lys Leu Ser His Ser Glu Val Ser Glu Leu Lys Phe Glu Gly
580 585 590

Asn Trp Gly Pro Leu Ile Asn Pro Val Lys Ser Gln Ser Ser
595 600 605

<210> 37
<211> 463
<212> PRT
<213> NONE

<400> 37

Met Ala Ala Thr Thr Leu Lys Asp Ser Phe Pro Leu Leu Thr Leu Leu
1 5 10 15

Gly Ile Ala Phe Leu Ala Ser Val Cys Leu Ser Ser Arg Ser Asp Gln
20 25 30

Asp Asn Pro Phe Val Phe Glu Ser Asn Arg Phe Gln Thr Leu Phe Glu
35 40 45

Asn Glu Asn Gly His Ile Arg Leu Leu Gln Lys Phe Asp Gln His Ser
50 55 60

Lys Leu Leu Glu Asn Leu Gln Asn Tyr Arg Leu Leu Glu Tyr Lys Ser
65 70 75 80

Lys Pro His Thr Ile Phe Leu Pro Gln Gln Thr Asp Ala Asp Phe Ile
85 90 95

Leu Val Val Leu Ser Gly Lys Ala Ile Leu Thr Val Leu Leu Pro Asn
100 105 110

Asp Arg Asn Ser Phe Ser Leu Glu Arg Gly Asp Thr Ile Lys Leu Pro
115 120 125

Ala Gly Thr Ile Gly Tyr Leu Val Asn Arg Asp Asp Glu Glu Asp Leu
130 135 140

Arg Val Leu Asp Leu Val Ile Pro Val Asn Arg Pro Gly Glu Pro Gln
145 150 155 160

Ser Phe Leu Leu Ser Gly Asn Gln Asn Gln Pro Ser Ile Leu Ser Gly
165 170 175

Phe Ser Lys Asn Ile Leu Glu Ala Ser Phe Asn Thr Asp Tyr Lys Glu
180 185 190

```
Ile Glu Lys Val Leu Leu Glu Glu His Gly Lys Glu Lys Tyr His Arg
        195                 200                 205

Arg Gly Leu Lys Asp Arg Arg Gln Arg Gly Gln Glu Glu Asn Val Ile
    210                 215                 220

Val Lys Ile Ser Arg Lys Gln Ile Glu Glu Leu Asn Lys Asn Ala Lys
225                 230                 235                 240

Ser Ser Ser Lys Lys Ser Thr Ser Ser Glu Ser Glu Pro Phe Asn Leu
                245                 250                 255

Arg Ser Arg Glu Pro Ile Tyr Ser Asn Lys Phe Gly Lys Phe Phe Glu
            260                 265                 270

Ile Thr Pro Lys Arg Asn Pro Gln Leu Gln Asp Leu Asn Ile Phe Val
        275                 280                 285

Asn Tyr Val Glu Ile Asn Glu Gly Ser Leu Leu Leu Pro His Tyr Asn
    290                 295                 300

Ser Arg Ala Ile Val Ile Val Thr Val Asn Glu Gly Lys Gly Asp Phe
305                 310                 315                 320

Glu Leu Val Gly Gln Arg Asn Glu Asn Gln Gln Gly Leu Arg Glu Glu
                325                 330                 335

Tyr Asp Glu Glu Lys Glu Gln Gly Glu Glu Glu Ile Arg Lys Gln Val
            340                 345                 350

Gln Asn Tyr Lys Ala Lys Leu Ser Pro Gly Asp Val Leu Val Ile Pro
        355                 360                 365

Ala Gly Tyr Pro Val Ala Ile Lys Ala Ser Ser Asn Leu Asn Leu Val
    370                 375                 380

Gly Phe Gly Ile Asn Ala Glu Asn Asn Gln Arg Tyr Phe Leu Ala Gly
385                 390                 395                 400

Glu Glu Asp Asn Val Ile Ser Gln Ile His Lys Pro Val Lys Glu Leu
                405                 410                 415

Ala Phe Pro Gly Ser Ala Gln Glu Val Asp Thr Leu Leu Glu Asn Gln
            420                 425                 430

Lys Gln Ser His Phe Ala Asn Ala Gln Pro Arg Glu Arg Glu Arg Gly
        435                 440                 445
```

Ser Gln Glu Ile Lys Asp His Leu Tyr Ser Ile Leu Gly Ser Phe
    450                 455                 460

<210>  38
<211>  477
<212>  PRT
<213>  NONE

<400>  38

Met Ala Thr Thr Asn Lys Ser Arg Phe Ala Leu Val Leu Leu Pro Gly
1               5                   10                  15

Ile Ile Phe Leu Ala Ser Val Cys Val Thr Cys Ala Asn Tyr Asp Glu
            20                  25                  30

Gly Ser Glu Pro Arg Val Pro Gly Lys Arg Glu Arg Gly Arg Gln Glu
        35                  40                  45

Gly Glu Lys Glu Glu Lys Arg His Gly Glu Trp Ser Pro Ser His Glu
    50                  55                  60

Lys Glu Ala Gln Pro Gly Arg Glu Arg Trp Glu Arg Lys Glu Asp Glu
65                  70                  75                  80

Glu Gln Val Glu Glu Glu Trp Arg Gly Ser Gln Arg His Glu Asp Pro
                85                  90                  95

Asp Glu Arg Glu Arg Glu Arg Asn Arg Glu Glu Arg Thr Lys Arg Lys
            100                 105                 110

Glu Glu Glu Lys Arg Gly Glu Arg Asp Thr Arg His Gln His Glu Gly
        115                 120                 125

Glu Glu Glu Glu Ser Ser Ser Lys Ser Gln Glu Arg Arg Asn Pro Phe
    130                 135                 140

Leu Phe Lys Ser Asn Lys Phe Leu Thr Leu Phe Gln Asn Lys Asn Gly
145                 150                 155                 160

Tyr Ile Arg Arg Leu Gln Arg Phe Asp Thr Arg Ser Asp Leu Phe Glu
                165                 170                 175

Asn Leu Gln Asn Tyr Arg Leu Val Glu Tyr Arg Ala Lys Pro His Thr
            180                 185                 190

Ile Phe Leu Pro Gln His Ile Asp Ala Asp Leu Ile Leu Val Val Phe
        195                 200                 205

```
Ser Gly Lys Ala Ile Leu Thr Val Leu Ser Pro Asn Asp Arg Asn Ser
    210                 215                 220

Tyr Asn Leu Glu Arg Gly Asp Thr Ile Lys Ile Pro Ala Gly Thr Thr
225                 230                 235                 240

Ser Tyr Leu Val Asn Gln Asp Asp Glu Gln Asp Leu Glu Ala Phe Asp
                245                 250                 255

Leu Ser Arg Asn Lys Asn Gln Tyr Leu Arg Ala Phe Ser Lys Asn Ile
            260                 265                 270

Leu Glu Ala Ser Leu Asn Thr Lys Tyr Glu Thr Ile Glu Lys Val Leu
        275                 280                 285

Leu Glu Glu Arg Arg Gln Gln Gly Lys Glu Thr Asn Ala Ile Val Lys
    290                 295                 300

Leu Ser Arg Glu Gln Ile Ala Glu Leu Arg Lys Leu Ala Lys Ser Ser
305                 310                 315                 320

Ser Lys Arg Ser Leu Pro Ser Lys Phe Glu Pro Ile Asn Leu Arg Ser
                325                 330                 335

Gln Asn Pro Lys Tyr Ser Asn Lys Phe Gly Arg Leu Phe Glu Ile Thr
            340                 345                 350

Pro Glu Lys Lys Tyr Pro Gln Leu Gln Asp Leu Asp Ile Phe Val Ser
        355                 360                 365

Phe Ser Glu Ile Ser Glu Gly Ala Leu Leu Leu Pro His Tyr Asn Ser
    370                 375                 380

Arg Ala Ile Val Val Leu Val Val Asn Glu Gly Gln Gly Asn Leu Glu
385                 390                 395                 400

Leu Val Gly Phe Lys Asn Glu Gln Gln Glu Gln Ser Leu Lys Glu Asp
                405                 410                 415

Glu Gln Gln Glu Arg Asn Lys Gln Val Gln Arg Tyr Glu Ala Arg Leu
            420                 425                 430

Ser Pro Gly Asp Val Val Val Ile Pro Ser Gly His Pro Phe Ser Val
        435                 440                 445

Ser Ala Leu Ser Asn Leu Thr Leu Phe Gly Phe Gly Ile Asn Ala Glu
```

450 455 460

Asn Asn Glu Arg Asn Phe Leu Thr Gly Ser Asp Asp Asn
465 470 475

<210> 39
<211> 497
<212> PRT
<213> NONE

<400> 39

Met Ala Thr Thr Asn Lys Ser Arg Phe Pro Leu Val Leu Leu Leu Gly
1 5 10 15

Ile Ile Phe Leu Ala Ser Val Cys Val Thr Cys Ala Asn Tyr Asp Glu
20 25 30

Gly Ser Glu Pro Arg Val Pro Gly Gln Arg Glu Arg Gly Arg Gln Glu
35 40 45

Gly Glu Lys Glu Glu Lys Arg His Gly Glu Trp Ser Pro Ser Arg Glu
50 55 60

Lys Glu Ala Gln Pro Gly Arg Glu Arg Trp Glu Arg Lys Glu Asp Glu
65 70 75 80

Glu Gln Val Glu Glu Glu Trp Arg Gly Ser Gln Arg His Glu Asp Pro
85 90 95

Asp Glu Arg Ala Arg Glu Arg Tyr Arg Ala Glu Thr Thr Lys Lys Arg
100 105 110

Val Glu Glu Glu Arg Glu Glu Arg Tyr Thr Pro Tyr Gln Pro Glu Gly
115 120 125

Glu Glu Glu Lys Gly Ser Ser Lys Ser Gln Glu Arg Arg Asn Pro Phe
130 135 140

Leu Phe Lys Ser Asn Lys Phe Leu Thr Leu Phe Glu Asn Glu Asn Gly
145 150 155 160

His Ile Arg Leu Leu Gln Arg Phe Asp Lys Arg Ser Asp Leu Phe Glu
165 170 175

Asn Leu Gln Asn Tyr Arg Leu Val Glu Tyr Arg Ala Lys Pro His Thr
180 185 190

Ile Phe Leu Pro Gln His Ile Glu Ala Asp Leu Ile Leu Thr Val Leu

```
        195                 200                 205

Ser Gly Lys Ala Ile Leu Thr Val Leu Ser Pro Asn Asp Arg Asn Ser
    210                 215                 220

Tyr Asn Leu Glu Arg Gly Asp Thr Ile Lys Val Pro Ala Gly Thr Thr
225                 230                 235                 240

Ser Tyr Leu Val Asn Gln Asp Asp Glu Gln Asp Leu Arg Val Val Asp
                245                 250                 255

Leu Ala Ile Ser Val Asn Arg Pro Gly Lys Val Glu Ser Phe Asn Leu
            260                 265                 270

Tyr Gly Asn Lys Asn Gln Tyr Leu Arg Gly Phe Ser Lys Asn Ile Leu
        275                 280                 285

Glu Ala Ser Phe Asn Thr Lys Tyr Glu Thr Ile Glu Lys Val Leu Leu
    290                 295                 300

Glu Glu Gln Asp Lys Glu Ser Gln Gln Ser Ile Gly Gln Arg Arg Arg
305                 310                 315                 320

Ser Gln Arg Gln Glu Thr Asn Ala Leu Val Arg Val Ser Arg Gln Gln
                325                 330                 335

Ile Glu Glu Leu Lys Arg Leu Ala Lys Ser Ser Ser Lys Lys Gly Val
            340                 345                 350

Ser Ser Glu Leu Glu Pro Phe Asn Leu Arg Ser Gln Asn Pro Lys Tyr
        355                 360                 365

Ser Asn Lys Phe Gly Lys Leu Phe Glu Ile Thr Pro Glu Lys Lys Tyr
    370                 375                 380

Pro Gln Leu Gln Asp Leu Asp Ile Phe Ile Ser Ser Val Glu Ile Lys
385                 390                 395                 400

Glu Gly Ala Leu Ile Leu Pro His Tyr Asn Ser Arg Ala Ile Val Val
                405                 410                 415

Leu Leu Val Asn Glu Gly Lys Gly Asn Leu Glu Leu Val Gly Ile Gln
            420                 425                 430

Asn Glu Gln Gln Glu Gln Gln Glu Arg Asn Lys Gln Val Gln Arg Tyr
        435                 440                 445
```

Glu Ala Arg Leu Ser Pro Gly Asp Val Val Ile Ile Pro Ala Gly His
    450                 455                 460

Pro Val Ala Val Ser Ala Ser Ser Asn Leu Asn Leu Phe Ala Phe Gly
465                 470                 475                 480

Ile Asn Ala Glu Asn Asn Gln Arg Asn Phe Leu Thr Gly Ser Asp Asp
                485                 490                 495

Asn

<210>  40
<211>  127
<212>  PRT
<213>  NONE

<400>  40

Met Gly Tyr Val Arg Val Ala Pro Leu Ala Leu Phe Leu Leu Ala Thr
1               5                   10                  15

Ser Met Met Phe Ser Met Lys Lys Ile Glu Ala Val Val Cys Ser Gly
            20                  25                  30

Val Cys Ser Pro Phe Glu Met Pro Pro Cys Gly Ser Ser Asp Cys Arg
        35                  40                  45

Cys Val Pro Tyr Gly Leu Phe Ile Gly Ser Cys Ile His Pro Thr Gly
    50                  55                  60

Leu Ser Ala Ala Ala Lys Met Ile Asp Glu His Pro Asn Leu Cys Gln
65                  70                  75                  80

Ser His Glu Glu Cys Met Lys Lys Gly Ser Gly Asn Phe Cys Ala Arg
                85                  90                  95

Tyr Pro Asn His Tyr Met Asp Tyr Gly Trp Cys Phe Asn Ser Asp Ser
            100                 105                 110

Asp Ala Leu Lys Gly Phe Leu Ala Met Pro Arg Ala Ile Ser Lys
        115                 120                 125

<210>  41
<211>  127
<212>  PRT
<213>  NONE

<400>  41

Met Gly Tyr Val Arg Val Ala Pro Leu Ala Leu Phe Leu Leu Ala Thr

```
1               5                   10                  15

Ser Met Met Phe Ser Met Lys Lys Ile Glu Ala Val Val Cys Ser Gly
            20                  25                  30

Phe Cys Ser Pro Phe Glu Met Pro Pro Cys Gly Ser Ser Asp Cys Arg
        35                  40                  45

Cys Val Pro Tyr Gly Leu Phe Val Gly Ser Cys Ile His Pro Thr Gly
    50                  55                  60

Leu Ser Ala Ala Ala Lys Met Ile Asp Glu His Pro Asn Leu Cys Gln
65                  70                  75                  80

Phe His Glu Glu Cys Met Lys Lys Gly Ser Gly Asn Phe Cys Ala Arg
                85                  90                  95

Tyr Pro Asn His Tyr Met Asp Tyr Gly Trp Cys Phe Asn Ser Asp Phe
            100                 105                 110

Asp Ala Leu Lys Gly Phe Leu Ala Met Pro Arg Ala Ile Ser Lys
        115                 120                 125


<210>  42
<211>  126
<212>  PRT
<213>  NONE

<400>  42

Met Ala Tyr Val Arg Val Ala Pro Leu Ala Leu Phe Leu Leu Ala Thr
1               5                   10                  15

Ser Ile Met Phe Pro Met Lys Ile Glu Ala Val Asp Cys Ser Gly Ala
            20                  25                  30

Cys Ser Pro Phe Glu Val Pro Pro Cys Gly Ser Arg Asp Cys Arg Cys
        35                  40                  45

Ile Pro Ile Ala Leu Phe Val Gly Phe Cys Ile Tyr Pro Thr Gly Leu
    50                  55                  60

Ser Ser Val Ser Lys Met Ile Asp Glu His Pro Asn Leu Cys Gln Ser
65                  70                  75                  80

His Gly Glu Cys Met Lys Lys Gly Ser Gly Asn Phe Cys Ala Arg Tyr
                85                  90                  95

Pro Asn His Tyr Val Asp Tyr Gly Trp Cys Phe Asn Ser Gly Ser Glu
```

100 105 110

Glu Leu Lys Gly Phe Leu Ala Met Pro Arg Ala Ile Ser Lys
115 120 125

<210> 43
<211> 128
<212> PRT
<213> NONE

<400> 43

Met Gly Tyr Val Arg Val Ala Pro Leu Ala Leu Phe Leu Leu Ala Thr
1 5 10 15

Ser Ile Met Phe Pro Met Lys Lys Thr Glu Ala Val Val Cys Ser Gly
20 25 30

Leu Cys Ser Pro Phe Glu Val Pro Pro Cys Gly Ser Ala Arg Asp Cys
35 40 45

Arg Cys Ile Pro Val Gly Leu Val Val Cys Phe Cys Thr Asn Pro Ser
50 55 60

Gly Leu Ser Ser Val Ala Lys Thr Ile Asp Glu His Pro Asn Ile Cys
65 70 75 80

Gln Thr His Glu Glu Cys Thr Lys Lys Gly Ser Gly Asn Phe Cys Ala
85 90 95

Arg Tyr Pro Asn His Tyr Met Asp Tyr Gly Trp Cys Phe Asn Ser Gly
100 105 110

Ser Glu Glu Leu Lys Gly Phe Leu Ala Met Pro Arg Ala Ile Ser Lys
115 120 125

<210> 44
<211> 128
<212> PRT
<213> NONE

<400> 44

Met Gly Tyr Val Arg Val Ala Pro Leu Ala Leu Phe Leu Leu Ala Thr
1 5 10 15

Ser Ile Met Phe Pro Met Lys Lys Thr Glu Ala Val Val Cys Ser Gly
20 25 30

Val Cys Ser Pro Phe Glu Arg Pro Pro Cys Gly Ser Thr Arg Asp Cys
35 40 45

Arg Cys Ile Pro Tyr Gly Leu Phe Ile Gly Ala Cys Thr Asn Pro Ser
    50                  55                  60

Gly Leu Ser Ser Val Ala Lys Thr Ile Asp Glu His Pro Asn Ile Cys
65                  70                  75                  80

Gln Thr His Glu Glu Cys Thr Lys Lys Gly Ser Gly Asn Phe Cys Ala
                85                  90                  95

Arg Tyr Pro Asn Asp Tyr Met Asp Tyr Gly Trp Cys Phe Asn Ser Asp
            100                 105                 110

Ser Glu Glu Leu Lys Gly Phe Leu Ala Met Pro Arg Ala Ile Ser Lys
        115                 120                 125

<210> 45
<211> 127
<212> PRT
<213> NONE

<400> 45

Met Gly Tyr Val Arg Val Ala Pro Leu Thr Leu Phe Leu Leu Ala Thr
1               5                   10                  15

Ser Ile Met Phe Trp Met Lys Lys Thr Glu Ala Val Val Cys Ser Gly
            20                  25                  30

Val Cys Ser Pro Phe Glu Met Pro Pro Cys Gly Ser Ser Asp Cys Arg
        35                  40                  45

Cys Ile Pro Tyr Gly Leu Phe Ile Gly Ala Cys Thr Tyr Pro Arg Gly
    50                  55                  60

Leu Ser Ser Val Ala Lys Thr Ile Asp Glu His Pro Asn Leu Cys Gln
65                  70                  75                  80

Ser His Gly Glu Cys Met Lys Lys Gly Ser Gly Asn Phe Cys Ala Arg
                85                  90                  95

Tyr Pro Asn Asp Tyr Val Asp Tyr Gly Trp Cys Phe Asn Ser Asp Ser
            100                 105                 110

Asp Glu Leu Lys Gly Phe Leu Ala Met Pro Arg Glu Ile Ser Lys
        115                 120                 125

<210> 46
<211> 127

<212> PRT
<213> NONE

<400> 46

Met Gly Tyr Val Arg Val Ala Pro Leu Ala Leu Phe Leu Leu Ala Thr
1               5                   10                  15

Ser Met Met Phe Ser Met Lys Lys Ile Glu Ala Val Val Cys Ser Gly
            20                  25                  30

Phe Cys Ser Pro Phe Glu Met Pro Pro Cys Gly Ser Ser Asp Cys Arg
        35                  40                  45

Cys Val Pro Tyr Gly Leu Phe Val Gly Ser Cys Ile His Pro Thr Gly
    50                  55                  60

Leu Ser Ala Ala Ala Lys Met Ile Asp Glu His Pro Asn Leu Cys Gln
65                  70                  75                  80

Phe His Glu Glu Cys Met Lys Lys Gly Ser Gly Asn Phe Cys Ala Arg
                85                  90                  95

Tyr Pro Asn His Tyr Met Asp Tyr Gly Trp Cys Phe Asn Ser Asp Ser
            100                 105                 110

Asp Ala Leu Lys Gly Phe Leu Ala Met Pro Arg Ala Ile Ser Lys
        115                 120                 125

<210> 47
<211> 127
<212> PRT
<213> NONE

<400> 47

Met Gly Tyr Val Arg Val Ala Pro Ser Ala Leu Phe Leu Leu Ala Thr
1               5                   10                  15

Ser Ile Met Phe Ser Met Lys Lys Thr Glu Ala Val Val Cys Ser Gly
            20                  25                  30

Val Cys Ser Pro Phe Glu Met Pro Pro Cys Gly Ser Ser Asp Cys Arg
        35                  40                  45

Cys Ile Pro Tyr Gly Leu Phe Ile Gly Ala Cys Thr Tyr Pro Arg Gly
    50                  55                  60

Leu Ser Ser Val Ala Lys Thr Ile Gly Glu His Pro Asn Leu Cys Gln
65                  70                  75                  80

Ser His Gly Glu Cys Ile Lys Lys Gly Ser Gly Asn Phe Cys Ala Arg
85 90 95

Tyr Pro Asn Asp Tyr Val Asp Tyr Gly Trp Cys Ser Asn Ser Asp Ser
100 105 110

Glu Glu Leu Lys Gly Phe Leu Ala Met Pro Arg Glu Ile Ser Lys
115 120 125

<210> 48
<211> 132
<212> PRT
<213> NONE

<400> 48

Met Gly Phe Val Arg Val Ala Pro Leu Ala Leu Phe Phe Leu Val Thr
1 5 10 15

Ser Ile Leu Val Met Phe Ser Met Lys Asn Ile Glu Ala Val Asp Cys
20 25 30

Ser Gly Ala Cys Ser Pro Ala Ser His Glu Pro Pro Cys Gly Ser Ser
35 40 45

Asp Cys Arg Cys Leu Pro Val Ile Ala Pro Phe Val Gly Thr Cys Tyr
50 55 60

His Pro Thr Ala Leu Ser Ser Ser Val Ala Lys Met Ile Asp Glu His
65 70 75 80

Pro Asn Leu Cys Glu Ser Asp Glu Glu Cys Ile Lys Lys Gly Thr Gly
85 90 95

Asn Phe Cys Ala Arg Tyr Pro Asn His Tyr Val Asp Tyr Gly Trp Cys
100 105 110

Phe Asn Ser Gly Ser Asp Glu Leu Lys Gly Phe Leu Ala Met Pro Thr
115 120 125

Ala Ile Ser Lys
130

<210> 49
<211> 227
<212> PRT
<213> NONE

<400> 49

```
Met Ala Tyr Ile Asn Ala Ala Phe Arg Ser Ser Arg Glu Tyr Glu Val
1               5                   10                  15

Tyr Phe Phe Leu Lys Lys Asn His Tyr Val Arg Val His Tyr Thr Pro
            20                  25                  30

Gly Lys Thr Asp Asp Lys Ile Leu Thr Asn Leu Arg Leu Ile Ser Ser
        35                  40                  45

Gly Phe Pro Ser Leu Ala Gly Thr Pro Phe Ala Glu Gln Gly Ile Asp
    50                  55                  60

Ser Ala Phe Asp Thr Glu Gly Lys Glu Ala Tyr Val Phe Ser Thr Lys
65                  70                  75                  80

His Cys Ala Tyr Ile Asp Tyr Ala Pro Gly Thr Thr Asn Asp Lys Ile
                85                  90                  95

Leu Lys Gly Pro Thr Thr Ile Ala Glu Met Phe Pro Val Leu Lys Asn
            100                 105                 110

Thr Val Phe Glu Asn Gly Ile Asp Ser Ala Phe Arg Ser Ile Lys Gly
        115                 120                 125

Lys Glu Val Tyr Leu Phe Lys Gly Asn Lys Tyr Thr Arg Ile Asp Tyr
    130                 135                 140

Asp Ser Lys Gln Leu Ile Gly Asn Ile Arg Asn Ile Thr Asp Gly Phe
145                 150                 155                 160

Thr Val Leu Lys Gly Thr Val Phe Glu Ser Gly Ile Asp Ala Cys Phe
                165                 170                 175

Ala Ser His Glu Glu Ser Glu Ala Tyr Leu Phe Lys Gly Asn Gln Tyr
            180                 185                 190

Val Arg Ile Lys Tyr Thr Pro Gly Ser Ser Asn Asp Thr Leu Leu Gly
        195                 200                 205

Asp Val Arg Pro Ile Leu Asp Gly Trp Pro Cys Leu Lys Asp Ile Leu
    210                 215                 220

Ser Gly Asn
225
```

<210> 50
<211> 358
<212> PRT

<213> NONE

<400> 50

```
Met Glu Leu Asp Leu Thr Pro Lys Val Ala Glu Thr Leu Leu Glu Gly
1               5                   10                  15

Asp Gly Gly Gly Tyr Tyr Ile Trp Leu Ser Ser Gln Val Pro Val Leu
            20                  25                  30

Ala Lys Thr Asn Val Gly Ala Gly Gln Leu Val Leu Arg Pro Arg Gly
        35                  40                  45

Phe Ala Leu Pro His Tyr Ala Asp Ser Asn Lys Ile Gly Tyr Val Ile
    50                  55                  60

Gln Gly Thr Asn Gly Val Ala Gly Leu Val Leu Pro Asn Ser Gly Lys
65                  70                  75                  80

Glu Val Val Val Lys Leu Lys Lys Gly Asp Val Ile Pro Val Pro Ile
                85                  90                  95

Gly Gly Val Ser Trp Trp Phe Asn Asp Gly Asp Ser Asp Leu Asn Ile
            100                 105                 110

Ile Phe Leu Gly Glu Thr Ser Lys Ala His Val Pro Gly Glu Phe Thr
        115                 120                 125

Tyr Phe Phe Leu Asn Gly Ile Gln Gly Leu Leu Ala Ser Phe Ser Ser
    130                 135                 140

Asp Leu Ile Ser Lys Val Tyr Asn Phe Asn Gln Asp Glu Val Asn Lys
145                 150                 155                 160

Leu Thr Gln Ser Gln Ser Gly Val Val Ile Ile Ser Val Glu Lys Gly
                165                 170                 175

Gln Pro Met Pro Lys Pro Gln Leu Glu Leu Thr Lys Glu Leu Val Tyr
            180                 185                 190

Asp Ile Asp Ala Ala Gly Pro Asn Ile Glu Ala Gln Asn Gly Gly Leu
        195                 200                 205

Val Thr Ile Leu Thr Asp Lys Glu Leu Pro Phe Ile Lys Glu Val Gly
    210                 215                 220

Leu Ser Val Ile Arg Val Lys Leu Glu Pro Asn Ala Ile Lys Ala Pro
225                 230                 235                 240
```

Ser Asn Leu Ile Thr Pro Gly Ile Gln Leu Ile Tyr Ile Ala Arg Gly
245 250 255

Gly Gly Lys Ile Glu Ile Val Gly Leu Asn Gly Lys Arg Val Leu Asp
260 265 270

Ala Gln Val Lys Val Gly His Leu Ile Val Val Pro Arg Phe Phe Val
275 280 285

Val Ala Lys Ile Ala Gly Glu Glu Gly Met Glu Thr Tyr Ser Ile Val
290 295 300

Thr Thr Thr Lys Pro Leu Phe Glu Glu Leu Ala Gly Lys Lys Ser Val
305 310 315 320

Trp Gly Val Leu Ser Pro Thr Val Gln Glu Val Ser Phe Asn Val Asn
325 330 335

Ser Glu Phe Gln Lys Leu Phe Ile Ser Lys Ala Thr Leu Asp Thr Asn
340 345 350

Leu Ile Pro Pro Thr Ile
355

<210> 51
<211> 496
<212> PRT
<213> NONE

<400> 51

Met Ala Lys Leu Leu Ala Leu Ser Leu Ser Phe Cys Phe Leu Leu Phe
1 5 10 15

Gly Thr Cys Phe Ala Leu Arg Asp Gln Pro Gln Gln Asn Glu Cys Gln
20 25 30

Leu Glu His Leu Asn Ala Leu Lys Pro Asp Asn Arg Ile Lys Ser Glu
35 40 45

Gly Gly Leu Ile Glu Thr Trp Asn Pro Ser Asn Lys Gln Phe Ala Cys
50 55 60

Ala Gly Val Ala Leu Ser Arg Ala Thr Leu Gln Pro Asn Ser Leu Leu
65 70 75 80

Gln Thr Phe Leu His Gln Arg Ser Pro Glu Ile Phe Ile Gln Gln Gly
85 90 95

Asn Gly Tyr Phe Gly Met Val Phe Pro Gly Cys Val Glu Thr Phe Glu
100 105 110

Glu Pro Arg Glu Ser Glu Gln Gly Glu Gly Ser Lys Phe Ser Asp Ser
115 120 125

His Gln Lys Val Asn Arg Phe Arg Glu Gly Asp Ile Ile Ala Val Pro
130 135 140

Thr Gly Val Val Phe Trp Met Phe Asn Asp Gln Asp Thr Pro Val Ile
145 150 155 160

Ala Val Ser Leu Ile Asp Thr Ser Ser Phe Gln Asn Gln Leu Asp Gln
165 170 175

Met Pro Arg Arg Phe Tyr Leu Ala Gly Asn His Glu Gln Glu Phe Leu
180 185 190

Arg Tyr Gln Gln Glu Gly Ser Glu Glu Glu Glu Asn Glu Gly Gly Asn
195 200 205

Ile Phe Ser Gly Phe Lys Arg Asp Phe Leu Glu Asp Ala Leu Asn Val
210 215 220

Asn Arg Arg Ile Val Asn Lys Leu Gln Gly Arg Asn Glu Asp Glu Glu
225 230 235 240

Lys Gly Ala Ile Val Lys Val Lys Gly Gly Leu Ser Ile Thr Thr Pro
245 250 255

Pro Glu Lys Glu Pro Arg Gln Lys Arg Gly Ser Arg Gln Glu Glu Asp
260 265 270

Glu Asp Glu Asp Glu Lys Arg Gln Pro His Arg His Ser Arg Gln Asp
275 280 285

Glu Asp Glu Asp Glu Lys Arg Gln Pro His His His Ser Arg Gly Gly
290 295 300

Ser Lys Ser Gln Arg Asp Asn Gly Phe Glu Glu Thr Ile Cys Thr Ala
305 310 315 320

Arg Leu His Gln Asn Ile Gly Ser Ser Ser Ser Pro Asp Ile Tyr Asn
325 330 335

Pro Gln Ala Gly Arg Ile Lys Thr Val Thr Ser Phe Asp Leu Gln Ala
340 345 350

Leu Arg Phe Leu Lys Leu Ser Ala Glu Phe Gly Ser Leu His Lys Asn
355 360 365

Ala Met Phe Val Pro His Tyr Asn Leu Asn Ala Asn Ser Ile Leu Tyr
370 375 380

Ala Leu Lys Gly Arg Ala Arg Leu Leu Tyr Ala Leu Asn Cys Lys Gly
385 390 395 400

Asn Ser Val Phe Asp Gly Glu Leu Glu Ala Gly Arg Ala Leu Ile Val
405 410 415

Pro Gln Asn Phe Ala Ile Ala Ala Lys Ser Leu Ser Asp Arg Phe Ser
420 425 430

Tyr Val Ala Phe Lys Thr Asn Asp Arg Ala Leu Ile Asn Val Cys Gln
435 440 445

Lys Lys Leu Leu Gln Leu Leu Ser Ile Trp Lys Glu Met Arg Pro Gly
450 455 460

Ser Ser Ser Ser Thr Ala Pro Phe His Phe Leu Phe His Pro Ala Val
465 470 475 480

Thr Gln Thr Thr Lys Gln Gln Leu Asp Leu Val Pro Asn Gln Tyr Glu
485 490 495

<210> 52
<211> 499
<212> PRT
<213> NONE

<220>
<221> misc_feature
<222> (189)..(196)
<223> Xaa can be any naturally occurring amino acid

<400> 52

Met Ala Lys Leu Leu Ala Leu Ser Leu Ser Phe Cys Phe Leu Leu Phe
1 5 10 15

Gly Ser Cys Phe Ala Leu Arg Asp Gln Pro Glu Gln Asn Glu Cys Gln
20 25 30

Leu Glu His Leu Asn Ala Leu Glu Pro Asp Asn Arg Ile Lys Ser Glu
35 40 45

```
Gly Gly Leu Ile Glu Thr Trp Asn Pro Ser Asn Lys Gln Phe Arg Cys
    50                  55                  60

Ala Gly Val Ala Leu Ser Arg Ala Thr Leu Gln Pro Asn Ser Leu Arg
65                  70                  75                  80

Arg Pro Phe Tyr Thr Asn Ala Pro Gln Glu Ile Phe Ile Gln Gln Gly
                85                  90                  95

Asn Gly Tyr Phe Gly Met Val Phe Pro Gly Cys Val Glu Thr Phe Glu
            100                 105                 110

Glu Pro Arg Glu Ser Glu Gln Gly Glu Gly Ser Lys Phe Arg Asp Ser
        115                 120                 125

His Gln Lys Val Asn Arg Phe Arg Glu Gly Asp Ile Ile Ala Val Pro
    130                 135                 140

Thr Gly Val Val Phe Trp Met Phe Asn Asp Gln Asp Thr Pro Val Ile
145                 150                 155                 160

Ala Val Ser Leu Ile Asp Thr Ser Ser Phe Gln Asn Gln Leu Asp Gln
                165                 170                 175

Met Pro Arg Arg Phe Tyr Leu Ala Gly Asn His Glu Xaa Xaa Xaa Xaa
            180                 185                 190

Xaa Xaa Xaa Xaa Gln Gln Glu Gly Ser Glu Glu Glu Glu Asn Glu Gly
        195                 200                 205

Gly Asn Ile Phe Ser Gly Phe Lys Arg Asp Phe Leu Glu Asp Ala Leu
    210                 215                 220

Asn Val Asn Arg Arg Ile Val Asn Lys Leu Gln Gly Arg Asn Glu Asp
225                 230                 235                 240

Glu Glu Lys Gly Ala Ile Val Lys Val Lys Gly Gly Leu Ser Ile Ile
                245                 250                 255

Thr Pro Pro Glu Lys Glu Pro Arg Gln Lys Arg Gly Ser Arg Gln Glu
            260                 265                 270

Glu Asp Glu Asp Glu Asp Glu Lys Arg Gln Pro His Arg His Ser Arg
        275                 280                 285

Gln Asp Glu Asp Glu Asp Glu Lys Arg Gln Pro Arg Arg His Ser Arg
    290                 295                 300
```

Gly Gly Ser Lys Ser Gln Arg Asp Asn Gly Phe Glu Glu Thr Ile Cys
305 310 315 320

Thr Ala Arg Leu His Gln Asn Ile Gly Ser Ser Ser Ser Pro Asp Ile
325 330 335

Tyr Asn Pro Gln Ala Gly Arg Ile Lys Thr Val Thr Ser Phe Asp Leu
340 345 350

Pro Ala Leu Arg Phe Leu Lys Leu Ser Ala Glu Phe Gly Ser Leu His
355 360 365

Lys Asn Ala Met Phe Val Pro His Tyr Asn Leu Asn Ala Asn Ser Ile
370 375 380

Leu Tyr Ala Leu Lys Gly Arg Ala Arg Leu Gln Ile Val Asn Cys Lys
385 390 395 400

Gly Asn Ser Val Phe Asp Gly Glu Leu Glu Ala Gly Arg Ala Leu Ile
405 410 415

Val Pro Gln Asn Phe Ala Ile Ala Ala Lys Ser Leu Ser Asp Arg Phe
420 425 430

Ser Tyr Val Ala Phe Lys Thr Asn Asp Arg Ala Ala Ile Gly Arg Leu
435 440 445

Leu Gly Ala Ser Ser Leu Ile Asn Gly Met Pro Glu Glu Val Val Ala
450 455 460

Ala Ala Phe Asn Met Glu Arg Asn Glu Ala Arg Gln Leu Lys Phe Asn
465 470 475 480

Ser Pro Phe Ser Phe Leu Val Pro Pro Arg Ser Asp Ser Asp Asn Lys
485 490 495

Ala Ala Ala

<210> 53
<211> 532
<212> PRT
<213> NONE

<400> 53

Met Arg Arg Ser Asn Ser Tyr Leu Ser Ser Leu Ser Leu Cys Phe Leu
1 5 10 15

Leu Phe Thr Ser Ala Cys Leu Ala Thr Arg Ser Glu Glu Phe Asp Arg
20 25 30

Phe Asn Gln Cys Gln Leu Asp Ser Ile Asn Ala Leu Glu Pro Asp His
35 40 45

Arg Val Glu Ser Glu Ala Gly Leu Thr Glu Thr Trp Asn Pro Asn His
50 55 60

Pro Glu Leu Gln Cys Ala Gly Val Ser Leu Ile Arg Arg Thr Ile Asp
65 70 75 80

Pro Asn Gly Leu His Ile Pro Ser Phe Ser Pro Ser Pro Gln Leu Ile
85 90 95

Phe Ile Val Gln Gly Lys Gly Val Leu Gly Leu Ser Ile Pro Gly Cys
100 105 110

Pro Thr Thr Phe Glu Glu Gln Arg Gln His Phe Asp Asn His Gln Lys
115 120 125

Ile Arg Arg Phe Ser Lys Gly Asp Ile Ile Ala Ile Pro Pro Gly Ile
130 135 140

Pro Tyr Trp Ser Tyr Asn Asn Gly Asp Glu Pro Val Val Ala Ile Thr
145 150 155 160

Leu Leu Asp Thr Ser Asn Phe Ala Asn Gln Leu Asp Ser Thr Pro Arg
165 170 175

Val Phe Tyr Leu Gly Gly Asn Pro Glu Val Glu Phe Pro Glu Thr Gln
180 185 190

Gln Gln Gln Gln Gln Gln Lys Arg His Ser Leu Pro Phe Pro Gly Gly
195 200 205

Arg Lys Gly Gly Lys Asn Gln Gln Glu Glu Gln Asn Glu Gly Asn Ser
210 215 220

Val Leu Ser Gly Phe Ser Ser Glu Phe Leu Ala Gln Ala Leu Asn Thr
225 230 235 240

Asp Glu Asp Thr Ala Lys Lys Leu Gln Ser Pro Arg Asp Gln Arg Ala
245 250 255

Gln Ile Val Arg Val Glu Gly Gly Leu Arg Ile Ile Ser Pro Asp Leu
260 265 270

Gln Asp Glu Glu Asp Glu Asp Glu Asp Glu Asp Glu Gln Glu Gln Gly
275 280 285

His Ser Gln Arg Glu Glu Glu Glu Asp Asp Asp Glu Asp Glu Ser His
290 295 300

Ser His Glu Ser Arg Gln Lys Trp Arg Lys Tyr Arg Glu Glu Glu Lys
305 310 315 320

Gln Gly Ser His Arg Arg Glu His Lys Glu Glu Glu Glu Glu Glu Glu
325 330 335

Glu Lys Ile Cys Lys Lys Arg Ser Cys Arg Gly Lys Gly Arg Lys Asn
340 345 350

Gly Leu Glu Glu Thr Ile Cys Ser Ala Arg Ile Arg Glu Asn Ile Ile
355 360 365

Arg Pro Ala Arg Ala Asp Leu Tyr Asn Pro Arg Ala Gly Arg Ile Ser
370 375 380

Thr Val Asn Ser Leu Thr Leu Pro Ile Leu Ser Tyr Leu Arg Leu Ser
385 390 395 400

Ala Glu Tyr Val Leu Leu Tyr Arg Asn Gly Ile Asn Ala Pro His Trp
405 410 415

Asn Met Asn Ala Asn Ser Leu Met Tyr Val Val Arg Gly Glu Gly Lys
420 425 430

Val Arg Ile Val Asn Cys Glu Gly Lys Ala Val Phe Asp Asp Asn Val
435 440 445

Arg Lys Gly Gln Met Leu Val Val Pro Gln Asn Phe Val Val Ala Glu
450 455 460

Gln Ala Gly Asn Glu Glu Gly Phe Glu Tyr Val Val Phe Lys Thr Asn
465 470 475 480

Asp Arg Ala Ala Val Ser Asn Val Lys Gln Val Phe Arg Ala Thr Pro
485 490 495

Ala Gln Val Leu Ala Asn Ala Phe Gly Leu Arg Gln Asn Glu Val Thr
500 505 510

Lys Ile Lys Phe Ser Gly Asn Arg Gly Pro Leu Val Gln Pro Arg Ser

515 520 525

His Ala His Ala
530

<210> 54
<211> 518
<212> PRT
<213> NONE

<400> 54

Met Ala Lys Leu Leu Ala Leu Ser Leu Ser Leu Cys Phe Leu Leu Phe
1 5 10 15

Gly Gly Cys Phe Ala Phe Arg Glu Gln Pro Gln Gln Asn Glu Cys Gln
20 25 30

Leu Glu His Leu Asn Ala Leu Glu Pro Asp Asn Arg Ile Glu Ser Glu
35 40 45

Gly Gly Leu Ile Glu Thr Trp Asn Pro Asn Asn Arg Gln Phe Arg Cys
50 55 60

Ala Gly Val Ala Leu Ser Arg Ala Thr Leu Gln Arg Asn Ala Leu Arg
65 70 75 80

Arg Pro Phe Tyr Thr Asn Ala Pro Gln Glu Ile Phe Ile Gln Gln Gly
85 90 95

Asn Gly Tyr Phe Gly Met Val Phe Pro Gly Cys Pro Glu Thr Phe Met
100 105 110

Glu Pro Arg Glu Ser Glu Gln Arg Glu Gly Arg Arg Phe Arg Asp Ser
115 120 125

His Gln Lys Val Asn Arg Phe Arg Glu Gly Asp Ile Ile Ala Val Pro
130 135 140

Thr Gly Val Val Phe Trp Met Tyr Asn Asp Gln Asp Thr Pro Val Ile
145 150 155 160

Ala Val Ser Leu Ile Asp Thr Ser Ser Phe Gln Asn Gln Leu Asp Gln
165 170 175

Met Pro Arg Arg Phe Tyr Leu Ala Gly Asn Gln Glu Gln Glu Phe Leu
180 185 190

Gln Tyr Gln Gln Gln Glu Gly Arg Lys Glu Glu Gln Glu Asn Glu Gly

```
        195                 200                 205

Asn Asn Ile Phe Ser Gly Phe Arg Arg Asp Phe Leu Glu Asp Ala Leu
    210                 215                 220

Asn Val Asn Arg His Ile Val Asp Lys Leu Gln Gly Arg Asp Glu Asp
225                 230                 235                 240

Glu Glu Lys Gly Ala Ile Val Lys Val Lys Gly Gly Leu Ser Phe Ile
                245                 250                 255

Ser Pro Ser Glu Lys Gln Pro Arg His Gln Arg Gly Ser Arg Gln Glu
            260                 265                 270

Glu Asp Glu Asp Glu Asp Glu Asp Glu Lys Arg Gln Pro His Arg His
        275                 280                 285

Ser Arg Gln Asp Glu Asp Glu Asp Glu Lys Arg Arg Pro Arg Arg His
    290                 295                 300

Ser Arg Gln Asp Glu Asp Glu Asp Glu Glu Glu Lys Arg Gln Pro Arg
305                 310                 315                 320

Arg His Ser Arg Gly Gly Ser Arg Ser Gln Arg Asp Asn Gly Phe Glu
                325                 330                 335

Glu Thr Ile Cys Thr Ala Arg Leu His Gln Asn Ile Asp Ser Ser Ser
            340                 345                 350

Ser Pro Asp Ile Tyr Ile Pro Gln Ala Gly Arg Ile Lys Thr Val Thr
        355                 360                 365

Ser Phe Asp Leu Pro Ala Leu Arg Trp Leu Lys Leu Ser Ala Glu His
    370                 375                 380

Gly Ser Leu His Lys Asn Ala Met Phe Val Pro His Tyr Asn Leu Asn
385                 390                 395                 400

Ala Asn Ser Ile Leu Tyr Ala Leu Lys Gly Arg Ala Arg Leu Gln Val
                405                 410                 415

Val Asn Cys Asn Gly Asn Asn Val Phe Asp Gly Glu Leu Glu Ala Gly
            420                 425                 430

Arg Ala Leu Ile Val Pro Gln Asn Phe Ala Val Ala Ala Lys Ser Met
        435                 440                 445
```

Ser Asp Arg Phe Ser Tyr Val Ala Phe Lys Thr Asn Asp Arg Ala Ala
    450                 455                 460

Ile Gly Lys Leu Val Gly Ala Thr Ser Ser Leu Ser Ser Ile Pro Glu
465                 470                 475                 480

Asp Val Ile Ala Ala Thr Phe Asn Met Glu Arg Asn Glu Ala Arg Lys
                485                 490                 495

Leu Lys Ser Ser Asn Pro Phe Thr Phe Leu Val Pro Pro Arg Glu Ser
            500                 505                 510

Glu Asn Lys Ala Ala Ala
        515

<210> 55
<211> 455
<212> PRT
<213> NONE

<400> 55

Met Cys Trp Cys Cys Pro Leu Ser Cys Tyr Pro Ser Thr Gln Cys Pro
1               5                   10                  15

Ser Gln Thr Phe Leu His Gln Arg Ser Pro Gly Asn Phe His Pro Thr
            20                  25                  30

Gly Asn Gly Tyr Phe Gly Met Val Phe Pro Gly Cys Pro Glu Thr Phe
        35                  40                  45

Met Glu Pro Arg Glu Ser Glu Gln Arg Glu Gly Arg Arg Phe Arg Asp
    50                  55                  60

Ser His Gln Lys Val Asn Arg Phe Arg Glu Gly Asp Ile Ile Ala Val
65                  70                  75                  80

Pro Thr Gly Val Val Phe Trp Met Tyr Asn Asp Gln Asp Thr Pro Val
                85                  90                  95

Ile Ala Val Ser Leu Ile Asp Thr Ser Ser Phe Gln Asn Gln Leu Asp
            100                 105                 110

Gln Met Pro Arg Arg Phe Tyr Leu Ala Gly Asn Gln Glu Gln Glu Phe
        115                 120                 125

Leu Gln Tyr Gln Gln Gln Glu Gly Arg Lys Glu Glu Gln Glu Asn Glu
    130                 135                 140

```
Gly Asn Asn Ile Phe Ser Gly Phe Arg Arg Asp Phe Leu Glu Asp Ala
145                 150                 155                 160

Leu Asn Val Asn Arg His Ile Val Asp Lys Leu Gln Gly Arg Asp Glu
                165                 170                 175

Asp Glu Glu Lys Gly Ala Ile Val Lys Val Lys Gly Gly Leu Ser Phe
            180                 185                 190

Ile Ser Pro Ser Glu Lys Gln Pro Arg His Gln Arg Gly Ser Arg Gln
        195                 200                 205

Glu Glu Asp Glu Asp Glu Asp Glu Asp Glu Lys Arg Gln Pro His Arg
    210                 215                 220

His Ser Arg Gln Asp Glu Asp Glu Asp Glu Lys Arg Arg Pro Arg Arg
225                 230                 235                 240

His Ser Arg Gln Asp Glu Asp Glu Asp Glu Glu Glu Lys Arg Gln Pro
                245                 250                 255

Arg Arg His Ser Arg Gly Gly Ser Arg Ser Gln Arg Asp Asn Gly Phe
            260                 265                 270

Glu Glu Thr Ile Cys Thr Ala Arg Leu His Gln Asn Ile Asp Ser Ser
        275                 280                 285

Ser Ser Pro Asp Ile Tyr Ile Pro Gln Ala Gly Arg Ile Lys Thr Val
    290                 295                 300

Thr Ser Phe Asp Leu Pro Ala Leu Arg Trp Leu Lys Leu Ser Ala Glu
305                 310                 315                 320

His Gly Ser Leu His Lys Asn Ala Met Phe Val Pro His Tyr Asn Leu
                325                 330                 335

Asn Ala Asn Ser Ile Leu Tyr Ala Leu Lys Gly Arg Ala Arg Leu Gln
            340                 345                 350

Val Val Asn Cys Asn Gly Asn Asn Val Phe Asp Gly Glu Leu Glu Ala
        355                 360                 365

Gly Arg Ala Leu Ile Val Pro Gln Asn Phe Ala Val Ala Ala Lys Ser
    370                 375                 380

Met Ser Asp Arg Phe Ser Tyr Val Ala Phe Lys Thr Asn Asp Arg Ala
385                 390                 395                 400
```

Ala Ile Gly Lys Leu Val Gly Ala Thr Ser Ser Leu Ser Ser Ile Pro
405 410 415

Glu Asp Val Ile Ala Ala Thr Phe Asn Met Glu Arg Asn Glu Ala Arg
420 425 430

Lys Leu Lys Ser Ser Asn Pro Phe Thr Phe Leu Val Pro Pro Arg Glu
435 440 445

Ser Glu Asn Lys Ala Ala Ala
450 455

<210> 56
<211> 551
<212> PRT
<213> NONE

<400> 56

Met Ala Lys Pro Asn Pro His Ile Leu His Tyr Phe Phe Ser Leu Phe
1 5 10 15

Cys Leu Leu Leu Phe Thr Cys Gly Cys Ser Ser Arg Gln Leu Pro Arg
20 25 30

Gln Gln Ser Glu Phe Asn Glu Cys Gln Leu Asp Ser Ile Asp Ala Leu
35 40 45

Glu Pro Asp Asn Arg Ile Glu Ser Glu Ala Gly Leu Thr Glu Ile Trp
50 55 60

Asp Ala Asn His Pro Glu Leu Arg Cys Ala Gly Val Ser Val Leu Lys
65 70 75 80

Arg Thr Ile Asn Ala Asn Gly Leu His Leu Pro Ser Tyr Val Thr Tyr
85 90 95

Pro Glu Leu His Phe Val Glu Gln Gly Thr Gly Val Leu Gly Met Ala
100 105 110

Ile Pro Gly Cys Ala Glu Thr Tyr Glu Glu Pro Gln Trp Glu Arg Arg
115 120 125

Gly Arg Pro Gln Leu Gln Gln Asp Arg His Gln Lys Val Arg Tyr Val
130 135 140

Lys Gln Gly Asp Leu Ile Ala Ile Pro Pro Gly Val Pro Tyr Trp Thr
145 150 155 160

```
Tyr Asn Tyr Gly Asn Thr Pro Leu Ile Ile Ile Thr Leu Leu Asp Thr
                165                 170                 175

Ser Asn Pro Leu Asn Gln Leu Asp Arg Thr Pro Thr Arg Phe Tyr Leu
            180                 185                 190

Ala Gly Asn Pro Glu Thr Glu Lys Gln Ser Gly Arg Lys Arg Gly Gln
        195                 200                 205

Glu Glu Glu Glu Glu Glu Ser Asn Asn Met Phe Ser Gly Phe Asp Ser
    210                 215                 220

Arg Phe Leu Gly Gln Val Leu Lys Val Lys Glu Ser Ile Ile Arg Lys
225                 230                 235                 240

Leu Gln Ser Pro Asp Lys His Arg Lys His Gln Ile Ile Glu Val Lys
                245                 250                 255

Gly Gly Leu Ser Ile Ile Arg Pro Pro Leu Glu Pro Glu Ile Arg Ser
            260                 265                 270

Glu Glu Glu Glu Thr His Arg Arg His Thr Glu Lys Arg Val Glu Glu
        275                 280                 285

Arg Glu Glu Glu Val Glu Asp Glu Pro Cys Lys Arg Glu His Arg Glu
    290                 295                 300

Trp Arg Arg Glu Arg Arg Thr His Gly Glu Gly Glu Gly Glu Gly Gly
305                 310                 315                 320

Cys Glu Gly Glu Glu Glu Glu Val Val Glu Glu Lys Glu Thr Lys Thr
                325                 330                 335

Lys Glu Arg Arg Arg His Gly Glu Arg Glu Glu Lys Gln Thr Arg Thr
            340                 345                 350

Thr Thr Arg Glu Arg Glu Gly Gln Lys Asp Asn Ser Leu Glu Glu Thr
        355                 360                 365

Val Cys Thr Leu Lys Leu His Glu Asn Ile Ala Asp Pro Ser Arg Ala
    370                 375                 380

Asp Val Phe Asn Pro Arg Ala Gly Arg Ile Thr Thr Ala Asn Ser Leu
385                 390                 395                 400

Thr Leu Pro Val Leu Lys Ser Leu His Leu Ser Ala Gln Trp Val Asn
                405                 410                 415
```

```
Leu Tyr Lys Asp Gly Ile Phe Val Pro His Trp Asn Met Asn Ser Asn
            420                 425                 430

Gly Val Met Tyr Val Thr Arg Gly Lys Gly Arg Val Gln Val Val Asn
        435                 440                 445

Asn Glu Gly Lys Ser Val Phe Asn Gly Glu Val Gly Arg Gly Gln Leu
    450                 455                 460

Leu Val Val Pro Gln Asn Phe Ala Val Ala Lys Gln Ala Gly Asn Glu
465                 470                 475                 480

Gly Met Glu Tyr Val Ala Phe Lys Thr Asn Asp Arg Ala Glu Ile Ser
                485                 490                 495

Thr Met Ile Gly Arg Asn Ser Ala Ile Ser Ala Thr Pro Gly Glu Val
            500                 505                 510

Leu Ala Asn Ala Phe Gly Leu Ser Pro Glu Glu Val Ile Ala Leu Lys
        515                 520                 525

Asn Asn Arg Asn Glu Gly Val Leu Ala Thr Pro Asp Ser Arg Ile Gln
    530                 535                 540

Asp Gly Tyr Ile Ala Met Leu
545                 550


<210>  57
<211>  440
<212>  PRT
<213>  NONE

<400>  57

Met Ser Asn Ser Ile Phe Gln Tyr Leu Leu Leu Leu Phe Phe Ile Ala
1               5                   10                  15

Pro Thr Phe Ser Gln Gln Ser Phe Arg Pro Lys Ala Leu Val Leu Pro
            20                  25                  30

Val Thr Lys Asp Thr Leu Thr Thr Asn Gln Tyr Ile Ala Gln Ile Asn
        35                  40                  45

Gln Arg Thr Pro Leu Val Pro Leu Asn Leu Val Val Asp Leu Gly Gly
    50                  55                  60

Gln Phe Leu Trp Leu Asp Cys Glu Gln His Tyr Thr Ser Ser Thr Tyr
65                  70                  75                  80
```

```
Arg Pro Ala Arg Cys Arg Ser Ala Gln Cys Ser Leu Ala Lys Ser Thr
                85                  90                  95

Ala Cys Gly Asp Cys Phe Ser Ser Pro Lys Pro Gly Cys Asn Asn Asn
            100                 105                 110

Thr Cys Gly Leu Phe Pro Asp Asn Thr Val Thr His Thr Ala Thr Ser
        115                 120                 125

Gly Glu Leu Ala Glu Asp Val Leu Ser Val Gln Ser Thr Asn Gly Phe
    130                 135                 140

Asn Pro Thr Gln Asn Val Ala Val Ser Arg Phe Leu Phe Ser Cys Ala
145                 150                 155                 160

Pro Thr Ser Leu Leu Gln Gly Leu Ala Ser Gly Ala Ser Gly Met Ala
                165                 170                 175

Gly Leu Gly Arg Thr Lys Ile Ala Phe Pro Ser Gln Leu Ala Ser Ala
            180                 185                 190

Phe Ser Phe Asp Arg Lys Phe Ala Ile Cys Phe Ser Ser Ser Asp Gly
        195                 200                 205

Val Val Leu Phe Gly Asp Gly Pro Tyr Gly Phe Leu Ala Asn Asn Arg
    210                 215                 220

Ser Leu Pro Asn Val Val Tyr Asp Ser Glu Ser Leu Thr Tyr Thr Pro
225                 230                 235                 240

Leu Leu Ile Asn Pro Val Ser Thr Ala Ser Ala Phe Thr Gln Gly Glu
                245                 250                 255

Pro Ser Ala Glu Tyr Phe Ile Gly Val Lys Lys Ile Lys Ile Asp Lys
            260                 265                 270

Lys Val Val Ala Leu Asn Thr Ser Leu Leu Thr Ile Asn Ser Asn Gly
        275                 280                 285

Phe Gly Gly Thr Lys Leu Ser Thr Val Asn Pro Tyr Thr Val Leu Glu
    290                 295                 300

Ser Ser Ile Tyr Lys Ala Val Thr Asp Ala Phe Val Lys Ala Ser Val
305                 310                 315                 320

Ala Arg Asn Ile Thr Arg Thr Asp Ser Phe Glu Pro Phe Glu Phe Cys
```

325 330 335

Tyr Ser Phe Asp Asn Leu Ser Gly Thr Arg Leu Gly Ala Ala Val Pro
340 345 350

Thr Ile Glu Leu Val Leu Gln Asn Glu Asn Val Val Trp Ser Ile Phe
355 360 365

Gly Ala Asn Ser Met Val Asn Ile Asn Asp Asp Val Leu Cys Leu Gly
370 375 380

Phe Met Asn Gly Gly Glu Asn Val Arg Thr Ser Ile Val Ile Gly Gly
385 390 395 400

Tyr Gln Leu Asp Asn Asn Leu Leu Gln Phe Asp Leu Ala Ala Ser Arg
405 410 415

Leu Gly Phe Ser Gly Ser Val Phe Ala Arg Gln Thr Asp Cys Phe Arg
420 425 430

Phe Asn Ser Thr Thr Ser Ile Ala
435 440

<210> 58
<211> 440
<212> PRT
<213> NONE

<400> 58

Met Ala Ser Ile Ser Ile Leu His Phe Leu Phe Cys Phe Phe Leu Leu
1 5 10 15

Val Leu Ser Ser Glu Ser Thr Thr Gln Thr Gln Ser His Ser Gln Ser
20 25 30

Gln Gln Asn Ser Asn Ser Lys Pro Asn Leu Leu Val Leu Pro Val Gln
35 40 45

Gln Asp Ala Asn Thr Gly Leu His Trp Ala Tyr Ile His Lys Arg Thr
50 55 60

Pro Leu Met Gln Leu Pro Val Leu Ile Asp Leu Asn Gly Lys His Leu
65 70 75 80

Trp Val Asn Cys Glu Gln His Tyr Ser Ser Ser Thr Tyr Gln Ala Pro
85 90 95

Phe Cys His Ser Thr Gln Cys Ala Arg Ala Asn Thr His Thr Cys His

100 105 110

Thr Cys Val Ser Ser Phe Arg Pro Gly Cys His Asn Asn Thr Cys Gly
115 120 125

Leu Met Ser Ala Asn Pro Val Thr Gln Gln Thr Ala Leu Gly Glu Leu
130 135 140

Ala Gln Asp Val Phe Ala Ile Tyr Thr Thr Arg Gly Pro Lys Leu Gly
145 150 155 160

Pro Met Val Thr Val Pro Gln Phe Leu Phe Ser Cys Ala Pro Ser Phe
165 170 175

Leu Ala Gln Lys Gly Leu Pro Asn Asn Val Gln Gly Val Ala Gly Leu
180 185 190

Ala His Ser Pro Ile Ser Leu Pro Asn Gln Leu Ser Ser His Phe Gly
195 200 205

Leu Lys His Gln Phe Thr Thr Cys Leu Ser Arg Tyr Pro Lys Ser Asn
210 215 220

Gly Ala Ile Ile Phe Gly Asp Ala Ala Asn Asn Met Arg Phe Gly Asn
225 230 235 240

Asn Arg Asn Tyr Lys Asn Asn Pro Asn Ile Leu Asn Asn Leu Ile Tyr
245 250 255

Thr Pro Leu Thr Ile Thr Pro Glu Gly Glu Tyr Arg Met His Val Thr
260 265 270

Ser Ile Gln Ile Asn Glu His Thr Val Val Pro Val Ser Gly Ser Met
275 280 285

Leu Ser Ser Tyr Pro Glu Gly Val Ile Gly Gly Thr Leu Ile Ser Thr
290 295 300

Ser Ile Pro Tyr Thr Thr Leu Gln Asp Ser Leu Phe Glu Thr Phe Val
305 310 315 320

Gln Val Phe Ala Lys Gln Phe Pro Lys Glu Ala Gln Val Asn Ala Val
325 330 335

Gly Pro Phe Gly Leu Cys Phe Asp Ser Lys Arg Ile Asn Lys Ala Val
340 345 350

Asn Val Glu Phe Val Leu Asp Arg Ser Asp Val Val Trp Arg Ile Ser
355 360 365

Gly Glu Asn Leu Met Val Glu Ala Lys Ser Gly Ile Ser Cys Leu Gly
370 375 380

Ile Val Asn Gly Gly Leu Lys Pro Lys Ala Thr Ile Ser Ile Gly Ser
385 390 395 400

Arg Gln Leu Glu Glu Asn Leu Leu Leu Phe Asp Leu Val Glu Ser Lys
405 410 415

Leu Gly Phe Ser Asn Ser Leu Ser Ser Arg Gly Ile Lys Cys Ser Asp
420 425 430

Leu Phe Asp Phe Asn Asn Asn Ala
435 440

<210> 59
<211> 436
<212> PRT
<213> NONE

<400> 59

Met Ser Ser Ser Asn Phe Gln Leu Gln His Leu Ile Thr Leu Leu Leu
1 5 10 15

Phe Phe Phe Ile Ser Ser Thr Phe Ser Gln Gln Ser Phe Arg Pro Lys
20 25 30

Ala Leu Val Leu Pro Val Thr Lys Asp Thr Ser Thr Thr Asn Gln Tyr
35 40 45

Ile Ala Gln Ile Asn Gln Arg Thr Pro Leu Val Pro Leu Asn Leu Val
50 55 60

Val Asp Leu Gly Gly Gln Phe Leu Trp Leu Asp Cys Glu Gln His Tyr
65 70 75 80

Thr Ser Ser Thr Tyr Arg Pro Ala Arg Cys Arg Ser Ala Gln Cys Ser
85 90 95

Leu Ala Lys Ser Thr Ala Cys Gly Asp Cys Phe Ser Ser Pro Lys Pro
100 105 110

Gly Cys Asn Asn Asn Thr Cys Ser Ile Phe Pro Asp Asn Thr Val Thr
115 120 125

```
His Thr Ala Thr Ser Gly Glu Leu Ala Glu Asp Val Leu Ser Ile Gln
    130                 135                 140

Ser Thr Asn Gly Phe Asn Pro Thr Gln Asn Val Ala Val Ser Arg Phe
145                 150                 155                 160

Leu Phe Ser Cys Ala Pro Thr Ser Leu Leu Glu Gly Leu Ala Ser Gly
                165                 170                 175

Ala Ser Gly Met Ala Gly Leu Gly Arg Thr Lys Ile Ala Phe Pro Ser
            180                 185                 190

Gln Leu Ala Ser Ala Phe Ser Phe Asp Arg Lys Phe Ala Ile Cys Phe
        195                 200                 205

Ser Ser Ser Asp Gly Val Val Leu Phe Gly Asp Gly Pro Tyr Gly Phe
    210                 215                 220

Leu Pro Asn Val Val Tyr Asp Ser Glu Ser Leu Thr Tyr Thr Pro Leu
225                 230                 235                 240

Leu Ile Asn Pro Val Ser Thr Ala Ser Ala Phe Thr Gln Gly Glu Pro
                245                 250                 255

Ser Ala Glu Tyr Phe Ile Gly Val Lys Lys Ile Lys Ile Asp Asp Lys
            260                 265                 270

Val Val Ser Leu Asn Thr Ser Leu Leu Ser Ile Asn Ser Asn Gly Ile
        275                 280                 285

Gly Gly Thr Lys Ile Ser Thr Val Asp Pro Tyr Thr Val Leu Glu Ala
    290                 295                 300

Ser Ile Tyr Lys Ala Val Thr Asp Gly Phe Val Lys Ala Ser Val Ala
305                 310                 315                 320

Arg Asn Leu Thr Arg Val Asp Ser Phe His Pro Phe Glu Phe Cys Tyr
                325                 330                 335

Thr Asn Leu Gly Gly Thr Arg Leu Gly Ala Ala Val Pro Thr Ile Glu
            340                 345                 350

Leu Val Leu Gln Asn Glu Asn Val Val Trp Ser Ile Phe Gly Ala Asn
        355                 360                 365

Ser Met Val Asn Ile Asn Asp Glu Val Ser Cys Leu Gly Phe Val Asn
    370                 375                 380
```

Gly Gly Glu Asn Val Arg Thr Ser Ile Val Ile Gly Gly Tyr Gln Leu
385 390 395 400

Asp Asn Asn Leu Leu Gln Phe Asp Leu Ala Ala Ser Arg Leu Gly Phe
405 410 415

Ser Ser Thr Leu Phe Gly Arg Gln Thr Gly Cys Tyr Asn Phe Asn Phe
420 425 430

Thr Ser Ala Thr
435

<210> 60
<211> 431
<212> PRT
<213> NONE

<400> 60

Met Lys Ile Val Pro Ser Phe Leu Phe Phe Tyr Thr Leu Ile Ile Thr
1 5 10 15

Leu Ile Ser Pro Ser Ile Ala Ser Thr Leu Ile Gln Pro Lys Ala Leu
20 25 30

Val Leu Pro Val Thr Lys Asp Ser Ala Thr Ser Gln Tyr Val Thr Leu
35 40 45

Ile His Gln Arg Thr Pro Leu Leu Pro Ile Lys Leu Thr Leu Asp Leu
50 55 60

Ser Gly Gln Phe Leu Trp Ile Asn Cys Glu Lys Gly Tyr Val Ser Ser
65 70 75 80

Thr Tyr His Pro Ala His Cys Thr Thr Lys Gln Cys Ser Ile Thr Lys
85 90 95

Ser Lys Phe Cys Arg Asn Cys Phe Leu Ser Lys Pro Gly Cys Asn Asn
100 105 110

Asn Thr Cys Asn Leu Phe Pro Asn Asn Ile Phe Thr His Ile Asn Glu
115 120 125

Ile Gly Glu Val Ala Leu Asp Val Val Ala Ile His Ser Thr Asn Gly
130 135 140

Leu Ser Leu Gly Lys Ile Val Thr Val Lys Asn Phe Leu Phe Thr Cys
145 150 155 160

```
Gly Arg Thr Asn Leu Leu Lys Gly Leu Ala Ser Gly Val Lys Gly Met
                165                 170                 175

Ala Gly Phe Gly Arg Ser Glu Ile Ser Val Pro Ser Leu Phe Ala Ser
            180                 185                 190

Ala Phe Ser Leu Asn Lys Lys Phe Ser Ile Cys Leu Ser Ser Ser Ser
        195                 200                 205

Gly Val Leu Phe Phe Gly Asp Gly Pro Phe Val Phe Leu Pro Arg Ile
    210                 215                 220

Asp Val Ser Lys Phe Leu Ile Phe Thr Pro Leu Ile Lys Asn Pro Asp
225                 230                 235                 240

Asn Ser Ala Gly Pro Ile Phe His Gly Arg Leu Gly Ala Glu Tyr Phe
                245                 250                 255

Ile Gly Val Lys Ala Ile Arg Ile Asn Glu Lys Leu Ile Ser Leu Asn
            260                 265                 270

Thr Ser Leu Leu Ser Ile Gly Asp Glu Gly Glu Gly Gly Thr Lys Ile
        275                 280                 285

Ser Thr Leu Asn Pro Tyr Thr Ile Met Glu Thr Ser Ile Tyr Asn Ser
    290                 295                 300

Phe Val Asp Ala Phe Ala Lys Glu Leu Glu Asp Val Pro Lys Ala Lys
305                 310                 315                 320

Ala Ile Lys Pro Phe Lys Leu Cys Phe Lys Leu Lys Asp Leu Gly Val
                325                 330                 335

Thr Arg Val Gly Leu Ala Val Pro Thr Ile Asp Phe Met Leu Gln Asn
            340                 345                 350

Lys Asp Val Phe Trp Arg Met Phe Gly Ala Asn Ser Met Val Gln Val
        355                 360                 365

Asn Asn Lys Val Ser Cys Leu Ala Phe Leu Asp Gly Gly Val Glu Val
    370                 375                 380

Thr Thr Ser Ile Val Ile Gly Gly Tyr Gln Leu Glu Asp Asn Phe Leu
385                 390                 395                 400

Gln Phe Asp Leu Val Asn Ser Arg Leu Gly Phe Ser Ser Ser Leu Leu
                405                 410                 415
```

```
Phe Leu Gln Thr Thr Cys Ala Asn Phe Asn Phe Thr Ser Ser Ile
            420                 425                 430


<210>  61
<211>  448
<212>  PRT
<213>  NONE

<400>  61

Met Thr Thr Ser Tyr Leu Phe Leu Phe Ser Leu Ser Leu Ile Ser Leu
1               5                   10                  15


Ile Ser Ser Ser Val Ala Leu Thr Lys Pro His Thr Pro Lys Pro Asn
            20                  25                  30


Asn Ala Phe Ile Leu Pro Ile Ala Lys Asp Pro Ile Thr Leu Gln Tyr
        35                  40                  45


Ser Thr Thr Ile Asn Met Gly Thr Pro Ala Val Thr Leu Asp Leu Val
    50                  55                  60


Val Asp Ile Arg Glu Arg Phe Leu Trp Phe Glu Cys Asp Glu Ser Tyr
65                  70                  75                  80


Asn Ser Ser Thr Tyr His Pro Ile Gln Cys Gly Thr Lys Lys Cys Lys
                85                  90                  95


Gln Ser Lys Gly Ala Glu Cys Ile Asp Cys Ile Asn His Pro Leu Lys
            100                 105                 110


Thr Gly Cys Thr Asn His Thr Cys Gly Val Gln Pro Phe Asn Pro Phe
        115                 120                 125


Gly Glu Phe Tyr Val Ser Gly Asp Ile Gly Glu Asp Ile Ile Ser Ser
    130                 135                 140


Leu Arg Gly Thr Leu Ser Asn Val Asn Val Pro Arg Val Ile Ser Ser
145                 150                 155                 160


Cys Ile Tyr Pro Asn Lys Phe Gly Val Gln Gly Phe Leu Gln Gly Leu
                165                 170                 175


Ala Lys Gly Lys Lys Gly Ile Leu Gly Leu Ala Arg Thr Phe Ile Ser
            180                 185                 190


Leu Pro Thr Gln Leu Ala Thr Arg Tyr Lys Leu Asp Arg Lys Phe Thr
        195                 200                 205
```

Leu Cys Leu Pro Ser Thr Ser Asn Ser Asp Gly Leu Gly Leu Gly Ser
    210                 215                 220

Leu Phe Ile Gly Gly Gly Pro Tyr His Leu Pro Ser Gln Lys Ile Asp
225                 230                 235                 240

Ala Ser Lys Phe Leu Glu Tyr Thr Pro Leu Ile Thr Asn Arg His Ser
                245                 250                 255

Thr Gly Pro Ile Phe Asp Asn Leu Pro Ser Thr Glu Tyr Phe Ile Lys
            260                 265                 270

Val Lys Ser Ile Lys Val Asp Asn Asn Ile Ile Asn Phe Asn Asn Ser
        275                 280                 285

Leu Leu Ser Ile Arg Lys Lys Leu Gly Ile Gly Gly Thr Lys Leu Ser
    290                 295                 300

Thr Val Ile Pro His Thr Lys Leu His Thr Leu Ile Tyr Lys Ser Leu
305                 310                 315                 320

Leu Asn Ala Phe Val Lys Lys Ala Glu Ile Arg Lys Ile Lys Arg Val
                325                 330                 335

Lys Glu Val Ala Pro Phe Gly Ala Cys Phe Asp Ser Arg Thr Ile Gly
            340                 345                 350

Lys Ser Val Thr Gly Pro Asn Val Pro Thr Ile Asp Phe Val Leu Lys
        355                 360                 365

Gly Gly Val Glu Trp Arg Phe Tyr Gly Ala Asn Ser Met Val Lys Val
    370                 375                 380

Gly Arg Asn Val Leu Cys Leu Gly Phe Val Asp Gly Gly Asn Glu Glu
385                 390                 395                 400

Val Gly Pro Trp Asp Thr Ser Ile Val Ile Gly Gly His Gln Leu Glu
                405                 410                 415

Asp Asn Leu Leu Glu Phe Asp Leu Val Ser Ser Lys Leu Gly Phe Ser
            420                 425                 430

Ser Ser Leu Leu Leu Asn Lys Ala Ser Cys Ser His Phe Lys Gly Phe
        435                 440                 445

<210>  62

<211> 429
<212> PRT
<213> NONE

<400> 62

Met Ser Ser Phe Ser Ser Ile Ile His Phe Phe Leu Leu Ser Leu Pro
1 5 10 15

Leu Leu Ser Ile Ser Cys Leu Ser Leu Ser Gln Pro Pro Asn Thr Lys
20 25 30

Pro Asn Pro Phe Ile Leu Pro Ile Lys Lys Asp Pro Ser Thr Asn Leu
35 40 45

Phe Tyr Thr Ser Val Gly Ile Gly Thr Pro Arg Lys Asp Phe Asn Leu
50 55 60

Ala Ile Asp Leu Ala Gly Glu Asn Leu Trp Tyr Asp Cys Asp Thr His
65 70 75 80

Tyr Asn Ser Ser Ser Tyr Ile Pro Ile Glu Cys Gly Ser Lys Lys Cys
85 90 95

Pro Asp Val Ala Cys Ile Gly Cys Asn Gly Pro Phe Lys Pro Gly Cys
100 105 110

Thr Asn Asn Thr Cys Ala Ala Asn Thr Ile Asn Thr Leu Ala Asn Phe
115 120 125

Ile Phe Gly Gly Gly Leu Gly Gln Asp Phe Ile Phe Ile Ser Gln Gln
130 135 140

Lys Val Ser Gly Leu Leu Ser Ser Cys Ile Asp Thr Asp Gly Phe Pro
145 150 155 160

Ser Phe Thr Gly Asn Asp Ser Pro Leu Asn Gly Leu Pro Lys Ile Thr
165 170 175

Lys Gly Ile Ile Gly Leu Ala Arg Ser Asn Leu Ser Leu Pro Thr Gln
180 185 190

Leu Ala Leu Lys Asn Glu Leu Pro Pro Lys Phe Ser Leu Cys Leu Pro
195 200 205

Ser Ser Asn Lys Gln Gly Phe Thr Asn Leu Leu Val Gly Ser Ile Gly
210 215 220

Lys Asp Pro Phe Gln Glu Leu Tyr Lys Phe Val Gln Thr Thr Pro Leu

225 230 235 240

Ile Val Asn Pro Val Ser Thr Gly Ala Val Ser Val Gln Gly Val Pro
245 250 255

Ser Ile Glu Tyr Phe Ile Asp Val Lys Ala Ile Lys Ile Asp Gly Lys
260 265 270

Val Val Asn Leu Lys Pro Ser Leu Leu Ser Ile Asp Asn Lys Gly Asn
275 280 285

Gly Gly Thr Lys Ile Ser Thr Met Ser Pro Phe Thr Glu Leu Gln Arg
290 295 300

Ser Val Tyr Lys Pro Phe Ile Arg Asp Phe Leu Lys Lys Ala Ser Asp
305 310 315 320

Arg Lys Leu Lys Lys Val Glu Ser Val Ala Pro Phe Glu Ala Cys Phe
325 330 335

Glu Ser Thr Asn Ile Glu Asn Ser Leu Pro Arg Ile Asp Leu Val Leu
340 345 350

Gln Gly Gly Val Gln Trp Ser Ile Tyr Gly Asn Asn Leu Met Val Asn
355 360 365

Val Lys Lys Asn Val Ala Cys Leu Gly Phe Val Asp Gly Gly Thr Glu
370 375 380

Pro Arg Met Ser Phe Ala Lys Ala Ser Ile Val Ile Gly Gly His Gln
385 390 395 400

Leu Glu Asp Asn Leu Leu Val Phe Asp Leu Asn Ser Ser Lys Leu Ser
405 410 415

Phe Ser Ser Ser Leu Leu Val His Asn Ala Ser Cys Ser
420 425

<210> 63
<211> 443
<212> PRT
<213> NONE

<400> 63

Met Leu Val Glu Met Val Val Ser Thr Lys Leu Leu Thr Leu Leu Ser
1 5 10 15

Ile Ile Ser Leu Ile Ile Ser Thr Ser Thr Ala Gln Thr Ser Leu Arg

20 25 30

Pro Lys Ala Leu Val Leu Pro Ile Thr Lys Asp Ile Ser Ser Thr Thr
35 40 45

Pro Gln Tyr Val Thr Gln Ile Lys Gln Arg Thr Pro Leu Val Pro Ile
50 55 60

Lys Leu Thr Ile Asp Leu Gly Gly Gly Tyr Leu Trp Val Asn Cys Glu
65 70 75 80

Lys Gly Asn Tyr Val Ser Ser Thr Ser Lys Pro Thr Leu Cys Thr Ser
85 90 95

Ser Gln Cys Ser Leu Phe Ala Ser His Gly Cys Asn Ser Glu Asn Ile
100 105 110

Cys Ala Arg Ser Pro Thr Asn Thr Ile Thr Gly Val Ser Ser Tyr Gly
115 120 125

Asp Ile His Ser Asp Ile Val Ser Val Gln Ser Thr Asp Gly Asn Asn
130 135 140

Pro Gly Arg Val Val Ser Val Pro Asn Phe Leu Phe Ile Cys Gly Ser
145 150 155 160

Asn Val Val Gln Lys Asp Leu Ala Lys Gly Val Lys Gly Met Ala Gly
165 170 175

Leu Gly Arg Thr Lys Val Ser Ile Phe Ser Gln Phe Ser Ser Ala Phe
180 185 190

Ser Phe Asn Arg Lys Phe Ala Ile Cys Leu Gly Ser Ser Thr Arg Ser
195 200 205

Lys Gly Val Leu Phe Phe Gly Asp Gly Pro Tyr Asn Leu Asn Met Asp
210 215 220

Val Ser Lys Thr Leu Thr Phe Thr Pro Leu Val Thr Asn Pro Phe Ser
225 230 235 240

Thr Ser Pro Ser Ser Phe Leu Gly Glu Pro Ser Tyr Glu Tyr Phe Ile
245 250 255

Asp Val Lys Ser Ile Arg Val Ser Gly Lys Asn Val Pro Leu Asn Lys
260 265 270

```
Thr Leu Leu Lys Ile Asp Thr Lys Asn Gly Val Gly Gly Thr Lys Ile
        275                 280                 285

Ser Thr Val Asn Pro Tyr Thr Ile Met Glu Thr Ser Ile Tyr Lys Ala
    290                 295                 300

Val Val Asp Ala Phe Val Lys Glu Leu Gly Ala Pro Thr Val Glu Pro
305                 310                 315                 320

Val Ala Pro Phe Gly Thr Cys Phe Ala Thr Lys Asp Ile Ser Phe Ser
                325                 330                 335

Arg Met Gly Pro Gly Val Pro Ser Ile Asp Phe Val Leu Gln Asn Glu
            340                 345                 350

Asp Val Leu Trp Ser Ile Ile Gly Ala Asn Ser Met Val Ser Leu Glu
        355                 360                 365

Asn Asp Val Ile Cys Leu Gly Phe Val Asp Ala Gly Ser Asn Pro Lys
    370                 375                 380

Ala Ser Gln Val Gly Phe Val Val Gly Gly Ser Lys Pro Met Thr Ser
385                 390                 395                 400

Ile Thr Ile Gly Ala His Gln Leu Glu Asn Asn Phe Leu Gln Phe Asp
                405                 410                 415

Leu Ala Ala Ser Arg Leu Gly Phe Arg Ser Leu Phe Leu Glu His Thr
            420                 425                 430

Asn Cys Asp Asn Phe Asn Phe Thr Ser Ser Val
        435                 440


<210>  64
<211>  411
<212>  PRT
<213>  NONE

<400>  64

Met Ala Ser Phe Ser Ile Phe Leu Pro Leu Phe Phe Ser Leu Leu Ser
1               5                   10                  15

Leu Val Ser Ser Ser Pro Asn Ser Leu Leu Leu Pro Ile Thr Lys Asp
            20                  25                  30

Thr Thr Thr Leu Gln Tyr Leu Thr Thr Leu Ser Tyr Gly Thr Pro Phe
        35                  40                  45
```

```
Leu Pro Thr Lys Leu Val Val Asp Leu Thr Gly Ser Leu Phe Trp Leu
    50                  55                  60

His Cys Ser Ser Arg Lys Thr Pro Ser Phe Ser Leu Arg Thr Ile Pro
65                  70                  75                  80

His Arg Ser Leu Gln Cys Leu Thr Ala Lys Thr Asn Gln Thr Phe Glu
                85                  90                  95

Phe Pro Ile Asp Gln Asn His Pro Cys Gln Ile Leu Thr Glu Asn Thr
            100                 105                 110

Ile Thr Gly Lys Val Ala Thr Asp Gly Lys Leu Val Glu Asp Val Val
        115                 120                 125

Ala Phe Lys Ser Thr Lys Asp Ser Ile Phe Glu His Ala His Asp Phe
    130                 135                 140

Leu Phe Thr Cys Ser His Thr Leu Leu Leu Asn Gly Leu Ser Ser Asp
145                 150                 155                 160

Ala Lys Gly Ile Val Gly Phe Gly Arg Ser Arg Thr Ser Phe Ser Ser
                165                 170                 175

Gln Val Phe Asn Ser Ile Asn Ser Gln Arg Lys Ile Thr Phe Cys Leu
            180                 185                 190

Ser Ser Ser Ser Gly Phe Val Gln Leu Gly Ser Thr Ser Thr Val Phe
        195                 200                 205

Glu Ser Ser Thr Lys Ser Glu Ile Phe Arg Ser Leu Thr Phe Thr Pro
    210                 215                 220

Leu Val Glu Ser Gln Asn His Glu Ser Phe Ile Asn Val Lys Ser Ile
225                 230                 235                 240

Lys Ile Gly Gly Lys Lys Val Ser Phe Asn Thr Pro Ser Leu Ser Gln
                245                 250                 255

Glu Gly Asn Ser Gly Thr Lys Leu Ser Ser Ile Val Pro Tyr Thr Thr
            260                 265                 270

Ile Gln Ser Ser Ile Tyr Gly Asn Phe Lys Ser Ala Phe Leu Lys Ala
        275                 280                 285

Ala Phe Ser Met Asn Met Thr Arg Val Thr Ser Val Ala Pro Phe Asp
    290                 295                 300
```

Val Cys Phe Asp Ser Asn Gly Ile Asp Glu Ser Lys Val Gly Pro Asn
305 310 315 320

Val Pro Val Ile Asp Phe Val Leu Gln Ser Glu Met Val Lys Trp Ser
325 330 335

Ile Tyr Gly Arg Asn Ser Met Val Lys Leu Ser Asp Asp Val Met Cys
340 345 350

Leu Gly Leu Leu Asp Gly Gly Val Glu Gln Lys Asn Pro Ile Val Ile
355 360 365

Gly Gly Tyr Gln Phe Glu Asp Val Leu Val Gln Phe Asp Phe Asp Ser
370 375 380

Ser Met Val Gly Phe Ser Ser Ser Leu Leu Met Arg Asp Ser Ser Cys
385 390 395 400

Ser Asn Phe Arg Phe Gly Ser Ile Ser Ser Gln
405 410

<210> 65
<211> 474
<212> PRT
<213> NONE

<400> 65

Met Thr Lys Asn Ile Thr Thr Leu Leu Leu Leu Leu Leu Val Gly Val
1 5 10 15

Thr Ile Thr Leu Cys Glu Asp Glu Glu Arg Glu Lys Gly Ser Ser Ser
20 25 30

Ser Asn Lys Leu Phe Leu Met Gln Asp Ser Lys Ser Val Val Lys Thr
35 40 45

Asp Ala Gly Glu Met Arg Leu Phe Gln Asn Lys Asp Thr Glu Phe Leu
50 55 60

Asp Arg Asn Met His Ile Gly Leu Ile Asn Met Glu Pro Arg Ser Leu
65 70 75 80

Phe Ile Pro Gln Tyr Leu Asp Ser Asn Phe Ile Ile Phe Val Arg Arg
85 90 95

Gly Asp Ala Lys Leu Gly Phe Ile Tyr Gly Asp Glu Leu Glu Glu Arg
100 105 110

```
Arg Leu Lys Thr Gly Asp Ile Tyr Val Ile Pro Ala Gly Ser Val Phe
        115                 120                 125

Tyr Leu Val Asn Ile Gly Glu Gly Gln Arg Leu His Leu Ile Cys Ser
    130                 135                 140

Ile Asp Pro Ser Thr Ser Leu Gly Asp Thr Phe Gln Pro Phe Tyr Ile
145                 150                 155                 160

Gly Gly Gly Asp Asn Ser Gln Ser Val Leu Ala Gly Phe Glu Pro Val
                165                 170                 175

Ile Leu Glu Thr Ala Phe Asn Glu Ser Arg Glu Lys Leu Glu Arg Ile
            180                 185                 190

Phe Ser Lys Lys Val Asp Gly Pro Ile Val Tyr Ile Asp Asp Ser His
        195                 200                 205

Ala Pro Ser Leu Trp Thr Lys Phe Leu Gln Leu Lys Lys Glu Glu Lys
    210                 215                 220

Val Gln His Leu Asn Lys Met Val Gln Gly Gln Glu Glu Asn Asn Glu
225                 230                 235                 240

Glu Lys Lys Gln Thr Ser Trp Ser Trp Arg Lys Leu Phe Gln Ser Val
                245                 250                 255

Leu Gly Glu Glu Asn Lys Asn Ile Glu Asn Lys Asp Arg Gly Asp Ser
            260                 265                 270

Pro Asp Ser Tyr Asn Leu Tyr Asp Arg Lys His Asp Phe Lys Asn Gly
        275                 280                 285

Tyr Gly Trp Ser Ser Ala Leu Asp Gly Asp Asp Tyr Ser Leu Leu Lys
    290                 295                 300

Ile Pro Asp Ile Gly Val Phe His Val Asn Leu Thr Ala Gly Ser Met
305                 310                 315                 320

Met Val Pro His Val Asn Pro Arg Ala Thr Glu Tyr Gly Ile Val Leu
                325                 330                 335

Arg Gly Ser Gly Arg Ile Gln Ile Leu Phe Pro Asn Gly Ser Asn Ala
            340                 345                 350

Met Asp Thr Glu Ile Lys Val Gly Asp Ile Phe Tyr Val Pro Arg Tyr
        355                 360                 365
```

Phe Pro Phe Cys Gln Ile Ala Ser Arg Asn Gly Ala Leu Glu Phe Phe
370 375 380

Gly Phe Thr Thr Ser Ser Lys Lys Ser Asn Pro Gln Phe Leu Val Gly
385 390 395 400

Ala Ala Ser Leu Leu Lys Thr Ile Met Gly Pro Glu Leu Ala Ala Gly
405 410 415

Phe Gly Val Arg Glu Asp Ile Met Arg Asp Val Val Gly Ala Gln His
420 425 430

Asp Ala Val Ile Val Ser Ser Thr Trp Ala Ala Pro Gly Thr Gly Gly
435 440 445

Val Arg Glu Glu Asp Ala Tyr Val Gln Ser Lys Gly Ile Ile Gly Ile
450 455 460

Phe Asp Asp Ile Leu Met Asp Asp Phe Glu
465 470

<210> 66
<211> 593
<212> PRT
<213> NONE

<400> 66

Met Ala Thr Thr Thr Lys Thr Arg Phe Pro Leu Leu Leu Leu Leu Gly
1 5 10 15

Ile Ile Phe Leu Ala Ser Val Cys Val Ser Tyr Gly Ile Val Gln Tyr
20 25 30

Glu Gln Gly Asn Pro Cys Leu Lys Lys Cys Met Gln Arg Tyr Asn Lys
35 40 45

Val Asn Glu Ala Phe Lys Arg Arg Glu Glu Glu Glu Tyr Glu Glu Arg
50 55 60

Ser Glu His Ser Val Pro Gly Gln Arg Glu Arg Gly Arg Gln Glu Gly
65 70 75 80

Glu Lys Glu Glu Glu His Arg Glu Gln Arg Arg Ser Arg Arg Glu Ser
85 90 95

Glu Arg Glu Arg Glu Glu Asp Glu Gln Lys Gln Arg His Pro His His
100 105 110

```
Glu Arg Glu Glu Glu Glu Asp Glu Glu Phe Arg Glu Arg Arg His Arg
        115                 120                 125

Gly Asp Thr Glu Glu Thr Arg Phe Arg Arg Arg Lys Glu Arg Arg Gln
    130                 135                 140

Glu Gln Gln Glu Lys Lys Ser Glu Ser Lys Glu Asp Glu Glu Glu Ser
145                 150                 155                 160

Ser Ser Glu Ser Gln Gly Arg Arg Asn Pro Phe Phe Phe Arg Ser Asn
                165                 170                 175

Lys Phe Gln Thr Ile Phe Glu Asn Gln His Gly His Ile Arg Leu Leu
            180                 185                 190

Gln Asn Phe Asp Lys Arg Ser Asn Leu Phe Gln Asn Leu Gln Asn Tyr
        195                 200                 205

Arg Phe Leu Glu Tyr Lys Ala Arg Pro His Thr Leu Leu Leu Pro Gln
    210                 215                 220

His Ile Asp Ala Asp Phe Ile Leu Val Val Leu Ser Gly Lys Ala Ile
225                 230                 235                 240

Leu Thr Val Leu Asn Pro Asn Asp Arg Asn Ser Phe Asn Leu Glu Arg
                245                 250                 255

Gly Asp Thr Ile Lys Leu Pro Ala Gly Thr Thr Ala Tyr Leu Ala Asn
            260                 265                 270

His Asp Asp Glu Glu Asp Leu Arg Val Val Glu Leu Ala Ile Pro Val
        275                 280                 285

Asn Arg Pro Gly Lys Phe Gln Ser Phe Phe Pro Ser Ser Asn Gln Asn
    290                 295                 300

Gln Gln Ser Tyr Phe Asn Gly Phe Ser Lys Ser Ile Leu Glu Ala Ser
305                 310                 315                 320

Phe Asn Thr Lys Tyr Glu Thr Ile Glu Arg Val Leu Leu Glu Glu Gln
                325                 330                 335

Glu Pro Glu Gln Ser Arg Gly Arg Arg Gly Ser Glu Glu Ser Glu Glu
            340                 345                 350

Gly Asp Ala Ile Val Lys Val Ser Arg Glu Gln Ile Glu Glu Leu Ser
```

```
        355                 360                 365

Lys His Ala Lys Ser Ser Ser Arg Lys Ser Ile Ser Ser Glu Ser Glu
    370                 375                 380

Pro Phe Asn Leu Arg Ser Arg Asn Pro Ile Tyr Ser Asn Lys Phe Gly
385                 390                 395                 400

Lys Phe Phe Glu Ile Thr Pro Glu Lys Ser Pro Gln Leu Gln Asp Leu
                405                 410                 415

Asn Ile Phe Val Ser Cys Val Glu Ile Asn Glu Gly Gly Leu Met Leu
            420                 425                 430

Pro His Phe Asn Ser Arg Ala Ile Val Val Leu Phe Ile Asn Glu Gly
        435                 440                 445

Lys Gly His Leu Glu Leu Val Gly Leu Arg Asn Glu Gln Gln Glu Gln
    450                 455                 460

Gln Gln Glu Glu Asp Glu Glu Gln Glu Glu Glu Glu Arg Asn Asn Gln
465                 470                 475                 480

Val Gln Arg Phe Arg Ala Arg Leu Ser Pro Gly Asp Val Phe Ile Ile
                485                 490                 495

Pro Ala Gly His Pro Val Ala Val Asn Ala Ser Ser Asp Leu Asn Phe
            500                 505                 510

Val Gly Phe Gly Ile Asn Ala Glu Asn Asn Gln Arg Asn Phe Leu Ala
        515                 520                 525

Gly Glu Asp Asp Asn Val Ile Ser Gln Ile Gln Asn Pro Val Lys Glu
    530                 535                 540

Leu Thr Phe Pro Gly Ser Ala Gln Glu Val Asn Arg Leu Ile Lys Asn
545                 550                 555                 560

Gln Arg Gln Ser Tyr Phe Ala Asn Ala Gln Pro Gln Gln Arg Glu Glu
                565                 570                 575

Glu Ser Gln Arg Arg Arg Gly Ser Leu Ser Ser Ile Leu Gly Gly Phe
            580                 585                 590

Tyr
```

<210> 67
<211> 446
<212> PRT
<213> NONE

<400> 67

```
Met Ala Ile Lys Ala Arg Phe Pro Leu Leu Val Leu Leu Gly Ile Val
1               5                   10                  15

Phe Leu Ala Ser Val Cys Ala Lys Ser Asp Lys Glu Asn Pro Phe Phe
            20                  25                  30

Phe Lys Ser Asn Asn Cys Gln Thr Leu Phe Glu Asn Glu Asn Gly His
        35                  40                  45

Val Arg Leu Leu Gln Arg Phe Asp Lys Arg Ser Gln Leu Phe Glu Asn
    50                  55                  60

Leu Gln Asn Tyr Arg Leu Met Glu Tyr Asn Ser Lys Pro His Thr Leu
65                  70                  75                  80

Phe Leu Pro Gln His Asn Asp Ala Asp Phe Ile Leu Val Val Leu Arg
                85                  90                  95

Gly Arg Ala Ile Leu Thr Val Leu Asn Pro Asn Asp Arg Asn Thr Phe
            100                 105                 110

Lys Leu Glu Arg Gly Asp Thr Ile Lys Leu Pro Ala Gly Thr Ile Ala
        115                 120                 125

Tyr Leu Ala Asn Arg Asp Asp Asn Glu Asp Leu Arg Val Leu Asp Leu
    130                 135                 140

Ala Ile Pro Val Asn Arg Pro Gly Gln Phe Gln Ser Phe Ser Leu Ser
145                 150                 155                 160

Gly Asn Glu Asn Gln Gln Ser Tyr Phe Gln Gly Phe Ser Lys Lys Ile
                165                 170                 175

Leu Glu Ala Ser Phe Asn Ser Asp Tyr Glu Glu Ile Glu Arg Val Leu
            180                 185                 190

Leu Glu Glu Gln Glu Gln Lys Pro Glu Gln Arg Arg Gly His Lys Gly
        195                 200                 205

Arg Gln Gln Ser Gln Glu Thr Asp Val Ile Val Lys Ile Ser Arg Glu
    210                 215                 220
```

Gln Ile Glu Glu Leu Ser Lys Asn Ala Lys Ser Asn Cys Lys Lys Ser
225 230 235 240

Val Ser Ser Glu Ser Glu Pro Phe Asn Leu Arg Ser Arg Ser Pro Ile
245 250 255

Tyr Ser Asn Arg Phe Gly Asn Phe Phe Glu Ile Thr Pro Glu Lys Asn
260 265 270

Pro Gln Leu Lys Asp Leu Asp Ile Phe Val Asn Ser Val Glu Ile Lys
275 280 285

Glu Gly Ser Leu Leu Leu Pro His Phe Asn Ser Arg Ala Thr Val Ile
290 295 300

Leu Val Val Asn Glu Gly Lys Gly Glu Val Glu Leu Val Gly Leu Arg
305 310 315 320

Asn Glu Asn Glu Gln Glu Asn Lys Lys Glu Asp Glu Glu Glu Glu Glu
325 330 335

Asp Arg Asn Val Gln Val Gln Arg Phe Gln Ser Lys Leu Ser Ser Gly
340 345 350

Asp Val Val Val Ile Pro Ala Ser His Pro Phe Ser Ile Asn Ala Ser
355 360 365

Ser Asp Leu Phe Leu Leu Gly Phe Gly Ile Asn Ala Gln Asn Asn Gln
370 375 380

Arg Asn Phe Leu Ala Gly Glu Glu Asp Asn Val Ile Ser Gln Ile Gln
385 390 395 400

Arg Pro Val Lys Glu Val Ala Phe Pro Gly Ser Ala Glu Glu Val Asp
405 410 415

Arg Leu Leu Lys Asn Gln Arg Gln Ser His Phe Ala Asn Ala Gln Pro
420 425 430

Gln Gln Lys Arg Lys Gly Ser Gln Arg Ile Arg Ser Pro Phe
435 440 445

<210> 68
<211> 455
<212> PRT
<213> NONE

<400> 68

```
Met Ala Ile Lys Ala Arg Phe Pro Leu Leu Val Leu Leu Gly Ile Val
1               5                   10                  15

Phe Leu Ala Ser Val Cys Ala Lys Ser Asp Lys Glu Asn Pro Phe Phe
            20                  25                  30

Phe Lys Ser Asn Asn Phe Gln Thr Leu Phe Lys Asn Glu Asn Gly His
        35                  40                  45

Val Arg Leu Leu Gln Arg Phe Asp Lys Arg Ser Gln Leu Phe Glu Asn
    50                  55                  60

Leu Gln Asn Tyr Arg Leu Val Glu Tyr Asn Ser Lys Pro His Thr Leu
65                  70                  75                  80

Phe Leu Pro Gln His Asn Asp Ala Asp Phe Ile Leu Val Val Leu Ser
                85                  90                  95

Gly Arg Ala Ile Leu Thr Val Leu Asn Pro Asn Asp Arg Asn Thr Phe
            100                 105                 110

Lys Leu Glu Arg Gly Asp Thr Ile Lys Leu Pro Ala Gly Thr Ile Ala
        115                 120                 125

Tyr Leu Ala Asn Arg Asp Asp Asn Glu Asp Leu Arg Val Leu Asp Leu
    130                 135                 140

Ala Ile Pro Val Asn Arg Pro Gly Gln Phe Gln Ser Phe Ser Leu Ser
145                 150                 155                 160

Gly Ser Glu Asn Gln Gln Ser Tyr Phe Gln Gly Phe Ser Lys Lys Ile
                165                 170                 175

Leu Glu Ala Ser Phe Asn Ser Asp Tyr Glu Glu Ile Glu Arg Val Leu
            180                 185                 190

Leu Glu Glu Gln Glu Gln Lys Pro Lys Gln Arg Arg Gly His Lys Asp
        195                 200                 205

Arg Gln Gln Ser Gln Arg Gln Glu Ala Asp Val Ile Val Lys Ile Ser
    210                 215                 220

Arg Glu Gln Ile Glu Glu Leu Ser Lys Asn Ala Lys Ser Ser Ser Lys
225                 230                 235                 240

Arg Ser Val Ser Ser Glu Ser Glu Pro Phe Asn Leu Arg Ser Arg Asn
                245                 250                 255
```

Pro Ile Tyr Ser Asn Lys Tyr Gly Asn Phe Phe Glu Ile Thr Pro Glu
260 265 270

Lys Asn Pro Gln Leu Gln Asp Leu Asp Ile Ser Leu Asn Ser Val Glu
275 280 285

Ile Asn Glu Gly Ser Leu Leu Leu Pro His Phe Asn Ser Arg Ala Thr
290 295 300

Val Ile Leu Val Val Asn Glu Gly Lys Gly Glu Val Glu Leu Val Gly
305 310 315 320

Leu Arg Asn Glu Asn Glu Gln Glu Asn Lys Lys Glu Asp Glu Glu Glu
325 330 335

Glu Glu Asp Arg Asn Val Gln Val Gln Arg Phe Gln Ser Lys Leu Ser
340 345 350

Ser Gly Asp Val Val Val Ile Pro Ala Ser His Pro Phe Ser Ile Asn
355 360 365

Ala Ser Ser Asp Leu Phe Leu Leu Gly Phe Gly Ile Asn Ala Gln Asn
370 375 380

Asn Gln Arg Asn Phe Leu Ala Gly Glu Glu Asp Asn Val Ile Ser Gln
385 390 395 400

Ile Gln Arg Pro Val Lys Glu Val Ala Phe Pro Gly Ser Ala Glu Glu
405 410 415

Val Asp Arg Leu Leu Lys Asn Gln Arg Gln Ser His Phe Ala Asn Ala
420 425 430

Gln Pro Gln Gln Lys Asp Glu Gly Ser Gln Lys Ile Arg Ile Pro Leu
435 440 445

Ser Ser Ile Leu Gly Gly Phe
450 455

<210> 69
<211> 488
<212> PRT
<213> NONE

<400> 69

Met Arg Asp Lys Ala Thr Leu Leu Ile Met Leu Leu Ile Leu Cys His
1 5 10 15

```
Gly Val Ala Met Thr Lys Gly Leu Trp Glu Ile Lys Asn Glu Asp Arg
            20                  25                  30

Glu Asp His Gly Pro Gln Met Pro Glu Lys Leu Phe Leu Leu Gln Asn
        35                  40                  45

Ser Lys Arg Val Val Lys Thr Asp Ala Gly Glu Met Arg Val Leu Glu
    50                  55                  60

Ser Tyr Gly Gly Arg Val Ser Glu Arg Arg Leu His Ile Gly Phe Ile
65                  70                  75                  80

Asn Met Glu Pro Ser Ser Leu Phe Val Pro Gln Tyr Leu Asp Ser Thr
                85                  90                  95

Leu Ile Val Phe Val Arg Ser Gly Glu Ala Lys Val Gly Phe Ile Tyr
            100                 105                 110

Glu Asp Glu Leu Ala Glu Arg Glu Leu Lys Arg Gly Asp Val Tyr Gln
        115                 120                 125

Ile Pro Ala Gly Ser Ala Phe Tyr Leu Leu Asn Ile Gly Lys Ala Gln
    130                 135                 140

Lys Leu His Ile Ile Cys Ser Ile Asp Pro Ser Glu Ser Leu Gly Ile
145                 150                 155                 160

Gly Ile Phe Gln Ser Phe Tyr Ile Gly Gly Gly Ala Pro Val Ser Val
                165                 170                 175

Leu Ser Gly Phe Glu Pro Gln Ile Leu Glu Thr Ala Phe Asn Ala Ser
            180                 185                 190

Glu Ala Glu Leu Ile Lys Phe Phe Thr Arg Gln His Glu Gly Pro Ile
        195                 200                 205

Val His Ile Gly Asp Ser His Val Ser Ala Ser Ser Leu Trp Asn Lys
    210                 215                 220

Phe Leu Gln Leu Lys Glu Asp Glu Lys Leu Asn His Leu Lys Lys Leu
225                 230                 235                 240

Val Gln Asp Gln Glu Glu Asp Glu Asp Asp Glu Glu Lys Glu Glu Glu
                245                 250                 255

Lys Pro Gln Pro Ser Trp Ser Trp Arg Lys Leu Leu Glu Ser Val Phe
            260                 265                 270
```

Gly Asp Glu Met Lys Lys Asn Lys Lys Asp Lys Val Ser His Lys Ser
275 280 285

Pro His Ser Cys Asn Leu Tyr Asp Arg Lys Pro Asp Phe Lys Asn Thr
290 295 300

Tyr Gly Trp Ser Val Ala Leu Asp Gly Ser Asp Tyr Ser Pro Leu Lys
305 310 315 320

Thr Ser Gly Ile Gly Ile Tyr His Val Asn Leu Lys Pro Gly Ser Met
325 330 335

Met Thr Pro His Val Asn Pro Arg Ala Thr Glu Tyr Gly Met Val Val
340 345 350

Lys Gly Ser Gly Arg Ile Gln Ile Val Phe Pro Asn Gly Ser Asn Ala
355 360 365

Met Asp Thr His Ile Lys Glu Gly Asp Val Phe Phe Val Pro Arg Tyr
370 375 380

Phe Ala Phe Cys Gln Ile Ala Ser Lys Asn Glu Pro Leu Asp Phe Phe
385 390 395 400

Gly Phe Thr Thr Ser Ser Arg Lys Asn Lys Pro Gln Phe Leu Val Gly
405 410 415

Ala Thr Ser Leu Met Lys Thr Met Met Gly Pro Glu Leu Ala Ala Ala
420 425 430

Phe Gly Val Ser Glu Asp Thr Met Arg His Ile Leu Asn Ala Gln His
435 440 445

Glu Ala Val Ile Leu Pro Thr Pro Trp Thr Glu Ser Lys Gln Asn Tyr
450 455 460

Gly Asn Lys Leu Asp Leu Glu Ser Leu Pro Thr Thr Lys Val Val Thr
465 470 475 480

Asn Asp Asp Met Ile Ile Gly Phe
485

<210> 70
<211> 458
<212> PRT
<213> NONE

<400>  70

Met Ala Ile Lys Ala Arg Phe Pro Leu Leu Val Leu Leu Gly Ile Val
1               5                   10                  15

Phe Leu Ala Ser Val Cys Ala Lys Ser Asn Lys Glu Asn Pro Phe Phe
            20                  25                  30

Phe Lys Ser Asn Asn Phe Gln Thr Leu Phe Lys Asn Glu Asn Gly His
        35                  40                  45

Val Arg Leu Leu Gln Arg Phe Asp Lys Arg Ser Gln Leu Phe Glu Asn
    50                  55                  60

Leu Gln Asn Tyr Arg Leu Val Glu Tyr Asn Ser Lys Pro His Thr Leu
65                  70                  75                  80

Phe Leu Pro Gln His Asn Asp Ala Asp Phe Ile Leu Val Val Leu Ser
                85                  90                  95

Gly Arg Ala Ile Leu Thr Val Leu Asn Pro Asn Asp Arg Asn Thr Phe
            100                 105                 110

Asn Leu Glu Arg Gly Asp Thr Ile Lys Leu Pro Ala Gly Thr Ile Ala
        115                 120                 125

Tyr Leu Ala Asn Arg Asp Asp Asn Glu Asp Leu Arg Val Leu Asp Leu
    130                 135                 140

Ala Ile Pro Val Asn Arg Pro Gly Gln Phe Gln Ser Phe Ser Leu Ser
145                 150                 155                 160

Gly Ser Glu Asn Gln Gln Ser Tyr Phe Gln Gly Phe Ser Lys Lys Ile
                165                 170                 175

Leu Glu Ala Ser Phe Asn Ser Asp Tyr Glu Glu Ile Glu Arg Val Leu
            180                 185                 190

Leu Glu Glu Gln Glu Gln Lys Pro Lys Gln Arg Arg Gly His Lys Asp
        195                 200                 205

Arg Gln Gln Ser Gln Ser Gln Ser Gln Gln Glu Ala Asp Val Ile Val
    210                 215                 220

Lys Ile Ser Arg Glu Gln Ile Glu Glu Leu Ser Lys Asn Ala Lys Ser
225                 230                 235                 240

Ser Ser Lys Lys Ser Val Ser Ser Glu Ser Glu Pro Phe Asn Leu Arg

245 250 255

Ser Arg Asn Pro Ile Tyr Ser Asn Lys Tyr Gly Asn Phe Phe Glu Ile
260 265 270

Thr Pro Glu Lys Asn Pro Gln Leu Gln Asp Leu Asp Ile Ser Leu Asn
275 280 285

Ser Val Glu Ile Asn Glu Gly Ser Leu Leu Leu Pro His Phe Asn Ser
290 295 300

Arg Ala Thr Val Ile Leu Val Val Asn Glu Gly Lys Gly Glu Val Glu
305 310 315 320

Leu Val Gly Leu Arg Asn Glu Asn Glu Gln Glu Asn Lys Lys Glu Asp
325 330 335

Glu Glu Glu Glu Glu Asp Arg Asn Val Gln Val Gln Arg Phe Gln Ser
340 345 350

Arg Leu Ser Ser Gly Asp Val Val Val Ile Pro Ala Ser His Pro Phe
355 360 365

Ser Ile Asn Ala Ser Ser Asp Leu Phe Leu Leu Gly Phe Gly Ile Asn
370 375 380

Ala Gln Asn Asn Gln Arg Asn Phe Leu Ala Gly Glu Glu Asp Asn Val
385 390 395 400

Ile Ser Gln Ile Gln Arg Pro Val Lys Glu Val Ala Phe Pro Gly Ser
405 410 415

Ala Glu Glu Val Asp Arg Leu Leu Lys Asn Gln Arg Gln Ser His Phe
420 425 430

Ala Asn Ala Gln Pro Gln Gln Lys Asp Glu Gly Ser Gln Lys Ile Arg
435 440 445

Ile Pro Leu Ser Ser Ile Leu Gly Gly Phe
450 455

<210> 71
<211> 138
<212> PRT
<213> NONE

<400> 71

Met Ala Tyr Val Lys Leu Pro Pro Leu Thr Leu Phe Leu Leu Ala Thr

```
1               5                   10                  15

Phe Leu Ile Thr Phe Ser Thr Lys Lys Val Gly Ala Thr Cys Thr Gly
            20                  25                  30

Val Cys Ser Phe Tyr Asp Ser Asn Pro Cys Gly Gly Asn Tyr Cys Phe
        35                  40                  45

Cys His Phe Leu Asp Val Gly Asp Tyr Lys Gly Ile Cys Ile Asp Arg
    50                  55                  60

Ser Phe Phe Ile Lys Thr Val Asp Glu Asp Pro Asn Leu Cys Gln Thr
65                  70                  75                  80

His Ala Glu Cys Thr Lys Lys Gly Ser Gly Asn Phe Cys Gly Arg Tyr
                85                  90                  95

Pro Asn Pro Asn Ile Lys Tyr Gly Arg Cys Phe Ala Ser Asn Thr Glu
            100                 105                 110

Ala Glu Glu Phe Ser Asn Lys Phe Ser Tyr Tyr Ser Ser Arg Phe Ile
        115                 120                 125

Lys Asp Phe Leu Asn Met Pro Val Val Ala
    130                 135


<210>  72
<211>  147
<212>  PRT
<213>  NONE

<400>  72

Met Ala Lys His Ile Phe Leu Val Thr Phe Phe Val Ala Leu Ile Leu
1               5                   10                  15

Phe Ile Ala His Thr Asn Ala Ser Lys Asp Glu Lys Glu Glu Ile Pro
            20                  25                  30

Glu Ser Cys His Lys Gln Leu Lys Ser Leu Asn Leu Lys His Cys Glu
        35                  40                  45

Lys Phe Leu Met Lys Arg Met Gln Lys Asp Glu Asp Glu Asp Asp Asp
    50                  55                  60

Asn Val Ile Lys Met Arg Gly Val Asn Tyr Ile Arg Asn Arg Glu Glu
65                  70                  75                  80

Gly Leu Lys Glu Asn Cys Cys Ala Gln Leu Ser Glu Val Asn Phe Leu
```

85 90 95

Asp Cys Arg Cys Glu Ala Leu Gln Lys Ile Gly Asp Asn Leu Ser Asp
100 105 110

Lys Cys Asp Lys Lys Glu Met Glu Glu Met Glu Arg Glu Leu Lys Ile
115 120 125

Leu Pro Leu Arg Cys Gly Ile Thr Pro Pro Leu Gly Cys Asp Leu Ser
130 135 140

Phe Asp Asn
145

<210> 73
<211> 149
<212> PRT
<213> NONE

<400> 73

Met Ser Met Gly Glu Val Glu Gln Asn Asn Tyr Gly Thr Glu Phe Glu
1 5 10 15

Lys Gly Val Asp Ser Ala Tyr Arg Ser Ser Tyr Gly Asn Glu Val Tyr
20 25 30

Leu Phe Lys Gly Asp Lys Tyr Ala Cys Ile Asp Tyr Gly Thr Asn Arg
35 40 45

Leu Val Gln Ser Ile Lys Lys Ile Ser Asp Gly Phe Thr Cys Phe His
50 55 60

Asp Thr Ile Phe Glu Asn Gly Ile Asp Ala Ala Phe Ala Ser His Arg
65 70 75 80

Thr Asp Glu Ala Tyr Phe Phe Lys Gly Glu Tyr Tyr Val Arg Val Ala
85 90 95

Val Val Pro Gly Ser Thr Ser Asp Lys Leu Met Gly Asn Pro Ala Lys
100 105 110

Ile Val Asp Tyr Trp Pro Ser Leu Arg Gly Leu Ile Pro Leu Lys Asn
115 120 125

Gln Tyr Gln Ser Ile Ile Thr Lys Asn Lys His Ser Phe Leu Phe Ile
130 135 140

Phe Leu Cys Ile Met

145

<210> 74
<211> 222
<212> PRT
<213> NONE

<400> 74

Met Ser Asn Leu Ser Ile Asp Ala Ala Tyr Arg Ser Ser Val His Asn
1 5 10 15

Glu Cys Tyr Ile Phe Val Lys Glu Arg Tyr Val Val Val Asn Tyr Asn
20 25 30

Pro Gly Gly Lys Lys Glu Lys Ile Ile His Gly Pro Lys His Ile Ser
35 40 45

Asp Gly Phe Pro Met Leu Ile Gly Thr Glu Phe Glu Lys Gly Ile Asp
50 55 60

Cys Ala Phe Asp Gly Asp Asn Asn Glu Ala Tyr Ile Phe Ser Gly Lys
65 70 75 80

Tyr Gly Gly Lys Ile Asp Tyr Val Asn Leu Thr Leu Leu Lys Asp Lys
85 90 95

Thr Pro Ile Lys Asp Met Phe Pro Ser Leu Lys Asp Thr Lys Leu Val
100 105 110

Asn Gly Ile Asp Ala Gly Ile Arg Ser Thr Gly Lys Asp Val Phe Leu
115 120 125

Phe Lys Gly Asp Glu Tyr Val Arg Ile Asp Tyr Gln Ser Glu Leu Val
130 135 140

Lys Ser Asn Lys Tyr Ile Arg Thr Gly Phe Gln Ser Leu Val Gly Thr
145 150 155 160

Val Phe Glu Phe Gly Ile Asp Ala Ala Phe Ala Ser His Val Gln Asn
165 170 175

Glu Ala Tyr Ile Phe Lys Gly Asp Tyr Tyr Ala Arg Ile Asn Val Ala
180 185 190

Pro Gly Thr Ala Ser Gly Asp Phe Ile Val Gly Gly Arg Ile Lys Arg
195 200 205

Ile His Asp Asp Trp Pro Ala Leu His Ser Ile Leu Lys Asn

210 215 220

<210> 75
<211> 230
<212> PRT
<213> NONE

<400> 75

Met Thr Asn Phe Gly Tyr Ile Asn Ala Ala Phe Arg Ser Ser Arg Asn
1 5 10 15

Asn Glu Ala Tyr Leu Phe Ile Asn Asp Lys Tyr Val Leu Leu Asp Tyr
20 25 30

Ala Pro Gly Thr Thr Asn Asp Lys Val Leu Tyr Gly Pro Ser Phe Val
35 40 45

Arg Asp Gly Tyr Lys Ser Leu Ala Asn Thr Ile Phe Gly Thr Tyr Gly
50 55 60

Ile Asp Cys Ala Phe Asp Thr Asp Asn Asn Glu Ala Phe Ile Phe Tyr
65 70 75 80

Glu Lys Phe Cys Ala Leu Ile Asp Tyr Ala Pro His Ser Asn Lys Asp
85 90 95

Lys Ile Ile Leu Gly Pro Lys Lys Ile Val Asp Met Phe Pro Phe Phe
100 105 110

Glu Gly Thr Met Phe Glu Asn Gly Ile Asp Ala Ala Phe Arg Ser Thr
115 120 125

Lys Gly Lys Glu Val Tyr Leu Phe Lys Gly Asp Lys Tyr Ala Arg Ile
130 135 140

Asp Tyr Glu Thr Asn Arg Leu Ile Gln Asn Ile Lys Asp Ile Thr Asp
145 150 155 160

Gly Phe Pro Cys Leu Arg Gly Thr Ile Phe Glu Asn Gly Ile Glu Ala
165 170 175

Ala Phe Ala Ser His Lys Thr Asn Glu Ala Tyr Leu Phe Lys Gly Glu
180 185 190

Tyr Tyr Ala Arg Ile His Phe Thr Pro Gly Ser Thr Asn Asp Thr Ile
195 200 205

Met Gly Gly Val Lys Asn Thr Leu Asp Tyr Trp Pro Ser Leu Gln Gly

```
        210                 215                 220

Ile Ile Pro Met Lys Asn
225                 230

<210>  76
<211>  230
<212>  PRT
<213>  NONE

<400>  76

Met Thr Lys Ser Gly Tyr Ile Asp Ala Ala Phe Arg Ser Ser Arg Asn
1               5                   10                  15

Asn Glu Ala Tyr Leu Phe Ile Asn Asp Lys Tyr Val Leu Leu Asp Tyr
            20                  25                  30

Ala Pro Gly Thr Ser Asn Asp Lys Val Leu Tyr Gly Pro Ser Leu Val
        35                  40                  45

Arg Asp Gly Tyr Lys Ser Leu Ala Lys Thr Ile Phe Gly Thr Tyr Gly
    50                  55                  60

Ile Asp Cys Ser Phe Asp Thr Glu Tyr Asn Glu Ala Phe Ile Phe Tyr
65                  70                  75                  80

Glu Asn Leu Cys Ala Arg Ile Asp Tyr Ala Pro His Ser Asp Lys Asp
                85                  90                  95

Lys Ile Ile Ser Gly Pro Lys Lys Ile Ala Asp Met Phe Pro Phe Phe
            100                 105                 110

Lys Gly Thr Val Phe Glu Asn Gly Ile Asp Ala Ala Phe Arg Ser Thr
        115                 120                 125

Lys Gly Lys Glu Val Tyr Leu Phe Lys Glu Asp Lys Tyr Ala Arg Ile
    130                 135                 140

Asp Tyr Gly Thr Asn Arg Leu Val Gln Asn Ile Lys Tyr Ile Ser Asp
145                 150                 155                 160

Gly Phe Pro Cys Leu Arg Gly Thr Ile Phe Glu Tyr Gly Met Asp Ser
                165                 170                 175

Ala Phe Ala Ser His Lys Thr Asn Glu Ala Tyr Leu Phe Lys Gly Glu
            180                 185                 190

Tyr Tyr Ala Arg Ile Asn Phe Thr Pro Gly Ser Thr Asn Asp Thr Ile
```

195 200 205

Met Gly Gly Val Lys Lys Thr Leu Asp Tyr Trp Pro Ser Leu Arg Gly
210 215 220

Ile Ile Pro Gln Asn Asn
225 230

<210> 77
<211> 418
<212> PRT
<213> NONE

<400> 77

Ser Arg Ser Asp Gln Glu Asn Pro Phe Ile Phe Lys Ser Asn Arg Phe
1 5 10 15

Gln Thr Ile Tyr Glu Asn Glu Asn Gly His Ile Arg Leu Leu Gln Arg
20 25 30

Phe Asp Lys Arg Ser Lys Ile Phe Glu Asn Leu Gln Asn Tyr Arg Leu
35 40 45

Leu Glu Tyr Lys Ser Lys Pro His Thr Ile Phe Leu Pro Gln Phe Thr
50 55 60

Asp Ala Asp Phe Ile Leu Val Val Leu Ser Gly Lys Ala Ile Leu Thr
65 70 75 80

Val Leu Asn Ser Asn Asp Arg Asn Ser Phe Asn Leu Glu Arg Gly Asp
85 90 95

Thr Ile Lys Leu Pro Ala Gly Thr Ile Ala Tyr Leu Ala Asn Arg Asp
100 105 110

Asp Asn Glu Asp Leu Arg Val Leu Asp Leu Ala Ile Pro Val Asn Arg
115 120 125

Pro Gly Gln Leu Gln Ser Phe Leu Leu Ser Gly Thr Gln Asn Gln Pro
130 135 140

Ser Phe Leu Ser Gly Phe Ser Lys Asn Ile Leu Glu Ala Ala Phe Asn
145 150 155 160

Thr Glu Tyr Glu Glu Ile Glu Lys Val Leu Leu Glu Glu Gln Glu Gln
165 170 175

Lys Ser Gln His Arg Arg Ser Leu Arg Asp Lys Arg Gln Glu Ile Thr

180 185 190

Asn Glu Asp Val Ile Val Lys Val Ser Arg Glu Gln Ile Glu Glu Leu
195 200 205

Ser Lys Asn Ala Lys Ser Ser Ser Lys Lys Ser Val Ser Ser Glu Ser
210 215 220

Glu Pro Phe Asn Leu Arg Ser Arg Asn Pro Ile Tyr Ser Asn Lys Phe
225 230 235 240

Gly Lys Phe Phe Glu Ile Thr Pro Glu Lys Asn Pro Gln Leu Gln Asp
245 250 255

Leu Asp Ile Phe Val Asn Ser Val Glu Ile Lys Glu Gly Ser Leu Leu
260 265 270

Leu Pro Asn Tyr Asn Ser Arg Ala Ile Val Ile Val Thr Val Asn Glu
275 280 285

Gly Lys Gly Asp Phe Glu Leu Val Gly Gln Arg Asn Glu Asn Gln Gln
290 295 300

Glu Gln Arg Glu Glu Asn Asp Glu Glu Glu Gly Gln Glu Glu Glu Thr
305 310 315 320

Thr Lys Gln Val Gln Arg Tyr Arg Ala Arg Leu Ser Pro Gly Asp Val
325 330 335

Leu Val Ile Pro Ala Gly His Pro Val Ala Ile Asn Ala Ser Ser Asp
340 345 350

Leu Asn Leu Ile Gly Phe Gly Ile Asn Ala Lys Asn Asn Gln Arg Asn
355 360 365

Phe Leu Ala Gly Glu Glu Asp Asn Val Ile Ser Gln Ile Gln Arg Pro
370 375 380

Val Lys Glu Leu Ala Phe Pro Gly Ser Ser Arg Glu Val Asp Arg Leu
385 390 395 400

Leu Thr Asn Gln Lys Gln Ser His Phe Ala Asn Ala Gln Pro Leu Gln
405 410 415

Ile Glu

<210> 78
<211> 415
<212> PRT
<213> NONE

<400> 78

```
Ser Arg Ser Asp Gln Glu Asn Pro Phe Ile Phe Lys Ser Asn Arg Phe
1               5                   10                  15

Gln Thr Ile Tyr Glu Asn Glu Asn Gly His Ile Arg Leu Leu Gln Lys
            20                  25                  30

Phe Asp Lys Arg Ser Lys Ile Phe Glu Asn Leu Gln Asn Tyr Arg Leu
        35                  40                  45

Leu Glu Tyr Lys Ser Lys Pro His Thr Leu Phe Leu Pro Gln Tyr Thr
    50                  55                  60

Asp Ala Asp Phe Ile Leu Val Val Leu Ser Gly Lys Ala Val Leu Thr
65                  70                  75                  80

Val Leu Asn Ser Asn Asp Arg Asn Ser Phe Asn Leu Glu Arg Gly Asp
                85                  90                  95

Thr Ile Lys Leu Pro Ala Gly Thr Ile Ala Tyr Leu Ala Asn Arg Asp
            100                 105                 110

Asp Asn Glu Asp Leu Arg Val Leu Asp Leu Ala Ile Pro Val Asn Asn
        115                 120                 125

Pro Gly Gln Leu Glu Ser Phe Leu Leu Ser Gly Thr Gln Asn Gln Pro
    130                 135                 140

Ser Phe Leu Ser Gly Phe Asn Lys Ser Ile Leu Glu Ala Ala Phe Asn
145                 150                 155                 160

Thr Asp Tyr Glu Glu Ile Glu Lys Val Leu Leu Glu Asp Gln Glu Gln
                165                 170                 175

Glu Pro Gln His Arg Arg Ser Leu Arg Asp Arg Arg Gln Glu Ile Asn
            180                 185                 190

Lys Glu Asn Val Ile Val Lys Val Ser Arg Glu Gln Ile Lys Glu Leu
        195                 200                 205

Ser Lys Asn Ala Lys Ser Ser Ser Lys Lys Ser Val Ser Ser Glu Ser
    210                 215                 220
```

Glu Pro Phe Asn Leu Arg Ser Arg Asn Pro Ile Tyr Ser Asn Lys Phe
225 230 235 240

Gly Lys Phe Phe Glu Ile Thr Pro Glu Lys Asn Pro Gln Leu Gln Asp
245 250 255

Leu Asp Ile Phe Val Asn Ser Val Glu Ile Lys Glu Gly Ser Leu Leu
260 265 270

Leu Pro Asn Tyr Asn Ser Arg Ala Ile Val Ile Val Thr Val Asn Glu
275 280 285

Gly Lys Gly Tyr Phe Glu Leu Val Gly Gln Arg Asn Glu Asn Gln Arg
290 295 300

Glu Glu Asn Asp Asp Glu Glu Glu Gln Glu Glu Glu Thr Ser Thr Gln
305 310 315 320

Val Gln Arg Tyr Arg Ala Lys Leu Ser Pro Gly Asp Val Phe Val Val
325 330 335

Pro Ala Gly His Pro Val Ala Ile Asn Ala Ser Ser Asp Leu Asn Leu
340 345 350

Ile Gly Phe Gly Ile Asn Ala Lys Asn Asn Gln Arg Asn Phe Leu Ala
355 360 365

Gly Glu Glu Asp Asn Val Ile Ser Gln Ile Gln Arg Pro Val Lys Glu
370 375 380

Leu Ala Phe Pro Gly Ser Ser Arg Glu Val Asp Arg Leu Leu Thr Asn
385 390 395 400

Gln Lys Gln Ser His Phe Ala Asn Ala Gln Pro Leu Gln Ile Glu
405 410 415

<210> 79
<211> 518
<212> PRT
<213> NONE

<400> 79

Met Ala Thr Thr Ile Lys Ser Arg Phe Pro Leu Leu Leu Leu Leu Gly
1 5 10 15

Ile Ile Phe Leu Ala Phe Val Cys Val Ala Tyr Ala Asn Asp Asp Glu
20 25 30

Gly Ser Glu Pro Arg Val Thr Gly Gln Arg Glu Arg Gly Arg Gln Glu
35 40 45

Gly Glu Lys Ala Glu Gln Ser Arg Glu Gln Ser Pro Gly Gln Trp Arg
50 55 60

Pro Ser His Gly Lys Glu Glu Asp Glu Glu Glu Lys Glu Gln Lys Glu
65 70 75 80

Ala Gln Ser Gly Arg Glu Lys Trp Glu Arg Lys Glu Asp Glu Glu Lys
85 90 95

Val Val Glu Glu Glu Glu Gly Glu Trp Arg Gly Ser Gln Arg His Gly
100 105 110

Asp Pro Glu Glu Arg Ser Arg Gln Arg His Arg Glu Glu Lys Thr Lys
115 120 125

Arg Gln Val Glu Glu Gln Thr Glu Glu Lys Asp Arg Arg Tyr Gln His
130 135 140

Glu Gly Lys Glu Glu Glu Thr Ser Ser Glu Ser Gln Glu Arg Arg Asn
145 150 155 160

Pro Phe Leu Phe Lys Ser Asn Lys Phe Leu Thr Leu Phe Glu Asn Glu
165 170 175

Asn Gly His Ile Arg Arg Leu Gln Arg Phe Asp Lys Arg Ser Asp Leu
180 185 190

Phe Glu Asn Leu Gln Asn Tyr Arg Leu Val Glu Tyr Arg Ala Lys Pro
195 200 205

His Ser Ile Phe Leu Pro Gln His Ile Asp Ala Glu Phe Ile Val Val
210 215 220

Val Leu Ser Gly Lys Ala Ile Leu Thr Val Leu Ser Pro Asn Asp Arg
225 230 235 240

Asn Ser Tyr Asn Leu Glu Arg Gly Asp Ala Ile Lys Ser Pro Ala Gly
245 250 255

Ala Thr Tyr Tyr Leu Val Asn Pro Asp Asp Glu Glu Asp Leu Arg Val
260 265 270

Val Asp Phe Val Ile Ser Leu Asn Arg Pro Gly Lys Phe Glu Ala Phe
275 280 285

```
Asp Leu Ser Ala Asn Arg Arg Gln Tyr Leu Arg Gly Phe Ser Lys Ser
    290                 295                 300

Val Leu Glu Ala Ser Leu Asn Thr Lys Tyr Asp Thr Ile Glu Lys Val
305                 310                 315                 320

Leu Leu Glu Glu Gln Glu Asn Glu Pro His Gln Arg Arg Asp Arg Lys
                325                 330                 335

Gly Arg Pro Gln Gly Gln Glu Lys His Ala Ile Val Lys Val Ser Arg
            340                 345                 350

Glu Gln Ile Glu Glu Leu Arg Arg Leu Ala Lys Ser Ser Ser Lys Lys
        355                 360                 365

Ser Leu Pro Ser Glu Phe Glu Pro Phe Asn Leu Arg Ser Gln Asn Pro
    370                 375                 380

Lys Tyr Ser Asn Lys Phe Gly Lys Phe Phe Glu Val Thr Pro Glu Lys
385                 390                 395                 400

Lys Tyr Pro Gln Leu Gln Asp Leu Asp Leu Leu Val Ser Ser Val Glu
                405                 410                 415

Ile Asn Glu Gly Gly Leu Leu Leu Pro His Tyr Asn Ser Arg Ala Ile
            420                 425                 430

Val Val Leu Leu Val Asn Glu Gly Lys Gly Asn Leu Glu Leu Val Gly
        435                 440                 445

Phe Lys Asn Glu Gln Gln Glu Arg Glu Asp Asn Lys Glu Arg Asn Asn
    450                 455                 460

Glu Val Gln Arg Tyr Glu Ala Arg Leu Ser Pro Gly Asp Val Val Ile
465                 470                 475                 480

Ile Pro Ala Gly His Pro Val Ser Ile Ser Ala Ser Ser Asn Leu Asn
                485                 490                 495

Leu Leu Gly Phe Gly Ile Asn Ala Glu Asn Asn Glu Arg Asn Phe Leu
            500                 505                 510

Thr Gly Ser Asp Asp Asn
        515
```

## Description

REFERENCE TO A SEQUENCE LISTING

This application contains Sequence Listings in computer readable form. The computer readable form is incorporated herein by reference.

FIELD OF THE INVENTION

The present invention relates to a detergent composition comprising a specific surfactant system and a plant derived protein or blend of plant derived proteins derived from a Leguminous plant family, preferably a Pulse protein, preferably selected from the group consisting of a Pea protein, a Chickpea protein, a Lentil protein, a Bean protein, and mixtures thereof, preferably a Pea protein, wherein the detergent composition is a hand dishwashing detergent composition.

BACKGROUND OF THE INVENTION

Detergent compositions should provide good soil and/or grease cleaning while presenting a good sudsing profile in particular a long-lasting suds profile especially in the presence of greasy soils. Users usually see suds as an indicator of the performance of the detergent composition. Moreover, the user of a detergent composition may also use the sudsing profile and the appearance of the suds (*e.g.*, density) as an indicator that the wash solution still contains sufficient active cleaning ingredients. This is particularly the case for manual washing, also referred to herein as hand-washing, where the user usually doses the detergent composition depending on the suds remaining and renews the wash solution when the suds subsides or when the suds does not look thick enough. Thus, a detergent composition, particularly a hand dishwashing detergent composition that generates or maintains low density suds during the dishwashing process would tend to be replaced by the user more frequently than is necessary. Thus, it is desirable for a detergent composition to provide a "good sudsing profile", which includes good suds height and/or density as well as good suds duration (*i.e.,* increased suds longevity) during the initial mixing of the composition with water and/or during the entire washing operation. In recent years, users also desire that hand dishwashing detergents are formulated with ingredients that will have minimal negative impact on the environment and/or the health of the users.

Suds can be formed and stabilized by surfactants and/or proteins. By co-formulating with naturally derived Leguminous plant proteins, it is possible to reduce the levels of surfactants utilized and mitigate against the negative environmental impact while still maintaining a good sudsing profile. Several families of plant derived proteins are naturally derived from Leguminous plant family (Leguminosae) including Pulse proteins, which are dried edible seeds of certain plants in the Legume family and a rich source of proteins. The United Nations Food and Agriculture Organization (FAO) recognizes 11 types of Pulse proteins, with the four main types of Pulse proteins being Pea protein, Chickpea protein, Lentil protein, and Bean protein. The Pulse proteins can be readily isolated from pulse seed sources using multi-step process, such as for example, as described in PCT Publication Nos. WO2016/01515A1, WO2014/190418, and WO2014/053052. The Pulse proteins are capable of foaming and are typically used in the development of food products (WO 2013/159192A1), nutritional supplements, and industrial and cosmetic applications (WO 2011/137524A1). However, the inclusion of Pulse proteins, particularly Pulse protein isolates, in the context of hand dishwashing detergent compositions for improving sudsing profile, particularly increased suds longevity, especially in the presence of greasy soil, has not been disclosed.

Accordingly, the need remains for an improved detergent composition comprising a plant derived protein derived from a Leguminous plant, preferably a Pulse protein, and a specific surfactant system, which provides a good sudsing profile, in particular enhanced suds boosting and/or increased suds longevity, especially in the presence of greasy soils. The composition may also provide good cleaning, particularly good grease emulsification. It is desirous to reduce the levels of surfactants in the composition versus traditional formulations without negatively impacting sudsing, grease cleaning and/or emulsification profile. The Applicant discovered that some or all of the above-mentioned needs can be at least partially fulfilled through the improved detergent composition as described herein below.

SUMMARY OF THE INVENTION

The present invention meets one or more of these needs based on the surprising discovery that by formulating a detergent composition comprising a specific surfactant system working in synergy with a plant derived protein or blend of plant derived proteins derived from a Leguminous plant family, preferably a Pulse protein, wherein the detergent composition is a hand dishwashing detergent composition, such a composition exhibits good sudsing profile, particularly desirable suds volume and/or increased suds longevity, especially in the presence of greasy soils. The composition also

provides good grease cleaning and emulsification benefits.

According to a first aspect, the present invention is directed to a detergent composition comprising: a) from about 1 wt% to about 60 wt%, preferably from about 5 wt% to about 50 wt%, more preferably from about 8 wt% to about 45 wt%, even more preferably from about 15 wt% to about 40 wt%, by weight of the composition of a surfactant system; and b) from about 0.1 wt% to about 10 wt%, preferably from about 0.5 wt% to about 5 wt%, by weight of the composition of a plant derived protein or blend of plant derived proteins derived from a Leguminous plant family, preferably a Pulse protein, wherein the detergent composition is a hand dishwashing detergent composition. The surfactant system comprises: i) an anionic surfactant, preferably the anionic surfactant is selected from the group consisting of alkyl sulfate, alkyl alkoxy sulfate, and mixtures thereof; and ii) a primary co-surfactant selected from the group consisting of an amphoteric surfactant preferably an amine oxide surfactant, a zwitterionic surfactant preferably a betaine surfactant, and mixtures thereof, preferably the primary co-surfactant is an amine oxide surfactant, wherein the weight ratio of the anionic surfactant to the primary co-surfactant is less than 9:1, preferably from 5:1 to 1:1, more preferably from 4:1 to 2:1. Preferably the Pulse protein is selected from the group consisting of a Pea protein, a Chickpea protein, a Lentil protein, a Bean protein and mixtures thereof, preferably the Pulse protein is a Pea protein. Preferably, the composition is essentially free, preferably free, of animal-, fungal- and/or bacterial-derived proteins. It has been surprisingly found that the composition of the present invention creates long lasting suds under a hand dishwashing operation, especially in the presence of greasy soils.

In another aspect, the present invention is directed to a method of manually washing dishware comprising the steps of delivering a composition according to the claims to a volume of water to form a wash liquor and immersing the dishware in the wash liquor, or delivering a composition according to the claims directly onto the dishware or cleaning implement and using the cleaning implement to clean the dishware. When the composition of the invention is used according to this method a good sudsing profile, with a long-lasting effect is achieved, especially in the presence of greasy soils.

There is also provided the use of a detergent composition of the claims to provide increased suds longevity of the composition, especially in the presence of greasy soils, wherein the detergent composition is a hand dishwashing detergent composition.

One aim of the present invention is to provide a detergent composition which can exhibit good sudsing profile, in particular enhanced suds boosting and/or increased suds longevity, especially in the presence of greasy soils, preferably over the entire dishwashing process, wherein the detergent composition is a hand dishwashing detergent composition.

Another aim of the present invention is to provide such a composition having good tough food cleaning (*e.g.*, cooked-, baked- and burnt-on soils) and/or good grease cleaning.

Yet another aim of the present invention is to provide a use of a composition, comprising a plant derived protein or blend of plant derived proteins which function to increase suds longevity and/or facilitate the reduction of surfactants in the formulation. Thus, it is an advantage of the invention to minimize production costs and/or reduce negative environmental impact.

A further aim of the present invention is to provide such a composition comprising a plant derived protein or blend of plant derived proteins, in a form which is water soluble and/or transparent resulting in improved water solubility and/or transparency of the composition, particularly in an aqueous environment.

Yet a further aim of the present invention is to provide such a composition comprising a plant derived protein or blend of plant derived proteins resulting in a composition that has low or is essentially free of phytic acid and/or protein-bound carbohydrate. This is believed to contribute to improved water solubility of the composition and/or improved plant derived protein performance to enhance sudsing profile.

The elements of the composition of the invention described in relation to the first aspect of the invention apply *mutatis mutandis* to the other aspects of the invention.

These and other features, aspects and advantages of the present invention will become evident to those skilled in the art from the detailed description which follows.

## DETAILED DESCRIPTION OF THE INVENTION

### Definitions

As used herein, the articles "a" and "an" when used in a claim, are understood to mean one or more of what is claimed or described.

As used herein, the term "amino acid identity" means the identity between a polypeptide subunit or a protomer of the vegetable protein and the reference amino acid sequence and is expressed in terms of the identity or similarity between the subunit or the protomer and the sequence. Sequence identity can be measured in terms of percentage identity; the higher the percentage, the more identical the sequences are. The percentage identity is calculated over the

length of comparison. For example, Pea Globulin proteins fall into two distinct structural groups of Pea Legumin with sedimentation coefficient of 11S (~320-400 kDa) and Pea Vincilin with sedimentation coefficient of 7S (~145-190 kDa). The Pea Legumin (11S) is the predominate storage protein in the Pea family and the mature Pea Legumin contains six subunits or protomers in which each protomer is comprised of two polypeptides linked by a disulphide bond. The amino acid identity of a Pea Legumin is typically calculated over the entire length of a subunit or protomer aligned against the entire length of the reference sequence (*e.g.*, SEQ ID NOs: 1-8). Methods of alignment of sequences for comparison are well known in the art and identity can be calculated by many known methods. Various programs and alignment algorithms are described in the art. It should be noted that the terms 'sequence identity' and 'sequence similarity' can be used interchangeably. For polypeptide sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps.

As used herein the term "animal protein" means protein that is derived from meat, or dairy products such as milk, eggs and the like.

As used herein the term "bacterial-derived protein" means protein that are produced by bacteria.

As used herein the term "fungal-derived protein" means protein that are derived from fungi.

As used herein the term "plant derived protein" or "blend of plant derived proteins" mean a protein that is derived from plant or Leguminous plant sources. As used herein the term "plant derived protein" or "blend of plant derived proteins" also mean a protein composition derived from plant sources that is uncontaminated by animal, fungal or bacterial products or any animal-, fungal, or bacterial-derived peptides that are derived from the fermentation media or the purification media.

As used herein, the term "dishware" includes cookware and tableware.

As used herein, the term "essentially free" when used to described a component means less than 0.005% by weight of the total composition of the component is present in the detergent composition.

As used herein, the term "hand dishwashing detergent composition" refers to a composition or formulation designed for cleaning dishware. The composition is commercially positioned for manual-washing of dishware. Preferred compositions are in the form of a liquid.

As used herein the term "enhanced suds boosting" means a higher volume of suds is generated upon the dissolution of the detergent composition in a washing solution for a composition comprising a plant derived protein or blend of plant derived proteins and a specific surfactant system of the present invention, as compared with the suds longevity provided by the same composition and process in the absence of the plant derived protein or blend of plant derived proteins and the specific surfactant system of the present invention.

As used herein the term "increased suds longevity" means an increase in the duration of visible suds in a washing process for cleaning soiled dishware in this case when using the composition comprising a plant derived protein or blend of plant derived proteins and a specific surfactant system of the present invention, as compared with the suds longevity provided by the same composition and process in the absence of the plant derived protein or blend of plant derived proteins and the specific surfactant system of the present invention.

As used herein the term "protein isolate" means a protein that has been isolated from a plant source based on well-known extraction processes to those skilled in the art, such as for example alkali extraction and acid preparation, protein micellation method (PMM), or low pH extraction combined with protein isolate preparation (Wanadundara et al., OCL 2016, 23(4) D407). Depending on the method of protein extraction employed, the final product could vary in terms of the protein content, type and extent of interaction with non-protein components. Isolates are more pure than other forms (e.g., concentrates) as other non-protein components have been removed to "isolate" the protein of interest. Preferably, the protein isolate has a protein content (as determined by Kjeldahl Nx6.25) of at least about 80 wt% or more, preferably about 90 wt% or more, more preferably 100%, is substantially undenatured (as determined by differential scanning calorimetry) and has a low residual fat content of less than about 1 wt%.

As used herein the term "protomer" means the structural unit of an oligomeric protein. It is the smallest unit composed of at least two different protein chains that form a larger heterooligomer by association of two or more copies of this unit.

As used herein the term "subunit" means a single protein molecule that assembles (or "co-assembles") with other protein molecules to form a protein complex.

As used herein the term "sudsing profile" refers to the properties of a detergent composition relating to suds character during the dishwashing process. For example, the sudsing profile of a detergent composition includes but is not limited to the suds generation upon dissolving of the detergent composition, and the volume and retention of the suds during the dishwashing process.

It is understood that the test methods that are disclosed in the Test Methods Section of the present application must be used to determine the respective values of the parameters of Applicants' inventions as described and claimed herein.

In all embodiments of the present invention, all percentages are by weight of the total composition, as evident by the context, unless specifically stated otherwise. All ratios are weight ratios, unless specifically stated otherwise, and all measurements are made at 25°C, unless otherwise designated.

Detergent Composition

The inventors have surprisingly discovered a new way of formulating a detergent composition to provide good sudsing profile, particularly increased suds longevity, preferably in the presence of greasy soils. Essentially, the solution is to formulate a specific surfactant system which synergizes with a plant derived protein or blend of plant derived proteins derived from a Leguminous plant family. In fact, the inventors have discovered that when the specific surfactant system is co-formulated with the plant derived protein or blend of plant derived proteins according to the invention, increased suds longevity, especially in the presence of greasy soil, is obtained. This enhanced sudsing is not observed with close surfactant systems outside the scope of the invention (*see* examples section). While not wishing to be bound by theory, it is believed that the specific surfactant system containing the plant derived protein or blend ofplant derived proteins may more easily go to the air-water interface and remain in the suds film lamellae due to its specific physical properties. As a result, the longevity of the suds is increased due to the plant derived protein-plant derived protein interactions that form strong continuous interfacial membrane that stabilizes the suds particles at the air-water interface.

In addition, the inventors have discovered that the plant derived protein or blend of plant derived proteins and surfactant system in the detergent composition also provides enhanced suds boosting benefit. Preferably, the detergent composition of the invention also provides good grease removal, in particular good uncooked grease removal.

The detergent composition of the present invention is a manual dishwashing composition, preferably in liquid form. It typically contains from about 30 wt% to about 95 wt%, preferably from about 40 wt% to about 90 wt%, more preferably from about 50 wt% to about 85 wt% by weight of the composition of a liquid carrier in which the other essential and optional components are dissolved, dispersed or suspended. One preferred component of the liquid carrier is water.

Preferably, the detergent composition of the present invention comprises a phytic acid content of about 0.5 wt% or less, preferably about 0.2 wt% or less, preferably about 0.1 wt% or less, preferably about 0.01 wt% or less by weight of the composition, most preferably the composition is essentially free, preferably free, of the phytic acid. Leguminous plant seed meals, including Pea, Chickpea, Lentil and Bean, contain phytic acid. Phytic acid (*i.e.,* myo-inositol 1,2,3,4,5,6-hexakis (dihydrogen phosphate)) is a form of phosphorus (P) in seeds which is stored in the form of phytate salts. The term "phytic acid" as used herein includes such phytate salt forms. Depending on the Leguminous plant type, the content of phytic acid may range from about 0.3 wt% to about 10 wt%. Extraction of the Leguminous plant seed meals results in the presence of phytic acid in the protein isolate recovered. Phytic acid has a negative impact on the protein isolates, specifically, the presence of phytic acid reduces the protein solubility and/or flexibility thereby preventing its absorption at the air-water interface. As the quantity of phytic acid in the protein isolate increases, the negative impact of the protein performance increases. Thus, it is desirable to incorporate Pulse protein isolates that have substantially reduced or are essentially free of phytic acid. Reduced amounts of phytic acid content in the protein isolates from extraction of the seed meal may be achieved by extraction at temperatures above 50°C, in the presence of $CaCl_2$ or $MgCl_2$, and/or in the presence of from about 0.01% to about 1% phytase. Following these actions, the precipitated phytate can be removed from the protein solution such as by centrifugation.

The detergent composition of the present invention preferably comprises a protein-bound carbohydrate content of about 2 wt% or less, preferably about 1 wt% or less, preferably about 0.5 wt% or less, preferably about 0.1 wt% or less, preferably about 0.01 wt% or less by weight of the composition, most preferably the composition is essentially free, preferably free, of the protein-bound carbohydrate. The term "protein-bound carbohydrate" as used herein means an isolated protein that has carbohydrate bound (chemically or physically) to it. Carbohydrate bound proteins have decrease performance because the carbohydrate screen the active sites of the protein and reduces the protein solubility, flexibility and/or mobility thereby preventing its absorption at the air-water interface. Therefore, it is desirable to limit the level of isolated proteins that are bound to carbohydrates in the detergent composition. Reduced amounts of protein-bound carbohydrates in the protein isolates from extraction of the seed meal may be achieved by extraction with from about 0.01% to about 1% of a carbohydrate hydrolyzing enzyme, preferably carbohydrase. The carbohydrate residues can then be separated from the protein isolate fractions such as by membrane or dialysis filtration.

Preferably the pH of the detergent composition, measured as a 10% product concentration (*i.e.,* dilution) in distilled water at 20°C, is adjusted to between about 6 and about 14, more preferably between about 7 and about 12, more preferably between about 7.5 and about 10. The pH of the composition can be adjusted using pH modifying ingredients known in the art.

The composition of the present invention can be Newtonian or non-Newtonian, preferably Newtonian. The composition has a viscosity of from about 10 to about 10,000 mPa·s, preferably from about 100 to about 5,000 mPa·s, more preferably from about 300 to about 2,000 mPa·s, or most preferably from about 500 to about 1,500 mPa·s. Viscosity is measured with a Brookfield DV-II+ Pro Viscometer using spindle 31 at 12 RPM at 20°C.

Plant Derived Protein

The plant derived protein or blend of plant derived proteins are derived from a Leguminous plant family. The Leguminous plant family is selected from the group consisting of Fabaceae (or Leguminosae), preferably Vicia faba, Vigna aconitifolia, Vigna angularis,Vigna mungo, Vigna radiata, Vigna subterranea, Vigna umbellata, Vigna unguiculata, Psophocarpus tetragonolobus, Phaseolus acutifolius, Phaseolus coccineus, Phaseolus lunatus, Phaseolus polyanthus, Phaseolus vulgaris Canavalia gladiata, Canavalia ensiformis, Lupinus mutabilis, Lupinus albus, Cicer arietinum, Lens culinaris and Pisum savitum, preferably Vicia faba, Cicer arietinum, Lens culinaris, and Pisum savitum. Preferably, the Leguminous plant protein is a Pulse protein, more preferably the Pulse protein is selected from the group consisting of a Pea protein, a Chickpea protein, a Lentil protein, a Bean protein, and mixtures thereof, most preferably the Pulse protein is a Pea protein.

Pulse proteins are an important source of food proteins, and possess functional properties including foaming that have been exploited for use in the formulation and development of food products such as bakery products, soups, and snacks. Typically, Pulse proteins contain protein components/fractions that are known as storage proteins that can be classified into four groups: Albumins, Globulins, Prolamins, and Glutelines. Globulins and Albumins are the two predominant storage proteins found in Pulse proteins. The storage proteins from different plants (*e.g.,* Pea, Chickpea, Lentil, Bean) can be classified by their sedimentation coefficient in Svedberg units (S). This coefficient indicates the speed of sedimentation of a macromolecule in a centrifugal field. It should be noted however that some small variations of sedimentations are expected depending on the type of Leguminous plant and/or the extraction protocol employed. Therefore, the sedimentation coefficient is not intended to be restrictive, but rather serve as a useful guide for the classification of the storage proteins.

Globulins represent 65%-80% of the Pulse proteins. The major Globulins found in Pulse proteins are Vicilin (7S) and Legumin (11S). The Vicilin (7S) has a trimeric structure with molecular mass (MM) of ~175-180 kDa and lacks disulfide bridging. In contrast, the Legumin (11S) has a hexameric (MM of ~340-360 kDa) quaternary structure composed of 6 subunits (MM of ~60kDa) linked by non-covalent interactions. Each subunit pair is comprised of an acidic (MM -40 kDa) and basic (MM -20 kDa) chain joined by a disulfide bond. The ratio of the Legumin:vicilin (L/V) is not fixed and may vary among different Pulse varieties and species. There is a third Globulin Pulse protein called Convicilin with its 3 or 4 subunits each having a MM of ~70 kDa and a sedimentation coefficient of ~8S. Convicilin is present in lesser amounts as compared to other Globulins. Albumins represent about 20% of the Pulse proteins. The Albumins found in Pulse proteins are soluble proteins with a variable MM (~12-28 kDa).

The Pulse proteins are capable of foaming, dispersing, gelling, and/or emulsifying. Depending on the type(s) of Pulse proteins selected and/or the levels/mixture of the Globulins:Albumins, the properties of the resulting Pulse proteins composition can be different. Preferably, the Pulse proteins can be selectively adjusted, for example by the choice of the extraction methods and/or parameters of the extraction, particularly for the purpose of optimizing their foaming properties.

The inventors have surprisingly discovered that by formulating with Leguminous plant proteins, preferably Pulse proteins, together with a specific surfactant system, it is possible to obtain a good sudsing profile, in particular enhanced suds boosting and/or increased suds longevity, especially in the presence of greasy soils. Use of protein isolates that target protein in highly pure form eliminates most of the undesirable interference from non-protein components. Therefore, it is preferred that the Leguminous plant proteins of the present invention are used in the form of Leguminous plant protein isolates. Preferably, the protein isolates have been extracted by protein micellization process and/or ultra-filtration, optionally followed by re-blending of the separated protein isolate fractions to achieve the desired ratio of the Globulins to Albumins to maximize sudsing performance.

**Pea Protein**

There are multiple varieties of Pea. The protein content for the different varieties range from 23-31%. The major Pea proteins are constituted of Globulins and Albumins, which range from ~15-25% of Pea Albumins and 50-60% of the Pea Globulins based on the total Pea protein level. Heterogeneity in the protein composition of different Pea varieties as well as in the polypeptide composition of individual proteins from a single Pea variety has been reported (Gueguen, et al., J. Science of Food and Agriculture, 35 (1984), pp. 1024-1033). The Pea proteins can be divided into various fractions according to the corresponding sedimentation coefficient. For Pea proteins, the main reported fractions are: 7S and 11S, which correspond to Pea Vicilin (7S) and Pea Legumin (11S) respectively. Pea Vicilin, the 7S Pea protein, has a trimeric structure each subunit having a MM of ~50 kDa. Pea Vicilin can be cleaved at one or two sites (called the $\alpha$-$\beta$ and $\beta$-$\gamma$ processing sites). Cleavage at the $\alpha$-$\beta$ site generates fragments of 19 kDa and 30 kDa, whereas cleavage at the $\beta$-$\gamma$ site generate fragments of 12.5 kDa or 16 kDa and 33 kDa. Cleavage at both sites generates fragments of 12.5 kDa, 13.5 kDa and 16 kDa or 19 kDa. As a result, there is extensive polypeptide heterogeneity of Pea Vicilin.

Pea Legumin comprises six subunits with a MM of -60 kDa. Each subunit is proteolytically cleaved into disulfide-linked basic and acidic polypeptides, with the size of the Pea polypeptides ranging from ~38-40 kDa for the acidic ones and ~19-22 kDa for the basic Pea polypeptides. These Pea Legumin polypeptides display charge and size heterogeneity, reflecting the possible production of varieties of the Pea Legumin. Other fractions of the Pea proteins include: 2S, 8S, and 15S, of which the 8S fraction corresponds to Pea Convicilin respectively. Pea Convicilin, the 8S Pea protein, has a trimeric structure each subunit having a MM of ~70 kDa. Pea Convicilin has an extensive homology with Pea Vicilin along the core of its protein, but holds an additional highly charged, hydrophilic sequence of 120-166 residues close to the polypeptide N-terminus.

Previous use of Pea protein isolates has reported good foaming properties, however, the Pea protein isolates must be concentrated in order to be more viscous to mediate its effect (para. [0045], US2008/0226810). Inventors have surprisingly overcome the need to concentrate the Pea protein isolates which can be achieved by co-formulating the Chickpea protein isolates with the specific surfactant system of the invention to provide good sudsing profile particularly increased suds longevity, especially in the presence of greasy soils.

Preferably, the composition of the present invention comprises a Pea protein which is a Pea protein isolate comprising the predominate storage proteins of Pea Albumin and Pea Globulin preferably selected from Pea Legumin (11S), Pea Vicilin (7S) and/or Pea Convicilin (8S), or mixtures thereof.

Preferably, the composition of the present invention may comprise a Pea protein which is a Pea protein isolate comprising:

i) a first Pea protein isolate component comprising a Pea Albumin protein, preferably from about 20 wt% to about 95 wt%, more preferably from about 50 wt% to about 95 wt%, by weight of the total Pea protein level in the composition;
ii) a second Pea protein isolate component comprising a Pea Vicilin protein and/or a Pea Convicilin protein, preferably from about 5 wt% to about 80 wt%, more preferably from about 5 wt% to about 50 wt%, by weight of the total Pea protein level in the composition; and
iii) a third Pea protein isolate component comprising a Pea Legumin protein, preferably from 0 wt% to about 30 wt%, more preferably 0 wt%, by weight of the total Pea protein level in the composition.

Preferably, the Pea protein isolate is obtained by extraction from a Pea seed meal according to a process as disclosed for example in US Publication No. US2008/0226810, and PCT Publications Nos. WO2016/15151 and WO2014/190418. Alternatively, the Pea protein isolate is commercially available as Nutralys® pea protein from Roquette Freres (France) or Pea Protein Isolate from MyProtein (United Kingdom).

There are two Pea Albumin proteins (PA1 and PA2). The major Pea Albumin protein contains two polypeptides with a MM of ~22-26 kDa, whereas the minor Pea Albumin protein is a low MM protein containing polypeptides with MM of ~6 kDa. Specifically, the Pea Albumin proteins are proposed for use in applications for suds generation. Without wishing to be bound by theory, it is believed that the Pea Albumin functions for suds generation because it has small molecular mass and high flexibility that allows for fast absorption at the air-water interface. Pea Globulins are the major storage protein in the Pea and are comprised of the Pea Legumins (11S), Pea Vicilins (7S) and/or Pea Convicilins (8S). Preferably, the Pea Globulins comprise of Pea Vicilins (7S), Pea Legumins (11S) and mixtures thereof, preferably present at a ratio of Pea Vicilins (7S): Pea Legumins (11S): of 0.5 to 1.7. Specifically, the Pea Globulins are proposed for use in applications for suds stabilization. It is believed that the Pea Globulins functions for suds stability because it has large molecular mass and can form protein-protein and protein-surfactant network at the air-water interface. Therefore, it is highly preferable for a composition of the present invention to include a blend of vegetable proteins, preferably Pulse proteins, comprising a Pea Albumin, a Pea Globulin, or mixtures thereof, in order to enhance suds boosting and/or increase suds longevity benefits, especially in the presence of greasy soils.

Preferably, the detergent composition of the present invention comprises a Pea protein which has at least 50%, preferably at least 60%, preferably at least 70%, preferably at least 80%, preferably at least 85%, preferably at least 90%, preferably at least 95%, preferably at least 98% or even 100% amino acid identity to a Pea Legumin protein (SEQ ID NOs: 1-8), a Pea Vicilin protein or a Pea Convicilin protein (SEQ ID NOs: 9-24), or a Pea Albumin protein (SEQ ID NOs: 25-31).

## Chickpea Protein

The term "Chickpea", also known as bengal gram, garbanzo bean, or gram, refers to any type of the species *Cicer arietinum.* Preferable species of Chickpeas are the two major types: Kabuli and Desi. Protein content of the Chickpea varieties range from ~20-26%. Chickpea Globulins represent the largest fraction (>50%) followed by Chickpea Albumins (>15%) based on the total Chickpea protein content. Preferably, the composition of the present invention comprises a Chickpea protein which is a Chickpea protein isolate (CPI) comprising the two predominant storage proteins which are a Chickpea Albumin, a Chickpea Globulin, or mixtures thereof. Preferably, the Chickpea protein isolate is obtained by extraction from a Chickpea source, preferably defatted Chickpea flour, according to the process as disclosed,

for example, in Papalamprou et al., J. Science of Food and Agriculture, 90(2);304-313, and PCT Publications Nos. WO2016/15151 and WO2014/190418. Alternatively, the Chickpea protein isolate can be extracted from commercially available protein concentrate as ProEarth® from Cambridge Commodities Ltd. (United Kingdom).

Previous use of Chickpea protein isolates has reported that they have low foaming capability and the foams generated with Chickpea protein isolates have low stability (Food Research Int., 52 (2013); 445-451). Inventors have surprisingly overcome these issues by co-formulating the Chickpea protein isolates with the specific surfactant system of the invention to provide good sudsing profile, particularly increased suds longevity, especially in the presence of greasy soils. Without wishing to be bound by theory, it is believed that the Chickpea Globulins and Chickpea Albumins provide the same function for suds generation and/or stabilization as described above for the Pea protein.

Preferably, the composition of the present invention may comprise a Chickpea protein which is a Chickpea protein isolate comprising:

i) a first Chickpea protein isolate component comprising a Chickpea Albumin protein, preferably from about 20 wt% to about 95 wt%, more preferably from about 50 wt% to about 95 wt%, by weight of the total Chickpea protein level in the composition; and

ii) a second Chickpea protein isolate component comprising a Chickpea Globulin protein preferably Chickpea Legumin protein, Chickpea Vicilin protein and/or Chickpea Convicilin protein, preferably from about 5 wt% to about 80 wt%, more preferably from about 5 wt% to about 50 wt%, by weight of the total Chickpea protein level in the composition.

Preferably, the detergent composition of the present invention comprises a Chickpea protein which has at least 50%, preferably at least 60%, preferably at least 70%, preferably at least 80%, preferably at least 85%, preferably at least 90%, preferably at least 95%, preferably at least 98% or even 100% amino acid identity to a Chickpea Legumin protein (SEQ ID NOs: 50-56), a Chickpea Vicilin protein or a Chickpea Convicilin protein (SEQ ID NOs: 57-70), or a Chickpea Albumin protein (SEQ ID NOs: 71-76).

**Lentil Protein**

Lentil (*Lens culinaris*), also known as *Lens esculenta,* is an edible Pulse. It's a member of the Legume family Fabaceae, known for its lens-shaped seeds. Like other Legume proteins, the Lentil varieties are rich in protein (~22-31%). Lentil Globulins represent the largest fraction at >50% of the total Lentil protein content, followed by Lentil Albumins at >15% of the total Lentil protein content. Lentil Globulins are composed of two major proteins - Lentil Legumin (11S) and Vicilin (7S) proteins. Lentil Globulins may also encompass Lentil Convicilin protein.

Preferably, the Lentil protein isolate is obtained by extraction from a Lentil source according to a process as disclosed, such as for example, Swanson, J. Amer. Oil Chemists' Society, Vol. 67(5) (1990); 276-280, and PCT Publication No. WO2016/062567. Alternatively, the Lentil protein can be extracted from commercially lentil flour available from Ingredion (Illinois, USA) or Archer Daniels Midland (Illinois, USA).

Lentil protein isolates and starch (particularly gelatinized starch) have been used in food products as foaming agents and can provide considerable foam stability, particularly associated with the Lentil Albumin (*see* WO2016/062567). Preferably, the compositions of the present invention comprise a Lentil protein isolate and are essentially free, preferably free, of a Lentil starch. The term "Lentil starch" as used herein refers to a native, unmodified starch derived form a Lentil source. Lentil starch consists of two types of molecules: the linear and helical amylose and the branched amylopectin.

Preferably, the composition of the present invention may comprise a Lentil protein which is a Lentil protein isolate comprising

i) a first Lentil protein isolate component comprising a Lentil Albumin protein, preferably from about 20 wt% to about 95 wt%, more preferably from about 50 wt% to about 95 wt%, by weight of the total Lentil protein level in the composition;

ii) a second Lentil protein isolate component comprising a Lentil Globulin protein preferably Lentil Legumin protein, Lentil Vicilin protein and/or Lentil Convicilin protein, preferably from about 5 wt% to about 80 wt%, more preferably from about 5 wt% to about 50 wt%, by weight of the total Lentil protein level in the composition.

Preferably, the detergent composition of the present invention comprises a Lentil protein which has at least 50%, preferably at least 60%, preferably at least 70%, preferably at least 80%, preferably at least 85%, preferably at least 90%, preferably at least 95%, preferably at least 98% or even 100% amino acid identity to a Lentil Vicilin protein or a Lentil Convicilin protein (SEQ ID NOs: 77-80).

**Bean Protein**

The Bean (*Phaseolus vulgaris* L.), also known as common bean or great northern bean, is an edible dry seed. The protein content of Bean varieties varies from ~18-24%. Bean Globulins represent the largest fraction at ~80% of the total Bean protein content, followed by Bean Albumins at >5% of the total Bean protein content. Bean Globulins are composed of two major proteins - Bean Vicilin (7S), which can also be refer to as phaseolin, and Bean Legumin (11S) proteins. Bean Globulins may also encompass Bean Convicilin protein.

Preferably, the Bean protein isolate is obtained by extraction from a Bean source according to a process as disclosed, such as for example, PCT Publication Nos. WO2016/01515A1, WO2014/190418, and WO2014/053052. Alternatively, the Bean protein can be extracted from commercially available protein concentrated as Vitessence™ from Ingredion (Illinois, USA).

The foaming ability and stability of the Bean protein has been reported as fair. Furthermore, the foamability of the Bean protein is concentration dependent (Sathe, et al., J. Food Science, (1981), Vol. 46(1), 71-81). Inventors have surprisingly overcome these issues by co-formulating the Bean protein isolates with the specific surfactant system of the invention to provide good sudsing profile, particularly increased suds longevity, especially in the presence of greasy soils. Without wishing to be bound by theory, it is believed that the Bean Globulins and Bean Albumins provide the same function for suds generation and/or stabilization as described above for the Pea protein.

Preferably, the composition of the present invention may comprise a Bean protein which is a Bean protein isolate comprising:

i) a first Bean protein isolate component comprising a Bean Albumin protein, preferably from about 10 wt% to about 90 wt%, more preferably from about 50 wt% to about 90 wt%, by weight of the total Bean protein level in the composition;

ii) a second Bean protein isolate component comprising a Bean Vicilin protein and/or a Bean Convicilin protein, preferably from about 5 wt% to about 85 wt%, more preferably from about 5 wt% to about 50 wt%, by weight of the total Bean protein level in the composition; and

iii) a third Bean protein isolate component comprising a Bean Legumin protein, preferably from about 5 wt% to about 85 wt%, more preferably from about 5 wt% to about 50 wt%, by weight of the total Bean protein level in the composition.

The Bean Globulin and Bean Albumin provides the same function for suds generation and stabilization as described above for the Pea protein. Preferably, the detergent composition of the present invention comprises a Bean protein which has at least 50%, preferably at least 60%, preferably at least 70%, preferably at least 80%, preferably at least 85%, preferably at least 90%, preferably at least 95%, preferably at least 98% or even 100% amino acid identity to a Bean Legumin protein (SEQ ID NOs: 32-36), a Bean Vicilin protein or a Bean Convicilin protein (SEQ ID NOs: 37-39), or a Bean Albumin protein (SEQ ID NOs: 40-49).

Preferably, the plant derived protein or blend of plant derived proteins, preferably a Pulse protein, comprise a subunit or a protomer of a Globulin, preferably the subunit or the protomer has a molecular weight ranging from 30 kDa to 80 kDa and comprises from 1% to 80% of the total plant derived protein load in the composition.

Preferably, the subunit or the protomer of the Globulin has at least 50%, preferably at least 60%, preferably at least 70%, preferably at least 80%, preferably at least 85%, preferably at least 90%, preferably at least 95%, preferably at least 98% or even 100% amino acid identity to SEQ ID NOs: 1-24, 32-39, 50-70, or 77-80.

Identity, or homology, percentages as mentioned herein in respect of the present invention are those that can be calculated with the GAP program, obtainable from GCG (Genetics Computer Group Inc., Madison, WI, USA). Alternatively, a manual alignment can be performed.

Surfactant System

The detergent composition of the present invention comprises a surfactant system. Preferably the detergent composition comprises from about 1 wt% to about 60 wt%, preferably from about 5 wt% to about 50 wt%, more preferably from about 8 wt% to 40%, by weight of the total composition of a surfactant system.

The surfactant system of the composition of the present invention comprises an anionic surfactant. Preferably, the surfactant system for the detergent composition of the present invention comprises from about 50 wt% to about 85 wt%, preferably from about 55 wt% to about 80 wt%, more preferably from about 60 wt% to about 75 wt% by weight of the surfactant system of an anionic surfactant. The anionic surfactant can be any anionic cleaning surfactant, preferably selected from sulfate and/or sulfonate anionic surfactants. HLAS (linear alkylbenzene sulfonate) would be the most preferred sulfonate anionic surfactant. Especially preferred anionic surfactant is selected from the group consisting of alkyl sulfate, alkyl alkoxy sulfate and mixtures thereof, and preferably wherein the alkyl alkoxy sulfate is an alkyl ethoxy sulfate. Preferred anionic surfactant is an alkyl ethoxy sulfate with an average ethoxylation degree of less than about 5,

preferably less than about 3, more preferably less than about 2 and more than about 0.5 and preferably wherein the alkyl ethoxy sulfate has an average alkyl carbon chain length of from about 8 to about 16, preferably from about 12 to about 15, more preferably from about 12 to about 14. Preferably, the alkyl ethoxy sulfate has an average level of branching of from about 5% to about 40%, more preferably from about 10% to about 35%, and even more preferably from about 20% to about 30%.

The average alkoxylation degree is the mol average alkoxylation degree of all the components of the mixture (*i.e.,* mol average alkoxylation degree) of the anionic surfactant. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching (\%)} = [(x1 * \text{wt\% branched alcohol 1 in alcohol } 1 + x2 * \text{wt\% branched alcohol 2 in alcohol } 2 + \ldots) / (x1 + x2 + \ldots)] * 100$$

wherein x1, x2, ... are the weight in grams of each alcohol in the total alcohol mixture of the alcohols which were used as starting material for the anionic surfactant for the composition of the invention. In the weight average branching degree calculation the weight of anionic surfactant components not having branched groups should also be included.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant also include the alkyl glyceryl sulfonate surfactants.

Preferably the surfactant system for the detergent composition of the present invention will comprise from about 1 wt% to about 40 wt%, preferably from about 6 wt% to about 32 wt%, more preferably from about 8 wt% to about 25 wt% by weight of the total detergent composition of an anionic surfactant.

The surfactant system of the detergent composition of the present invention further comprises a primary co-surfactant system, wherein the primary co-surfactant system is preferably selected from the group consisting of amphoteric surfactant preferably amine oxide, zwitterionic surfactant preferably betaine, and mixtures thereof. Preferably, the surfactant system for the detergent composition of the present invention comprises from about 15 wt% to about 50 wt%, preferably from about 20 wt% to about 45 wt%, more preferably from about 25 wt% to about 40 wt%, by weight of the surfactant system of a primary co-surfactant system. Preferably the detergent composition comprises from about 0.01 wt% to about 20 wt%, preferably from about 0.2 wt% to about 15 wt%, more preferably from about 0.5 wt% to about 10 wt% by weight of the detergent composition of an amphoteric and/or a zwitterionic surfactant, more preferably an amphoteric surfactant, even more preferably an amine oxide surfactant.

Preferably the primary co-surfactant system is an amphoteric surfactant. Preferably, the primary co-surfactant system is an amine oxide surfactant selected from the group consisting of linear or branched alkyl amine oxide, linear or branched alkyl amidopropyl amine oxide, and mixtures thereof, preferably linear alkyl dimethyl amine oxide, more preferably linear C10 alkyl dimethyl amine oxide, linear C12-C14 alkyl dimethyl amine oxides and mixtures thereof, most preferably C12-C14 alkyl dimethyl amine oxide. Preferably, the composition comprises anionic surfactant and amine oxide surfactant in a ratio of less than about 9:1, more preferably from about 5:1 to about 1:1, more preferably from about 4:1 to about 2:1, preferably from about 3:1 to about 2.5:1. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of CI-3 alkyl groups and CI-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl,

2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Preferred amine oxides include linear C10, linear C10-C12, and linear C12-C14 alkyl dimethyl amine oxides. As used herein "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 is from 10 to 24 carbon atoms, preferably from 12 to 20, and more preferably from 10 to 16. The number of carbon atoms for the one alkyl moiety (n1) should be approximately the same number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least about 50 wt%, more preferably at least about 75 wt% to about 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a CI-3 alkyl, more preferably both are selected as a C1 alkyl.

Preferably the amine oxide surfactant is a mixture of amine oxides comprising a low-cut amine oxide and a mid-cut amine oxide. The amine oxide of the composition of the invention then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls or mixtures thereof; and
b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl. In another preferred low-cut amine oxide for use herein R1 and R2 are both methyl. In an especially preferred low-cut amine oxide for use herein R1 and R2 are both methyl and R3 is n-decyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Compositions comprising R7R8R9AO tend to be unstable and do not provide very suds mileage.

Preferably the primary co-surfactant system is a zwitterionic surfactant. Suitable exampes of zwitterionic surfactants include betaines, such as alkyl betaines, alkylamidobetaine, amidazoliniumbetaine, sulfobetaine (INCI Sultaines) as well as the Phosphobetaine and preferably meets formula (I):

R1-[CO-X(CH2)n]x-N+(R2)(R3)-(CH2)m-[CH(OH)-CH2]y-Y- (I)

wherein:

R1 is a saturated or unsaturated C6-22 alkyl residue, preferably C8-18 alkyl residue, in particular a saturated C10-16 alkyl residue, for example a saturated C12-14 alkyl residue;
X is NH, NR4 with CI-4 Alkyl residue R4, O or S;
n is a number from 1 to 10, preferably 2 to 5, in particular 3;
x is 0 or 1, preferably 1;
R2 and R3 are independently a C1-4 alkyl residue, potentially hydroxy substituted such as a hydroxyethyl, preferably a methyl;
m is a number from 1 to 4, in particular 1, 2 or 3;
y is 0 or 1; and
Y is COO, SO3, OPO(OR5)O or P(O)(OR5)O, whereby R5 is a hydrogen atom
H or a C1-4 alkyl residue.

Preferred betaines are the alkyl betaines of the formula (Ia), the alkyl amido propyl betaine of the formula (Ib), the Sulfo betaines of the formula (Ic), and the Amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R1 has the same meaning as in formula I. Particularly preferred betaines are the Carbobetaine [wherein Y-=COO-], in particular the Carbobetaine of the formula (Ia) and (Ib), more preferred are the Alkylamidobetaine of the formula (Ib).

Examples of suitable betaines and sulfobetaine are the following [designated in accordance with INCI]: Almondamidopropyl of betaines, Apricotam idopropyl betaines, Avocadamidopropyl of betaines, Babassuamidopropyl of betaines, Behenam idopropyl betaines, Behenyl of betaines, betaines, Canolam idopropyl betaines, Capryl/Capram idopropyl betaines, Carnitine, Cetyl of betaines, Cocamidoethyl of betaines, Cocam idopropyl betaines, Cocam idopropyl Hydroxysultaine, Coco betaines, Coco Hydroxysultaine, Coco/Oleam idopropyl betaines, Coco Sultaine, Decyl of betaines, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl of PG-betaines, Erucam idopropyl Hydroxysultaine, Hydrogenated Tallow of betaines, Isostearam idopropyl betaines, Lauram idopropyl betaines, Lauryl of betaines, Lauryl Hydroxysultaine, Lauryl Sultaine, Milkam idopropyl betaines, Minkamidopropyl of betaines, Myristam idopropyl betaines, Myristyl of betaines, Oleam idopropyl betaines, Oleam idopropyl Hydroxysultaine, Oleyl of betaines, Olivamidopropyl of betaines, Palmam idopropyl betaines, Palm itam idopropyl betaines, Palmitoyl Carnitine, Palm Kernelam idopropyl betaines, Polytetrafluoroethylene Acetoxypropyl of betaines, Ricinoleam idopropyl betaines, Sesam idopropyl betaines, Soyam idopropyl betaines, Stearam idopropyl betaines, Stearyl of betaines, Tallowam idopropyl betaines, Tallowam idopropyl Hydroxysultaine, Tallow of betaines, Tallow Dihydroxyethyl of betaines, Undecylenam idopropyl betaines and Wheat Germam idopropyl betaines. A preferred betaine is, for example, Cocoamidopropylbetaine.

Preferably, the surfactant system of the composition of the present invention further comprises from about 1 wt% to about 25 wt%, preferably from about 1.25 wt% to about 20 wt%, more preferably from about 1.5 wt% to about 15 wt%, most preferably from about 1.5 wt% to about 5 wt%, by weight of the surfactant system of a secondary co-surfactant system preferably comprising a non-ionic surfactant. Preferably the non-ionic surfactant is an alkyl ethoxylated non-ionic surfactant, preferably comprising on average from about 9 to about 15 preferably from about 10 to about 14 carbon atoms in its alkyl chain and on average from about 5 to about 12, preferably from about 6 to about 10, most preferably from about 7 to about 8, units of ethylene oxide per mole of alcohol.

Suitable non-ionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred non-ionic surfactants are the condensation products of guerbet alcohols with from 2 to 18 moles, preferably 2 to 15, more preferably 5-12 of ethylene oxide per mole of alcohol. Preferably, the non-ionic surfactants are an alkyl ethoxylated surfactants, preferably comprising from 9 to 15 carbon atoms in its alkyl chain and from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides. Preferably the alkyl polyglucoside surfactant is a C8-C16 alkyl polyglucoside surfactant, preferably a C8-C14 alkyl polyglucoside surfactant, preferably with an average degree of polymerization of between 0.1 and 3, more preferably between 0.5 and 2.5, even more preferably between 1 and 2. Most preferably the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6. C8-C16 alkyl polyglucosides are commercially available from several suppliers (*e.g.*, Simusol® surfactants from Seppic Corporation; and Glucopon® 600 CSUP, Glucopon® 650 EC, Glucopon® 600 CSUP/MB, and Glucopon® 650 EC/MB, from BASF Corporation). Preferably, the composition comprises the anionic surfactant and the non-ionic surfactant in a ratio of from 2:1 to 50:1, preferably 2:1 to 10:1. Preferably the non-ionic surfactant is present from about 0.01 wt% to about 20 wt%, preferably from about 0.2 wt% to about 15 wt%, more preferably from about 0.5 wt% to about 10 wt% by weight of the total detergent composition.

Salt:

The composition of the present invention may optionally comprise from about 0.01% to about 3%, preferably from about 0.05% to about 2%, more preferably from about 0.2% to about 1.5%, or most preferably from about 0.5% to about 1%, by weight of the total composition of a salt, preferably a monovalent, divalent inorganic salt or a mixture thereof, preferably the divalent inorganic salt is chloride and/or sulfate salt of magnesium, calcium or zinc, most preferably magnesium chloride, sodium chloride or mixtures thereof. The composition alternatively or further comprises a multivalent metal cation in the amount of from about 0.01 wt% to about 2 wt%, preferably from about 0.1% to about 1%, more

preferably from about 0.2% to about 0.8% by weight of the composition, preferably the multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Suitable carbohydrates include alpha or beta glucan with 1,3 and/or 1.4 and/or 1,6 linkage. Glucans can be modified especially with carboxyl sulfate, glycol ether of amino groups. Glucan can be extracted from dextran. Glucan with structure close to natural glucan such as schizophyllan, scleroglucan or paramylon are particularly preferred. Preferably the composition comprises from about 0.005% to about 1% of the carbohydrates.

Hydrotrope

The composition of the present invention may optionally comprise from about 1% to about 10%, or preferably from about 0.5% to about 10%, more preferably from about 1% to about 6%, or most preferably from about 0.1% to about 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See *e.g.,* The Aqueous Phase Behaviour of Surfactants, Robert Laughlin, Academic Press, 1994, pp. 538-542. Preferably an isotropic composition is provided. Preferably the composition comprises 0.1% to 3% by weight of the total composition of a hydrotrope, preferably wherein the hydrotrope is selected from sodium, potassium, and ammonium xylene sulfonate, sodium, potassium and ammonium toluene sulfonate, sodium potassium and ammonium cumene sulfonate, and mixtures thereof.

Organic solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than about 50%, preferably from about 0.01% to about 25%, more preferably from about 0.1% to about 10%, or most preferably from about 0.5% to about 5%, by weight of the total composition of an organic solvent, preferably an alcohol, more preferably an ethanol, a polyalkyleneglycol, more preferably polypropyleneglycol, and mixtures thereof.

Amphiphilic Polymer

The composition of the present invention may further comprise from about 0.01% to about 5%, preferably from about 0.05% to about 2%, more preferably from about 0.07% to about 1% by weight of the total composition of an amphiphilic polymer selected from the groups consisting of amphiphilic alkoxylated polyalkyleneimine and mixtures thereof, preferably an amphiphilic alkoxylated polyalkyleneimine.

Preferably, the amphiphilic alkoxylated polyalkyleneimine is an alkoxylated polyethyleneimine polymer comprising a polyethyleneimine backbone having average molecular weight range from about 100 to about 5,000, preferably from about 400 to about 2,000, more preferably from about 400 to about 1,000 Daltons and the alkoxylated polyethyleneimine polymer further comprising:

(i) one or two alkoxylation modifications per nitrogen atom by a polyalkoxylene chain having an average of about 1 to about 50 alkoxy moieties per modification, wherein the terminal alkoxy moiety of the alkoxylation modification is capped with hydrogen, a C1-C4 alkyl or mixtures thereof;
(ii) an addition of one C1-C4 alkyl moiety and one or two alkoxylation modifications per nitrogen atom by a polyalkoxylene chain having an average of about 1 to about 50 alkoxy moieties per modification wherein the terminal

alkoxy moiety is capped with hydrogen, a C1-C4 alkyl or mixtures thereof; or
(iii)a combination thereof; and

wherein the alkoxy moieties comprises ethoxy (EO) and/or propxy (PO) and/or butoxy (BO) and wherein when the alkoxylation modification comprises EO it also comprises PO or BO.

Preferred amphiphilic alkoxylated polyethyleneimine polymers comprise EO and PO groups within their alkoxylation chains, the PO groups preferably being in terminal position of the alkoxy chains, and the alkoxylation chains preferably being hydrogen capped. Hydrophilic alkoxylated polyethyleneimine polymers solely comprising ethoxy (EO) units within the alkoxylation chain could also optionally be formulated within the scope of this invention.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion.

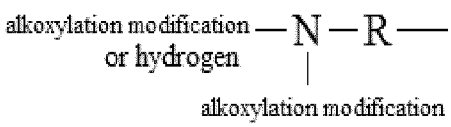

or

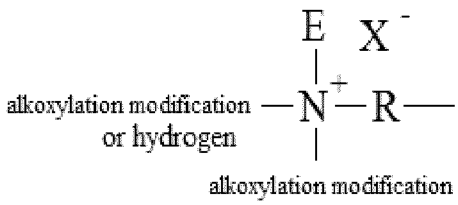

Also, for example, but not limited to, below is shown possible modifications to internal nitrogenatoms in the polyethyleneimine backbone where R represents an ethylene spacer and E represents a $C_1$-$C_4$ alkyl moiety and X- represents a suitable water soluble counterion.

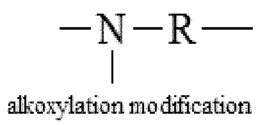

or

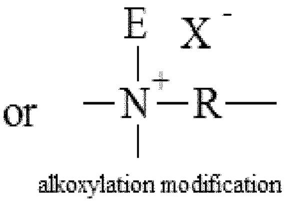

The alkoxylation modification of the polyethyleneimine backbone consists of the replacement of a hydrogen atom by a polyalkoxylene chain having an average of about 1 to about 50 alkoxy moieties, preferably from about 20 to about 45 alkoxy moieties, most preferably from about 30 to about 45 alkoxy moieties. The alkoxy moieties are selected from ethoxy (EO), propoxy (PO), butoxy (BO), and mixtures thereof. Alkoxy moieties solely comprising ethoxy units are outside the scope of the invention though. Preferably, the polyalkoxylene chain is selected from ethoxy/propoxy block moieties. More preferably, the polyalkoxylene chain is ethoxy/propoxy block moieties having an average degree of ethoxylation from about 3 to about 30 and an average degree of propoxylation from about 1 to about 20, more preferably ethoxy/propoxy block moieties having an average degree of ethoxylation from about 20 to about 30 and an average degree of propoxylation from about 10 to about 20.

More preferably the ethoxy/propoxy block moieties have a relative ethoxy to propoxy unit ratio between about 3 to about 1 and about 1 to about 1, preferably between about 2 to about 1 and about 1 to about 1. Most preferably the polyalkoxylene chain is the ethoxy/propoxy block moieties wherein the propoxy moiety block is the terminal alkoxy moiety block.

The modification may result in permanent quaternization of the polyethyleneimine backbone nitrogen atoms. The degree of permanent quaternization may be from 0% to about 30% of the polyethyleneimine backbone nitrogen atoms. It is preferred to have less than about 30% of the polyethyleneimine backbone nitrogen atoms permanently quaternized. Most preferably the degree of quaternization is about 0%.

A preferred polyethyleneimine has the general structure of Formula (II):

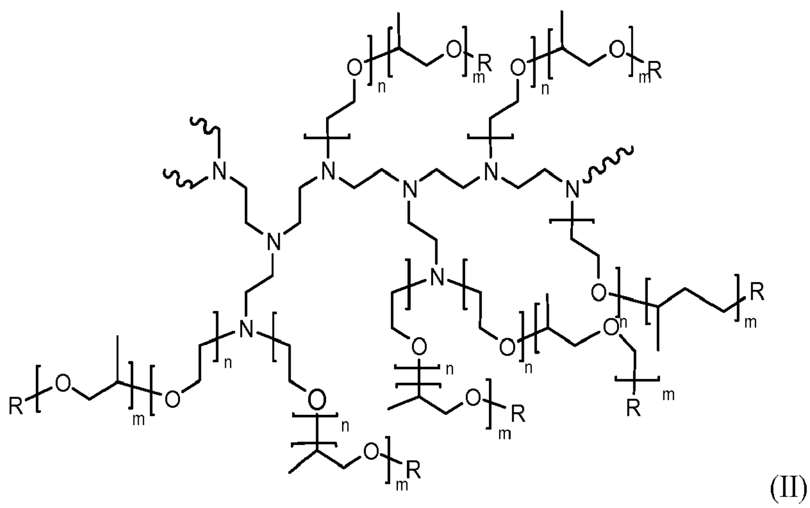

(II)

wherein the polyethyleneimine backbone has a weight average molecular weight of about 600, n of formula (II) has an average of about 10, m of formula (II) has an average of about 7 and R of formula (II) is selected from hydrogen, a C1-C4 alkyl and mixtures thereof, preferably hydrogen. The degree of permanent quaternization of formula (II) may be from 0% to about 22% of the polyethyleneimine backbone nitrogen atoms. The molecular weight of this polyethyleneimine preferably is between about 10,000 and about 15,000.

An alternative polyethyleneimine has the general structure of Formula (II) but wherein the polyethyleneimine backbone has a weight average molecular weight of about 600, n of Formula (II) has an average of about 24, m of Formula (II) has an average of about 16 and R of Formula (II) is selected from hydrogen, a $C_1$-$C_4$ alkyl and mixtures thereof, preferably hydrogen. The degree of permanent quaternization of Formula (II) may be from 0% to about 22% of the polyethyleneimine backbone nitrogen atoms. The molecular weight of this polyethyleneimine preferably is between about 25,000 and about 30,000.

Most preferred polyethyleneimine has the general structure of Formula (II) wherein the polyethyleneimine backbone has a weight average molecular weight of about 600, n of Formula (II) has an average of about 24, m of Formula (II) has an average of about 16 and R of Formula (II) is hydrogen. The degree of permanent quaternization of Formula (II) is 0% of the polyethyleneimine backbone nitrogen atoms. The molecular weight of this polyethyleneimine preferably is about from about 25,000 to about 30,000, most preferably about 28,000.

These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, and the like, as described in more detail in PCT Publication No. WO 2007/135645.

EO-PO-EO tri-block co-polymer

The composition of the present invention preferably comprises an EO-PO-EO tri-block co-polymer defined according to Formula (I): (EO)x(PO)y(EO)x, wherein EO represents ethylene oxide, and each x represents the number of EO units within the EO block. Each x is independently on average between 1 and 80, preferably between 3 and 60, more preferably between 5 and 50, most preferably between 5 and 30. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y is on average between 1 and 60, preferably between 10 and 55, more preferably between 10 and 50, more preferably between 15 and 48. The tri-block co-polymers according to the invention are preferably present in the composition at a level of from about 0.1 wt% to about 10 wt%, preferably from about 0.5 wt% to about 7.5 wt%, more preferably from about 1 wt% to about 5 wt%, by weight of the total composition.

Chelant

The detergent composition herein can comprise a chelant at a level of from about 0.1% to about 20%, preferably from about 0.2% to about 5%, more preferably from about 0.2% to about 3% by weight of total composition.

As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that

form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant.

Preferably, the composition of the present invention comprises one or more chelant, preferably selected from the group comprising carboxylate chelants, amino carboxylate chelants, amino phosphonate chelants such as MGDA (methylglycine-N,N-diacetic acid), GLDA (glutamic-N,N- diacetic acid), and mixtures thereof.

Suitable chelating agents can be selected from the group consisting of amino carboxylates, amino phosphonates, polycarboxylate chelating agents and mixtures thereof.

Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Adjunct Ingredients

The cleaning composition herein may optionally comprise a number of other adjunct ingredients such as builders (*e.g.*, preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.,* salt such as NaCl, and other mono-, di- and trivalent salts) and pH adjusters and buffering means (*e.g.*, carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Method of Washing

In another aspect, the invention is directed to a method of manually washing dishware comprising the steps of delivering a detergent composition of the invention into a volume of water to form a wash solution and immersing the dishware in the solution. As such, the composition herein will be applied in its diluted form to the dishware. Soiled surfaces e.g. dishes are contacted with an effective amount, typically from about 0.5 mL to about 20 mL (per 25 dishes being treated), preferably from about 3 mL to about 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from about 0.01 mL to about 150 mL, preferably from about 3 mL to about 40 mL of a liquid detergent composition of the invention is combined with from about 2,000 mL to about 20,000 mL, more typically from about 5,000 mL to about 15,000 mL of water in a sink having a volumetric capacity in the range of from about 1,000 mL to about 20,000 mL, more typically from about 5,000 mL to about 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from about 1 to about 10 seconds, although the actual time will vary with each application and user. The contacting of cloth, sponge, or similar article to the surface is preferably accompanied by a concurrent scrubbing of the surface.

In another aspect, the invention is directed to a method of manually washing dishware with the composition of the present invention. The method comprises the steps of: i) delivering a composition of the present invention onto the dishware or a cleaning implement; ii) cleaning the dishware with the composition in the presence of water; and iii) optionally, rinsing the dishware. The delivering step is preferably either directly onto the dishware surface or onto a cleaning implement, *i.e.,* in a neat form. The cleaning device or implement is preferably wet before or after the composition is delivered to it. Especially good grease removal has been found when the composition is used in neat form.

In another aspect, the invention is directed to a method of manually washing soiled articles comprising contacting a detergent composition of the invention with a surface, and wherein the composition modifies the hydrophobicity of the surface as a result of the contacting step.

Another aspect of the present invention is directed to a method of promoting suds longevity or grease emulsification in a washing process for washing soiled articles, preferably dishware. The method comprises the steps of: a) delivering a detergent composition of the invention to a volume of water to form a wash liquor; and b) immersing the soiled articles into said wash liquor. Preferably, the plant derived protein or blend of plant derived proteins according to the invention is present at a concentration of about 0.005 ppm to about 60 ppm, preferably at a concentration of about 0.02 ppm to about 12 ppm, in an aqueous wash liquor during the washing process.

Another aspect of the present invention is use, in a hand dishwashing detergent composition, of a combination of: i) a plant derived protein or blend of plant derived proteins selected from the group consisting of a Pea protein, a Chickpea protein, a Lentil protein, a Bean protein, and mixtures thereof, most preferably a Pea protein; and ii) a surfactant system comprising an anionic surfactant and a primary co-surfactant selected from the group consisting of amphoteric surfactant preferably an amine oxide surfactant, a zwitterionic surfactant preferably a betaine surfactant, and mixtures thereof, preferably the primary co-surfactant is amine oxide, wherein the weight ratio of anionic surfactant to the primary co-surfactant is less than about 9:1, more preferably from about 5:1 to about 1:1, more preferably from about 4:1 to about 2:1; to provide enhanced suds boosting and/or increased suds longevity in an aqueous wash liquor during a hand dish washing process, especially when in the presence of greasy soils.

TEST METHODS

The following assay set forth must be used in order that the invention described and claimed herein may be more fully understood.

Test Method 1: Glass Vial Suds Mileage

The method measures the evolution of suds volume over time generated by a certain solution of test detergent composition in the presence of a greasy soil, *e.g.,* olive oil. The following factors may affect the measurement results and therefore should be controlled carefully: (a) concentration of the test detergent composition; (b) hardness of the water; (c) water temperature; (d) speed of stirring; and (e) speed and number of the shaking. Following steps are followed to obtain the suds measurements for each test detergent composition:

1. Test solutions are prepared by subsequently adding aliquots into 40 mL glass vials (dimensions: 95 mm Height x 27.5 mm Diameter), preferably graduated vials at room temperature, of: a) 10 g of an aqueous detergent solution at 0.11% detergent concentration and water hardness (15 °dh), and b) 0.11 g of olive oil (Bertolli®, Extra Virgin Olive Oil). The test detergent contains 2% of the plant derived protein and is compared with a nil-plant derived protein detergent.
2. The test solutions are mixed in the closed test vials by stirring at room temperature for 2 minutes at 500 RPM on a magnetic stirring plate (IKA, model # RTC B S001; VWR magnetic stirrer, catalog # 58949-012), followed by manually shaking for 20 seconds with an upwards downwards movement (about 2 up and down cycles per second, +/- 30 cm up and 30 cm down) and the initial suds heights (HI) are recorded with a ruler. HI is a measurement of the suds height.
3. Following the shaking, the test solutions in the closed vials are further stirred at 500 RPM on the magnetic stirring plate for 60 minutes inside a water bath at 35°C to maintain a constant temperature. The samples are then shaken manually for another 20 seconds as described above. The final suds heights (H2) are recorded.
4. The Suds Stability Index (SSI) of an individual sample is expressed as (H2/H1)*100. Protein solutions that produce larger suds heights (HI and H2), preferably combined with lower drops in suds height between HI and H2, are more desirable, *i.e.,* high HI and high suds stability index. A Protein Impact Index (PII) can be further calculated by cross-comparing the Suds Stability Index of the protein comprising sample versus a reference sample single variably lacking the protein, *i.e.* (SSI (protein sample)/SSI (nil protein reference))*100.

EXAMPLES

The following examples are provided to further illustrate the present invention and are not to be construed as limitations of the present invention, as many variations of the present invention are possible without departing from its spirit or scope.

Example 1: Plant Derived Protein Detergent Compositions Impact on Suds Mileage

The ability to maintain suds mileage in the presence of greasy soil, *i.e.* olive oil, is assessed for test detergent compositions with or without the plant derived protein. The compositions are summarized in Table 1. Composition Ex. 4 is a plant derived protein containing test detergent compositions according to the present invention, made with surfactant

system comprising Alkyl(C12/C13)-0.6 ethoxylated sulfate and Alkyl(C12/C14)-dimethyl amine oxide in 4:1 weight ratio, in the presence of 2% Pea protein. Composition Ex. 3 is a reference composition containing the same surfactant system as in Test Composition Ex. 4 in the absence of the pea protein. Composition Ex. 2 is a test composition containing the Alkyl(C12/C13)-0.6 ethoxylated sulfate minus the amine oxide in the presence of the Pea protein. Composition Ex. 1 is a reference composition to Test Composition Ex. 2. Reference Composition Ex. 1 contains Alkyl(C12/C13)-0.6 ethoxylated sulfate minus the amine oxide in the absence of the Pea protein. The compositions are produced through standard mixing of the components described in Table 1.

Table 1 - Detergent Compositions

| Ingredients | Reference Comp. Ex. 1 | Test Comp. Ex. 2 | Reference Comp. Ex. 3 | Test Comp. Ex. 4 |
|---|---|---|---|---|
| Sodium alkyl ethoxy sulfate (C1213EO0.6S) | 26.25% | 26.25% | 21% | 21% |
| n-C 12-14 Di Methyl Amine Oxide | - | - | 5.25% | 5.25% |
| Lutensol® XP80 (non-ionic surfactant supplied by BASF) | 1% | 1% | 1% | 1% |
| Sodium Chloride | 0.7% | 0.7% | 0.7% | 0.7% |
| Poly Propylene Glycol (MW 2000) | 0.7% | 0.7% | 0.7% | 0.7% |
| Ethanol | 2% | 2% | 2% | 2% |
| Sodium Hydroxide | 0.2% | 0.2% | 0.2% | 0.2% |
| Pea protein* | - | 2% | - | 2% |
| Minors (perfume, preservative, dye) + water | To 100 % | To 100 % | To 100 % | To 100 % |
| pH (@ 0.12% solution) | 8.35 | 8.35 | 8.35 | 8.35 |
| * Pea protein from Myprotein (UK). | | | | |

The Compositions Ex. 1-4 are tested for the suds volume (*i.e.,* suds height) and suds stability and the data (not shown) are recorded. The Suds Stability Index ("SSI") for each of the compositions is calculated (not shown) according to Test Method 1. A Protein Impact Index ("PII"), which is a unitless number that indicates the relative impact of the plant derived protein on the suds mileage of a test composition as compared to a reference composition which is missing the protein, is calculated. The PII is calculated by dividing the SSI for the test composition containing the plant derived protein by the SSI for the reference composition minus the plant derived protein, followed by multiplying the quotient by 100. The higher the PII, the better the suds mileage performance of the test composition.

The PPI results for Test Composition Ex. 4 vs. Reference Composition Ex. 3 are summarized in Table 2, and the PPI results for Test Composition Ex. 2 vs. Reference Composition Ex. 1 are summarized in Table 3.

Table 2 - Performance on Suds Stability of Composition containing Pea Protein with AES/AO Surfactant System

| Composition | Protein Impact Index (PII) |
|---|---|
| Reference Comp. Ex. 3 | 100 |
| Test Comp. Ex. 4 | 127* |
| * vs. Reference comp. Ex. 3. | |

It is clear from the results in Table 2 that the addition of the Pea protein to a surfactant system (AES/AO) within the scope of the present invention leads to an enhanced increase of suds duration, particularly in the presence of greasy soil, as evidenced by a PII of 127.

Table 3 - Performance on Suds Stability of Composition containing Pea Protein with AES Surfactant System

| Composition | Protein Impact Index |
|---|---|
| Reference Comp. Ex. 1 | 100 |

(continued)

| Composition | Protein Impact Index |
|---|---|
| Test Comp. Ex. 2 | 68* |
| * vs. Reference Comp. Ex. 1. | |

The data in Table 3 shows that the addition of the Pea protein according to the invention to a surfactant system (AES and no primary co-surfactant) outside the scope of the invention results in a suds mileage represented by PII of 68. This suds mileage is a noticeable drop when compared to the Test Comp. Ex. 4 with PII of 127. Therefore, the enhanced increased suds mileage performance of the composition comprising the plant derived protein with the specific surfactant system according to the present invention is unexpected and a synergy between the specific surfactant system and the protein of the invention is observed.

All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A detergent composition comprising:

   a) from 1 wt% to 60 wt%, preferably from 5 wt% to 50 wt%, more preferably from 8% to 45%, even more preferably from 15% to 40%, by weight of the composition of a surfactant system, wherein the surfactant system comprises:

      i) an anionic surfactant, preferably the anionic surfactant is selected from the group consisting of alkyl sulfate, alkyl alkoxy sulfate, and mixtures thereof; and
      ii) a primary co-surfactant selected from the group consisting of an amphoteric surfactant preferably an amine oxide surfactant, a zwitterionic surfactant preferably a betaine surfactant, and mixtures thereof;

   wherein the weight ratio of the anionic surfactant to the primary co-surfactant is less than 9:1, more preferably from 5:1 to 1:1, more preferably from 4:1 to 2:1; and
   b) from 0.1 wt% to 10 wt%, preferably from 0.5 wt% to 5 wt%, by weight of the composition of a vegetable protein or blend of vegetable proteins derived from a Leguminous plant family selected from the group consisting of Fabaceae (or Leguminosae), preferably Vicia faba, Vigna aconitifolia, Vigna angularis,Vigna mungo, Vigna radiata, Vigna subterranea, Vigna umbellata, Vigna unguiculata, Psophocarpus tetragonolobus, Phaseolus acutifolius, Phaseolus coccineus, Phaseolus lunatus, Phaseolus polyanthus, Phaseolus vulgaris Canavalia gladiata, Canavalia ensiformis, Lupinus mutabilis, Lupinus albus, Cicer arietinum, Lens culinaris and Pisum savitum, preferably Vicia faba, Cicer arietinum, Lens culinaris, and Pisum savitum, preferably the Leguminous plant protein is a Pulse protein, more preferably the Pulse protein is selected from the group consisting of a Pea protein, a Chickpea protein, a Lentil protein, a Bean protein and mixtures thereof, most preferably the Pulse protein is a Pea protein; wherein the detergent composition is a hand dishwashing detergent composition.

2. The composition according to claim 1, wherein:

   i) Pea protein is a Pea protein isolate comprising a Pea Albumin, Pea Globulin preferably Pea Legumin, Pea Vicilin and/or Pea Convicilin, or mixtures thereof;
   ii) Chickpea protein is a Chickpea protein isolate comprising a Chickpea Albumin, Chickpea Globulin preferably Chickpea Legumin, Chickpea Vicilin and/or Chickpea Convicilin, or mixtures thereof;
   iii) Lentil protein is a Lentil protein isolate comprising a Lentil Albumin, Lentil Globulin preferably Lentil Legumin, Lentil Vicilin and/or Lentil Convicilin, or mixtures thereof; and
   iv) Bean protein is a Bean protein isolate comprising a Bean Albumin, a Bean Globulin preferably Bean Legumin, Bean Vicilin and/or Bean Convicilin, or mixtures thereof.

3. The composition according to any preceding claims, wherein the Pea protein is a Pea protein isolate comprising:

   i) a first Pea protein isolate component comprising a Pea Albumin protein, preferably from 20 wt% to 95 wt%, more preferably from 50 wt% to 95 wt%, by weight of the total Pea protein level in the composition;
   ii) a second Pea protein isolate component comprising a Pea Vicilin protein and/or a Pea Convicilin protein, preferably from 5 wt% to 80 wt%, more preferably from 5 wt% to 50 wt%, by weight of the total Pea protein level in the composition; and
   iii) a third Pea protein isolate component comprising a Pea Legumin protein, preferably from 0 wt% to 30 wt%, more preferably 0 wt%, by weight of the total Pea protein level in the composition.

4. The composition according to any preceding claims, wherein the Chickpea protein is a Chickpea protein isolate comprising:

   i) a first Chickpea protein isolate component comprising a Chickpea Albumin protein, preferably from 20 wt% to 95 wt%, more preferably from 50 wt% to 95 wt%, by weight of the total Chickpea protein level in the composition; and
   ii) a second Chickpea protein isolate component comprising a Chickpea Globulin protein preferably Chickpea Legumin protein, Chickpea Vicilin protein and/or Chickpea Convicilin protein, preferably from 5 wt% to 80 wt%, more preferably from 5 wt% to 50 wt%, by weight of the total Chickpea protein level in the composition.

5. The composition according to any preceding claims, wherein the Lentil protein is a Lentil protein isolate comprising:

i) a first Lentil protein isolate component comprising a Lentil Albumin protein, preferably from 20 wt% to 95 wt%, more preferably from 50 wt% to 95 wt%, by weight of the total Lentil protein level in the composition; and
ii) a second Lentil protein isolate component comprising a Lentil Globulin protein preferably Lentil Legumin protein, Lentil Vicilin protein and/or Lentil Convicilin protein, preferably from 5 wt% to 80 wt%, more preferably from 5 wt% to 50 wt%, by weight of the total Lentil protein level in the composition.

**6.** The composition according to any preceding claims, wherein the Bean protein is a Bean protein isolate comprising:

i) a first Bean protein isolate component comprising a Bean Albumin protein, preferably from 10 wt% to 90 wt%, more preferably from 50 wt% to 90 wt%, by weight of the total Bean protein level in the composition;
ii) a second Bean protein isolate component comprising a Bean Vicilin protein and/or a Bean Convicilin protein, preferably from 5 wt% to 85 wt%, more preferably from 5 wt% to 50 wt%, by weight of the total Bean protein level in the composition; and
iii) a third Bean protein isolate component comprising a Bean Legumin protein, preferably from 5 wt% to 985 wt%, more preferably from 5 wt% to 50 wt%, by weight of the total Bean protein level in the composition.

**7.** The composition according to any preceding claims, wherein:

i) the Pea protein has at least 50%, preferably at least 60%, preferably at least 70%, preferably at least 80%, preferably at least 85%, preferably at least 90%, preferably at least 95%, preferably at least 98% or even 100% amino acid identity to a Pea Legumin protein (SEQ ID NOs: 1-8), a Pea Vicilin protein or a Pea Convicilin protein (SEQ ID NOs: 9-24), or a Pea Albumin protein (SEQ ID NOs: 25-31);
ii) the Bean protein has at least 50%, preferably at least 60%, preferably at least 70%, preferably at least 80%, preferably at least 85%, preferably at least 90%, preferably at least 95%, preferably at least 98% or even 100% amino acid identity to a Bean Legumin protein (SEQ ID NOs: 32-36), a Bean Vicilin protein or a Bean Convicilin protein (SEQ ID NOs: 37-39), or a Bean Albumin protein (SEQ ID NOs: 40-49);
iii) the Chickpea protein has at least 50%, preferably at least 60%, preferably at least 70%, preferably at least 80%, preferably at least 85%, preferably at least 90%, preferably at least 95%, preferably at least 98% or even 100% amino acid identity to a Chickpea Legumin protein (SEQ ID NOs: 50-56), a Chickpea Vicilin protein or a Chickpea Convicilin protein (SEQ ID NOs: 57-70), or a Chickpea Albumin protein (SEQ ID NOs: 71-76); and
iv) the Lentil protein has at least 50%, preferably at least 60%, preferably at least 70%, preferably at least 80%, preferably at least 85%, preferably at least 90%, preferably at least 95%, preferably at least 98% or even 100% amino acid identity to a Lentil Vicilin protein or a Lentil Convicilin protein (SEQ ID NOs: 77-80).

**8.** The composition according to any preceding claims, wherein the Pulse protein comprises a subunit or a protomer of a Globulin, preferably the subunit or protomer has a molecular weight ranging from 30 kDa to 80 kDa and comprises from 1% to 80% of the total plant derived protein load in the composition.

**9.** The composition according to claim 8, wherein the subunit or the protomer of the Globulin has at least 50%, preferably at least 60%, preferably at least 70%, preferably at least 80%, preferably at least 85%, preferably at least 90%, preferably at least 95%, preferably at least 98% or even 100% amino acid identity to SEQ ID NOs: 1-24, 32-39, 50-70, or 77-80.

**10.** The composition according to any preceding claims, wherein the composition comprises a phytic acid content of 0.5% or less, preferably 0.2% or less, more preferably 0.1% or less, even more preferably 0.01% or less by weight of the composition, most preferably the composition is essentially free of the phytic acid.

**11.** The composition according to any preceding claims, wherein the composition comprises a protein-bound carbohydrate content of 2% or less, preferably 1% or less, preferably 0.5% or less, preferably 0.1% or less, preferably 0.01% or less by weight of the composition, most preferably the composition is essentially free of the protein-bound carbohydrate.

**12.** The composition according to any preceding claims, wherein: a) the surfactant system comprises:

i) from 50 wt% to 85 wt%, preferably from 55 wt% to 80 wt%, more preferably from 60 wt% to 75 wt%, by weight of the surfactant system of the anionic surfactant, preferably the anionic surfactant is an alkyl ethoxy sulfate, preferably an alkyl ethoxy sulfate with an average degree of ethoxylation of less than 5, preferably less than 3, more preferably less than 2 and more than 0.5, and preferably with a weight average level of branching of from

5% to 60%, preferably from 10% to 55%, more preferably from 15% to 50%, even more preferably from 20% to 45%, most preferably from 25% to 45%, and preferably wherein the alkyl ethoxy sulfate has an average alkyl carbon chain length of from 8 to 16, preferably from 12 to 15, more preferably from 12 to 14; and
ii) from 15 wt% to 50 wt%, preferably from 20 wt% to 45 wt%, more preferably from 25 wt% to 40 wt%, by weight of the surfactant system of the primary co-surfactant, preferably the primary co-surfactant is an amine oxide selected from the group consisting of linear or branched alkyl amine oxide, linear or branched alkyl amidopropyl amine oxide, and mixtures thereof, preferably linear alkyl dimethyl amine oxide, more preferably linear C10 alkyl dimethyl amine oxide, linear C12-C14 alkyl dimethyl amine oxides and mixtures thereof, most preferably C12-C14 alkyl dimethyl amine oxide.

**13.** The composition according to any preceding claims, further comprising from 1% to 25%, preferably from 1.25% to 20%, more preferably from 1.5% to 15%, most preferably from 1.5% to 5%, by weight of the surfactant system of a non-ionic surfactant, preferably an alkyl ethoxylated non-ionic surfactant, preferably comprising on average from 9 to 15 preferably from 10 to 14 carbon atoms in its alkyl chain and on average from 5 to 12, preferably from 6 to 10, most preferably from 7 to 8, units of ethylene oxide per mole of alcohol.

**14.** A method of manually washing dishware comprising the steps of delivering a composition according to any of the preceding claims to a volume of water to form a wash liquor and immersing the dishware in the wash liquor, or delivering a composition according to any of the preceding claims directly onto the dishware or cleaning implement and using the cleaning implement to clean the dishware.

**15.** Use of a composition according to any of claims 1 to 13 to provide enhanced suds longevity of the composition and/or facilitate reduction of surfactants in the composition.

Europäisches Patentamt
European Patent Office
Office européen des brevets

# EUROPEAN SEARCH REPORT

Application Number
EP 18 16 1364

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/327774 A1 (LANT NEIL JOSEPH [GB] ET AL) 16 November 2017 (2017-11-16) * paragraphs [0001] - [0004], [0008], [0045] - [0079], [0095] - [0096]; claims; examples 1,2 * | 1-15 | INV. C11D1/94 C07K14/415 C11D11/00 C11D3/382 |
| Y | US 2018/016522 A1 (LANT NEIL JOSEPH [GB] ET AL) 18 January 2018 (2018-01-18) * paragraphs [0002] - [0014], [0024], [0042] - [0080], [0118] - [0122]; claims; example 1 * | 1-15 | |
| Y,D | WO 2014/190418 A1 (BURCON NUTRASCIENCE MB CORP [CA]) 4 December 2014 (2014-12-04) * paragraphs [0002], [0012], [0021] * | 1-15 | |
| Y,D | WO 2016/062567 A1 (UNILEVER NV [NL]; UNILEVER PLC [GB]; CONOPCO INC DBA UNILEVER [US]) 28 April 2016 (2016-04-28) * page 1, paragraph 1 - page 4, paragraph 1 * * page 7, paragraph 6 * * page 9, lines 29-30 * | 1,2,5,8, 9,11-15 | TECHNICAL FIELDS SEARCHED (IPC) C11D C07K |
| | -/-- | | |

The present search report has been drawn up for all claims

1

| Place of search | Date of completion of the search | Examiner |
|---|---|---|
| The Hague | 24 August 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 16 1364

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TOEWS R ET AL: "Physicochemical and functional properties of protein concentrates from pulses", FOOD RESEARCH INTERNATIONAL, ELSEVIER, AMSTERDAM, NL, vol. 52, no. 2, 20 December 2012 (2012-12-20), pages 445-451, XP028539049, ISSN: 0963-9969, DOI: 10.1016/J.FOODRES.2012.12.009 * page 445, right-hand column, last paragraph - page 446, left-hand column, paragraph 2 * * page 449, left-hand column, paragraph 1 - page 445, right-hand column, paragraph 4 * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 24 August 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 16 1364

This annex lists the patent family members relating to the patent documents cited in the above-mentioned European search report. The members are as contained in the European Patent Office EDP file on
The European Patent Office is in no way liable for these particulars which are merely given for the purpose of information.

24-08-2018

| Patent document cited in search report | Publication date | Patent family member(s) | Publication date |
|---|---|---|---|
| US 2017327774 A1 | 16-11-2017 | EP 3243894 A1 | 15-11-2017 |
| | | US 2017327774 A1 | 16-11-2017 |
| | | WO 2017196813 A1 | 16-11-2017 |
| US 2018016522 A1 | 18-01-2018 | EP 3269729 A1 | 17-01-2018 |
| | | US 2018016522 A1 | 18-01-2018 |
| | | WO 2018013395 A1 | 18-01-2018 |
| WO 2014190418 A1 | 04-12-2014 | AU 2014273794 A1 | 03-12-2015 |
| | | BR 112015029903 A2 | 25-07-2017 |
| | | CA 2912731 A1 | 04-12-2014 |
| | | CN 105246348 A | 13-01-2016 |
| | | EP 3003062 A1 | 13-04-2016 |
| | | JP 2016519942 A | 11-07-2016 |
| | | KR 20160012227 A | 02-02-2016 |
| | | RU 2015155822 A | 05-07-2017 |
| | | TW 201505554 A | 16-02-2015 |
| | | US 2014356510 A1 | 04-12-2014 |
| | | US 2017156367 A1 | 08-06-2017 |
| | | US 2017156368 A1 | 08-06-2017 |
| | | WO 2014190418 A1 | 04-12-2014 |
| | | ZA 201508465 B | 30-08-2017 |
| WO 2016062567 A1 | 28-04-2016 | AR 102382 A1 | 22-02-2017 |
| | | EP 3209141 A1 | 30-08-2017 |
| | | WO 2016062567 A1 | 28-04-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201601515A1 A
- WO 2014190418 A
- WO 2014053052 A
- WO 2013159192 A1
- WO 2011137524 A1
- US 20080226810 A
- WO 201615151 A
- WO 2016062567 A
- WO 201601515 A1
- US 3915903 A
- WO 2007135645 A


### Non-patent literature cited in the description


- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **WANADUNDARA et al.** *OCL,* 2016, vol. 23 (4), D407
- **GUEGUEN et al.** *J. Science of Food and Agriculture,* 1984, vol. 35, 1024-1033
- **PAPALAMPROU et al.** *J. Science of Food and Agriculture,* vol. 90 (2), 304-313
- *Food Research Int.,* 2013, vol. 52, 445-451
- **SWANSON.** *J. Amer. Oil Chemists' Society,* 1990, vol. 67 (5), 276-280
- **SATHE et al.** *J. Food Science,* 1981, vol. 46 (1), 71-81
- **ROBERT LAUGHLIN.** The Aqueous Phase Behaviour of Surfactants. Academic Press, 1994, 538-542